# EUROPEAN PATENT APPLICATION

(11) **EP 1 067 188 A1**
(43) Date of publication of application: **10.01.2001**
(21) Application number: 99202234.3
(22) Date of filing: 08.07.1999
(51) Int. Cl.: C12N 15/34, C12N 15/86, C12N 15/10, A61K 48/00, C07K 14/705

(54) **Infection with chimaeric adenoviruses of cells negative for the adenovirus serotype 5 Coxsacki adenovirus receptor (CAR)**

(71) Applicant: Introgene B.V., 2333 AL Leiden (NL)
(72) Inventor: Havenga, Menzo, 2401 BV Alphen aan den Rijn (NL); Vogels, Ronald, 3461 HW Linschoten (NL)
(74) Representative: Ottevangers, Sietse Ulbe

(57) **Abstract**

The invention relates to the field of molecular genetics and medicine. The invention discloses a method for delivering a nucleic acid of interest to a host cell by means of a gene delivery vehicle based on adenoviral material, whereby said gene delivery vehicle delivers the nucleic acid to the host cell by associating with a binding site and/or a receptor present on CAR-negative cells, said binding site and/or receptor being a binding site and/or a receptor for adenovirus subgroups D and/or F.

## Description

The invention relates to the field of molecular genetics and medicine. In particular the present invention relates to the field of gene therapy, more in particular to gene therapy using adenoviruses.

In gene therapy, genetic information is delivered to a host cell in order to either correct (supplement) a genetic deficiency in said cell, or to inhibit an unwanted function in said cell, or to eliminate said host cell. Of course the genetic information can also be intended to provide the host cell with a wanted function, for instance to supply a secreted protein to treat other cells of the host, etc.

Thus there are basically three different approaches in gene therapy, one directed towards compensating a deficiency present in a (mammalian) host; the second directed towards the removal or elimination of unwanted substances (organisms or cells) and the third towards providing a cell with a wanted function.

For the purpose of gene therapy, adenoviruses have been proposed as suitable vehicles to deliver genes to the host. Gene-transfer vectors derived from adenoviruses (so-called adenoviral vectors) have a number of features that make them particularly useful for gene transfer. 1) the biology of the adenoviruses is characterised in detail, 2) the adenovirus is not associated with severe human pathology, 3) the virus is extremely efficient in introducing its DNA into the host cell, 4) the virus can infect a wide variety of cells and has a broad host-range, 5) the virus can be produced at high virus titers in large quantities, and 6) the virus can be rendered replication defective by deletion of the early-region 1 (E1) of the viral genome (Brody et al, 1994).

However, there are still drawbacks associated with the use of adenoviral vectors especially the well investigated serotypes of subgroup C adenoviruses. These serotypes require the presence of the Coxsacki adenovirus receptor (CAR) on cells for successful infection. Although this protein is expressed by many cells and established cell lines, this protein is absent for many other primary cells and cell lines making the latter cells difficult to infect with serotypes 1, 2, 5, and 6.

The adenovirus genome is a linear double-stranded DNA molecule of approximately 36000 base pairs. The adenovirus DNA contains identical Inverted Terminal Repeats (ITR) of approximately 90-140 base pairs with the exact length depending on the serotype. The viral origins of replication are within the ITRs exactly at the genome ends.

Most adenoviral vectors currently used in gene therapy have a deletion in the E1 region, where novel genetic information can be introduced. The E1 deletion renders the recombinant virus replication defective (Levrero et al, 1991). It has been demonstrated extensively that recombinant adenovirus, in particular serotype 5 is suitable for efficient transfer of genes *in vivo* to the liver, the airway epithelium and solid tumours in animal models and human xenografts in immunodeficient mice (Bout, 1996; Blaese *et al.,* 1995). Thus, preferred methods for *in vivo* gene transfer into target cells make use of adenoviral vectors as gene delivery vehicles.

At present, six different subgroups of human adenoviruses have been proposed which in total encompasses 51 distinct adenovirus serotypes. Besides these human adenoviruses an extensive number of animal adenoviruses have been identified (see Ishibashi et al, 1983).

A serotype is defined on the basis of its immunological distinctiveness as determined by quantitative neutralisation with animal antisera (horse, rabbit). If neutralisation shows a certain degree of cross-reaction between two viruses, distinctiveness of serotype is assumed if A) the hemagglutinins are unrelated, as shown by lack of cross-reaction on hemagglutination-inhibition, or B) substantial biophysical/ biochemical differences in DNA exist (Francki et al, 1991). The nine serotypes identified last (42-51) were isolated for the first time from HIV- infected patients (Hierholzer et al 1988; Schnurr et al 1993; De Jong et al 1998). For reasons not well understood, most of such immuno-compromised patients shed adenoviruses that were rarely or never isolated from immuno-competent individuals (Hierholzer et al 1988, 1992; Khoo et al, 1995, De Jong et al, 1998).

The adenovirus serotype 5 is most widely used for gene therapy purposes. Similar to serotypes 2, 4 and 7, serotype 5 has a natural affiliation towards lung epithelia and other respiratory tissues. In contrast, it is known that, for instance, serotypes 40 and 41 have a natural affiliation towards the gastrointestinal tract. For a detailed overview of the disease association of the different adenovirus serotypes see table 1. The underlying reason for the different natural affiliations of serotypes towards specific organs can be manifold. Such reasons may include but need not be limited to the observation that serotypes differ in the route of infection or make use of different receptor molecules or internalisation pathways or that a serotype can infect many tissues/organs but it can only replicate in one organ because of the requirement of certain cellular factors for replication. As mentioned before, it is presently unknown which mechanisms are responsible for the observed differences in human disease association.

One of the problems associated with the development of effective Gene Therapy protocols for the treatment of disease is the limitation of the current vectors to effectively transduce cells *in vivo.* One of the most effective ways to deliver foreign genetic material to cells *in vivo* is through the use of adenovirus vectors. Although, the vector system is very efficient the current adenovirus vector technology has its limitation. Specifically were certain cell types need to be transduced that are normally not very efficiently transduced by Adenovirus 2 or 5. Examples of such relatively resistant cell types include endothelial cells, smooth muscle cells, dendritic cells, neuronal cells, glial cells, synovical cells, primary fibroblasts, cells from the amniotic fluid, hemopoietic stem cells, and monocytic/ macrophage cells etc. Thus in one aspect the invention provides a method for delivering a nucleic acid of interest to a host cell by means of a gene delivery vehicle based on adenoviral material, whereby said gene delivery vehicle delivers the nucleic acid to the host cell by associating with a binding site and/or a receptor present on CAR-negative cells, said binding site and/or receptor being a binding site and/or a receptor for adenovirus subgroups D and/or F. The method may advantageously be used to efficiently transduce cells both *in vitro* and *in vivo.*

The present invention was made during research with chimaeric adenoviruses. Said chimaeric adenoviruses comprising capsids derived from adenovirus 5 of which at least part of the adenovirus 5 fiber protein was replaced by a fiber protein from a different adenovirus serotype. It was observed that chimaeric adenoviruses comprising fiber protein from adenovirus serotypes belonging to subgroup D or subgroup F were capable of efficiently transducing CAR negative target cells.

Adenovirus 2 and 5 belong to adenovirus subgroup C. Together with the adenoviruses of subgroups A, D-F, the subgroup C adenoviruses were before the present invention thought to attach to cells via the Coxsacki adenovirus receptor (CAR) (Roelvink et al, 1998).

It has been shown that adenoviruses of subgroup B such as Ad3 bind to a different receptor than CAR (Defer et al, 1990). Likewise, it was demonstrated that receptor specificity could be altered by exchanging the Ad3 with the Ad 5 knob protein, and vice versa (Krasnykh et al, 1996; Stevenson et al, 1995, 1997).

A host cell may be any host cell as long at it comprises a binding site and/or a receptor present on CAR-negative cells, said binding site and/or receptor being a binding site and/or a receptor for adenovirus subgroups D and/or F. Preferably, said cell is a human cell. Said cell may be a cell present in a culture dish or be part of a whole organism.

Preferably said CAR-negative cells are hemopoietic cells or amniotic fluid cells or derivatives thereof. Preferably, said CAR-negative hemopoietic cells are K562 cells. Preferably, said CAR-negative amniotic fluid cells are amniotic villi or chorion villi cells or derivatives thereof.

A gene delivery vehicle according to the invention may be any vehicle capable of transferring nucleic acid into cells. Preferably, said gene delivery vehicle is a viral vector particle, more preferably said gene delivery vehicle is an adenoviral vector particle. The word gene in the term gene delivery vehicle does not reflect a situation wherein always an entire gene is delivered by said vehicle. The word gene in this respect merely reflects the presence of a nucleic acid of interest. Said nucleic acid may comprise an entire gene, an artificial sequence, a recombinant nucleic acid, a protein coding domain, a cDNA, a sequence coding for anti-sense RNA, mRNA and/or other kind of nucleic acid.

Suitable adenovirus material may comprise an adenovirus capsid or a functional part, derivative and/or analogue thereof. Said adenovirus capsid preferably comprises an adenovirus subgroup D or subgroup F capsid, or a functional part, derivative and/or analogue thereof. Said adenovirus capsid may also be a chimaeric capsid comprising proteins or parts thereof from at least two different adenovirus serotypes or derivatives and/or analogues thereof. Preferably, at least part of a fiber protein of said chimaeric capsid is derived from an adenovirus of subgroup D and/or subgroup F or a functional derivative and/or analogue thereof. Preferably, capsid proteins other then said part of a fiber protein, are derived from an adenovirus of subgroup C, preferably of adenovirus 5 or adenovirus 2. Suitable derivatives of said adenovirus capsids may, among other, be obtained through so-called silent amino-acid substitution in one or more capsid proteins.

Preferably, said adenovirus material comprises at least part of an adenovirus fiber protein. Preferably, said adenovirus fiber protein is derived from an adenovirus of subgroup D or subgroup F or a functional part, derivative and/or analogue thereof. Preferably, said part of a fiber protein is a part involved in binding to a receptor and/or a binding site on a target cell. Typically, but not necessarily said part of an adenovirus fiber protein involved in binding to a receptor and/or a binding site on a target cell is a part of the knob. Adenovirus fiber protein comprises at least three functional regions. One region, the base, is responsible for anchoring the fiber to a penton base of the adenovirus capsid. Another region, the knob, is typically associated with receptor recognition whereas the shaft region functions as a spacer separating the base from the knob. Various regions may also have other functions. For instance, the shaft is presumably also involved in target cell specificity. Each of the regions mentioned above may be used to define a part of a fiber. However, regions of a fiber may also be identified in another way. For instance the knob region comprises of a receptor binding region and a shaft binding region. The base region comprises of a penton base binding region and a shaft binding region. Moreover, the shaft comprises of repeated stretches of amino acids. Each of these repeated stretches may be a part.
A receptor and/or binding site binding part of a fiber protein may be a single region of a fiber protein or a functional part thereof, or a combination of regions or parts thereof of at least one fiber protein, wherein said receptor and/or binding site binding part of a fiber protein, either alone or in combination with one or more other proteins of a adenovirus capsid, determines the efficiency with which a gene delivery vehicle can transduce a given cell or cell type, preferably but not necessarily in a positive way. Needless to say that said fiber and/or a capsid may comprise further modifications to adapt the fiber protein and/or the capsid to specific other needs, which a person skilled in the art will be capable of doing.

A receptor and/or a binding site for adenovirus subgroups D and/or F may be any kind of molecule capable of associating with an adenovirus of subgroup D and/or F. In and/or on the surface of a cell, said receptor and/or binding site must be able to associate with said adenovirus of subgroup D and/or F provided to said cell. Said receptor and/or binding site may be part of a complex present in and/or on said cell. Said receptor and/or binding site does not need to be able to associate with an adenovirus of subgroup D and/or F all the time as long as it is capable of doing so some of the time. Said receptor and/or binding site may further also be a receptor and/or binding site for another virus and/or gene delivery vehicle, although this does not have to be so. A person skilled in the art may want to determine whether an adenovirus serotype belonging to another subgroup than D and/or F can also utilise the receptor and/or binding site for adenovirus subgroups D and/or F.

In another aspect the invention provides the use of a gene delivery vehicle comprising a nucleic acid of interest and comprising adenoviral material involved in binding to a host cell, said material being from a subgroup D and/or F adenovirus, in delivering said nucleic acid of interest to a CAR-negative cell. With the knowledge of a novel pathway for the transduction of cells using adenovirus material it becomes possible to approach this novel pathway also through other means then said material derived from a subgroup D and/or F. A person skilled in the art recognises this and will be able to devise means to accomplish this for instance through the use of antibodies directed toward a crucial component of said pathway, together with a membrane fusion peptide. Such means and methods are also within the scope of the invention.

In another aspect the invention provides a gene delivery vehicle being a chimaera based on at least two adenoviruses, whereby a cell recognising element of said gene delivery vehicle is based on adenoviral material from a subgroup D and/or F adenovirus, which material confers the capability of infecting CAR negative cells.

Preferably, said adenoviral material is based on the fiber, penton and/or hexon proteins of a subgroup D and/or subgroup F adenovirus.

To date, six different subgroups of human adenoviruses have been proposed which in total encompasses 51 distinct adenovirus serotypes. A serotype is defined on the basis of its immunological distinctiveness as determined by quantitative neutralisation with animal antisera (horse, rabbit). If neutralisation shows a certain degree of cross-reaction between two viruses, distinctiveness of serotype is assumed if A) the hemagglutinins are unrelated, as shown by lack of cross-reaction on hemagglutination-inhibition, or B) substantial biophysical/ biochemical differences in DNA exist (Francki et al, 1991). The nine serotypes identified last (42-51) were isolated for the first time from HIV-infected patients (Hierholzer et al 1988; Schnurr et al 1993;). For reasons not well understood, most of such immune-compromised patients shed adenoviruses that were rarely or never isolated from immune-competent individuals (Hierholzer et al 1988, 1992; Khoo et al, 1995, De Jong et al, 1998). The usefulness of these adenoviruses or cross-immunising adenoviruses to prepare gene delivery vehicles may be seriously hampered, since the individual to which the gene delivery vehicle is provided, will raise a neutralising response to such a vehicle before long.

There is thus a need in the field of gene therapy to provide gene delivery vehicles, preferably based on adenoviruses, which do not encounter pre-existing immunity and/or which are capable of avoiding or diminishing neutralising antibody responses. Thus preferably, a gene delivery vehicle of the invention further comprises an element from adenovirus 35 or a functional equivalent thereof, responsible for at least partially avoiding an immune response against adenovirus 35. A functional equivalent/homologue of adenovirus 35 (element) for the purpose of the present invention is an adenovirus (element) which, like adenovirus 35, encounters pre-existing immunity in less than about 10% of the hosts, at least in a significant geographic region of the world, to which it is administered for the first time, or which is capable in more than about 90% of the hosts, at least in a significant geographic region of the world, to which it is administered to avoid or diminish the immune response. Typical examples of such adenoviruses are adenovirus serotypes 34, 26 and 48.

In another embodiment a gene delivery vehicle according to the invention comprises an element of adenovirus 16 or a functional equivalent thereof, which element confers said virus with an enhanced capability to infect smooth muscle cells and/or synoviocytes. A functional equivalent of an element of adenovirus 16 in this respect is an element from another subgroup B virus. Preferably, said element is a tissue tropism determining part of a fiber protein. Typically, a tissue tropism determining part of an adenovirus fiber protein is a part that influences the transduction efficiency of a cell.

For Gene Therapeutic purposes one typically does not want an adenovirus batch to be administered to a host cell which contains replication competent adenovirus, although this is not always true. In general therefor it is desired to omit a number of genes (but at least one) from the adenoviral genome on the vector encoding the virus and to supply these genes in the genome of the cell in which the vector is brought to produce adenovirus. Such a cell is usually called a packaging cell. The invention thus also provides a packaging cell for producing an adenovirus according to the invention, comprising *in trans,* all elements necessary for adenovirus production not present on the adenoviral vector according to the invention. Typically vector and packaging cell have to be adapted to one another in that they have all the necessary elements, but that they do not have overlapping elements which lead to replication competent virus by recombination. In a preferred embodiment said packaging cell is, or is derived from PER.C6 (ECCAC deposit number 96022940).

In another embodiment, a gene delivery vehicle according to the invention comprises an adenovirus vector. Said adenovirus vector may be a classical adenovirus vector, a minimal adenovirus vector or an integrating adenovirus such as an Ad/AAV chimaeric vector, a retro-adenovirus or a transposon-adenovirus or yet another different kind of adenovirus vector. With an integrating adenovirus vector for the purpose of the invention is meant a vector comprising nucleic acid derived from an adenovirus and further comprising means for the integration of at least part of the nucleic acid of said vector into the host cell genome. Said means are preferably derived from a nucleic acid with the inherent capacity to integrate into the host cell genome. Such nucleic acid with the inherent capacity to integrate into the host cell genome may derived from a transposon or transposon-like element, a retrovirus and/or an adeno-associated virus or a different virus with the capacity to integrate nucleic acid into the host cell genome.

In a preferred embodiment said adenovirus vector comprises nucleic acid encoding at least a receptor and/or binding site determining part of a fiber protein of an adenovirus of subgroup D or subgroup F. In a preferred embodiment the invention provides a method for producing said adenovirus vector, comprising welding together, preferably through homologous recombination, two nucleic acid molecules comprising partially overlapping sequences wherein said overlapping sequences allow essentially only one homologous recombination which leads to the generation of a physically linked nucleic acid comprising at least two functional adenovirus inverted terminal repeats, a functional encapsulation signal and a nucleic acid of interest or functional parts, derivatives and/or analogues thereof. In a preferred embodiment at least one of said at least two nucleic acid molecules comprises nucleic acid encoding at least a receptor and/or binding site determining part of a fiber protein of an adenovirus of subgroup D or subgroup F. An important aspect in this embodiment of the invention is that said partially overlapping sequences allow essentially only homologous recombination leading to the generation of a functional adenovirus vector capable of being replicated and packaged into adenovirus particles in the presence of the required transacting functions. With essentially only one is meant that said overlapping sequences in each nucleic acid comprise essentially only one continuous sequence wherein homologous recombination leading to the generation of a functional adenovirus may occur. Within said continuous sequence the actual number of homologous recombination events may be higher than one. Non continuous overlapping sequences are not desired because they reduce the reliability of said method. Non continuous overlapping sequences are also not desired because they reduce the overall efficiency of said method, presumably due to the generation of undesired homologous recombination products.

A preferred embodiment of the invention provides a method for generating an adenovirus vector wherein both of said nucleic acid molecules comprise only one adenovirus inverted terminal repeat or a functional part, derivative and/or analogue thereof. In one aspect one or both of said two nucleic acid molecules have undergone modifications prior to said welding together. Said modification may include the welding together of different nucleic acid molecules leading to the generation of one or both of said two nucleic acid molecules. In a preferred embodiment said different nucleic acids are welded together through homologous recombination of partially overlapping sequences. In a further aspect said welding together is performed in a cell or a functional part, derivative and/or analogue thereof. Preferably said cell is a mammalian cell. More preferably, said welding together is performed in a cell expressing E1-region encoded proteins. Preferably said cell is a PER.C6 cell (ECACC deposit number 96022940) or a derivative thereof. In a preferred embodiment said nucleic acid molecules are not capable of replicating in said mammalian cell prior to said welding together. Said replication is undesired since it reduces the reliability of the methods of the invention presumably through providing additional targets for undesired homologous recombination. Said replication is also not desired because it reduces the efficiency of the methods of the invention presumably because said replication competes for substrate or adenovirus transacting functions with the replication of said adenovirus vector.

In a preferred embodiment, one of said nucleic acid molecules is relatively small and the other is relatively large. This configuration is advantageous because it allows easy manipulation of said relatively small nucleic acid molecule allowing for example the generation of a large number of small nucleic acid molecules comprising different nucleic acid of interest for instance for the generation of an adenovirus vector library. Said configuration is also desired because it allows the production of a large batch of quality tested large nucleic acid molecule. The amplification of large nucleic acid molecules for instance in bacteria is difficult in terms of obtaining sufficient amounts of said large nucleic acid. The amplification of large nucleic acid molecules for instance in bacteria is also difficult to control because a small modification of said large nucleic acid is not easily detected. Moreover, for reasons not quite understood some large vectors are more stable in bacteria or yeasts than others. Said configuration however, allows the generation of a standard batch of a large nucleic acid molecule which can be thoroughly tested, for instance through generating a control adenovirus of which the efficiency and the reliability of production is known, and determining said parameters of a new batch of large nucleic acid molecule. Once validated said batch may be used for the generation of a large number of different adenovirus vectors through combining said large molecule with a large number of different small nucleic acid molecules. Said system therefore also allows for the selection and/or manipulation of vectors comprising a large nucleic acid molecule of the invention to allow a suitable yield of intact large nucleic acid.

In another embodiment said cell comprising nucleic acid encoding E1-region proteins further comprises a nucleic acid encoding an adenovirus E2-region and/or an adenovirus E4-region protein. Preferably, said cell further comprising nucleic acid encoding an adenovirus E2-region and/or an adenovirus E4-region protein is a derivative of PER.C6.

In another aspect the invention provides a receptor and/or a binding site for adenoviruses type D and/or F, present on or associated with CAR negative cells. Preferably said receptor and/or a binding site is present on K562 cells, amniotic fluid derived cells and/or primary fibroblast cells.

In yet another aspect, the invention provides the use of receptor and/or a binding site for adenoviruses type D and/or F, present in and/or on a cell, for the delivery nucleic acid to said cell.

In yet another embodiment the invention provides the use of a gene delivery vehicle according to anyone of claims 1-14, in a pharmaceutical.

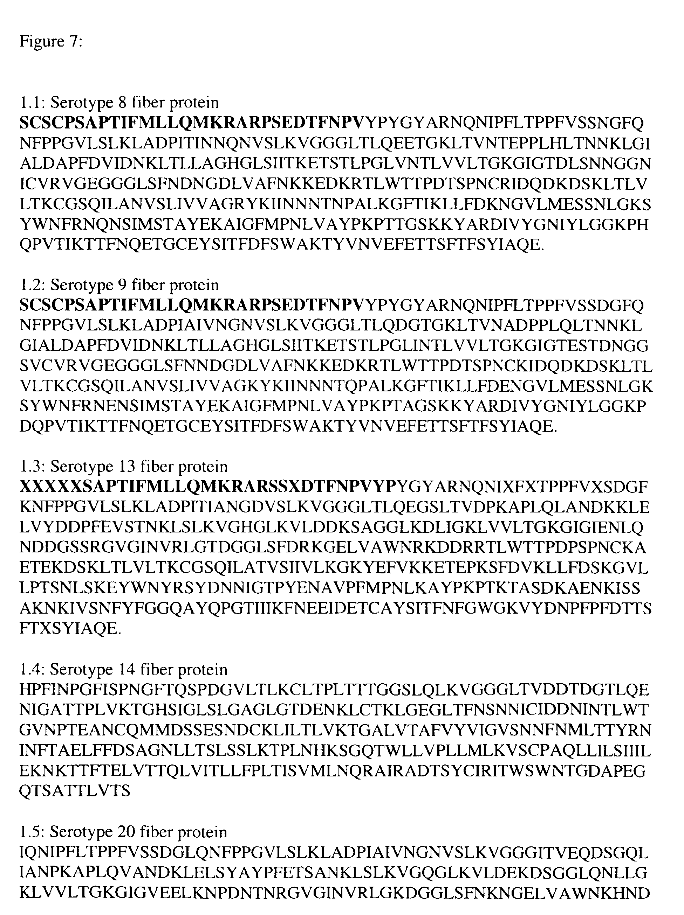

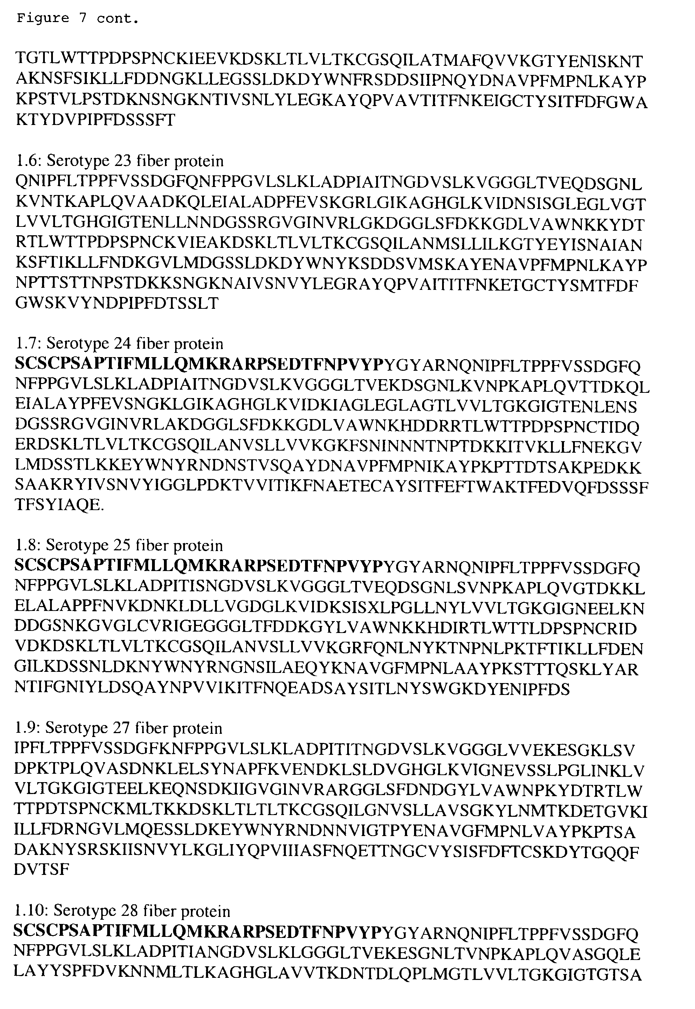

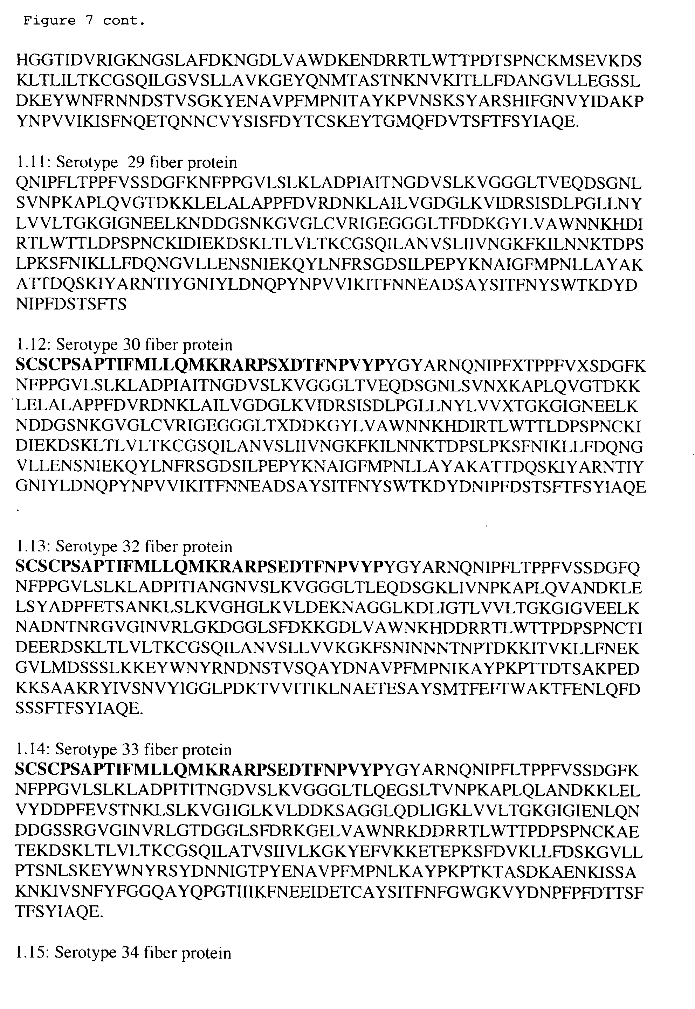

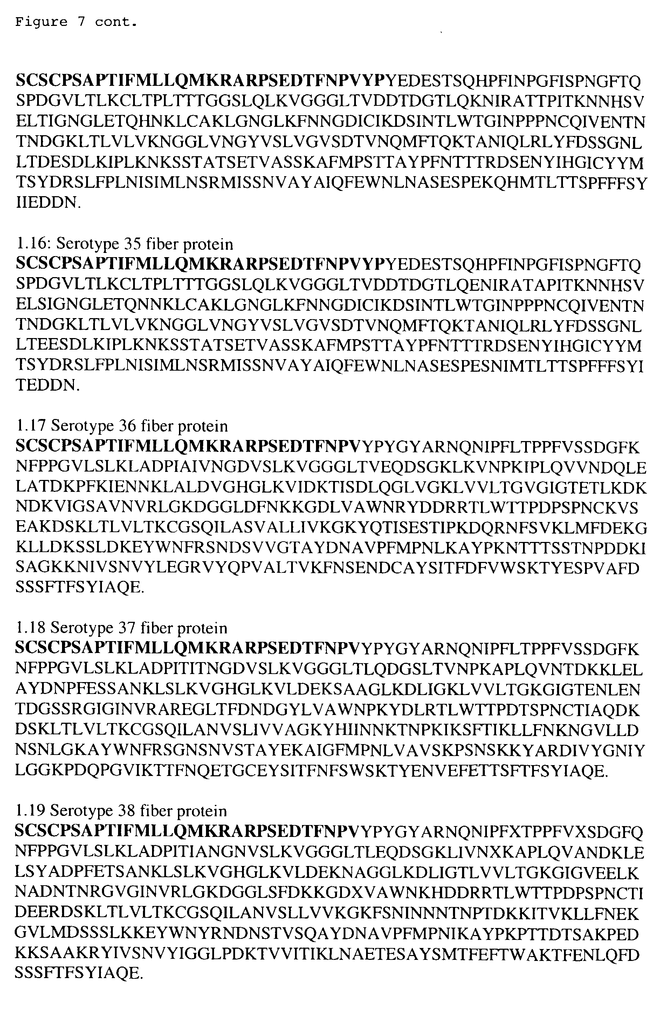

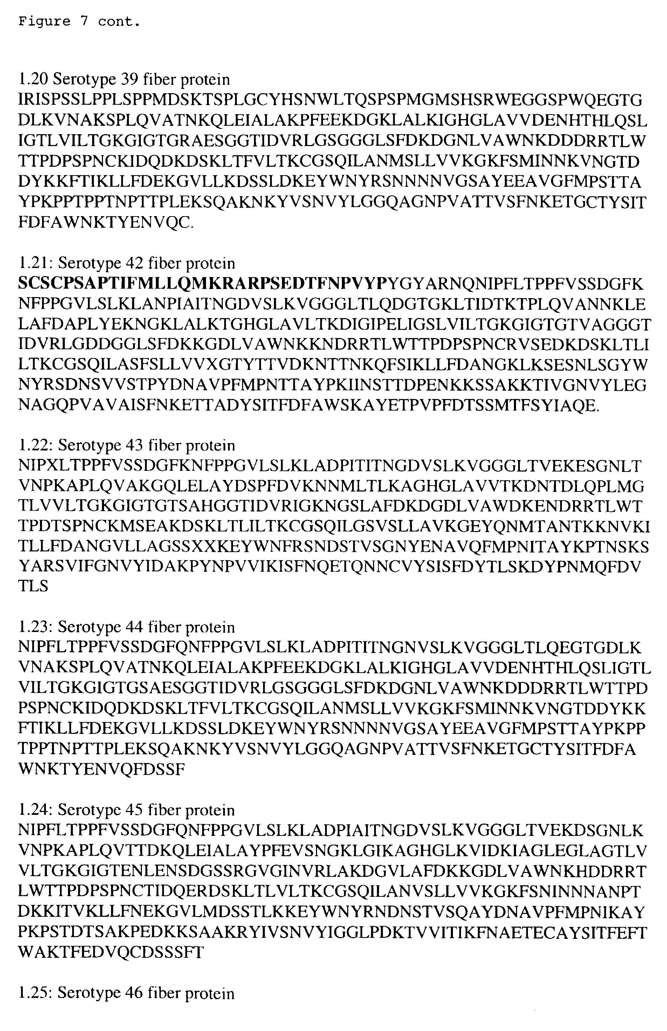

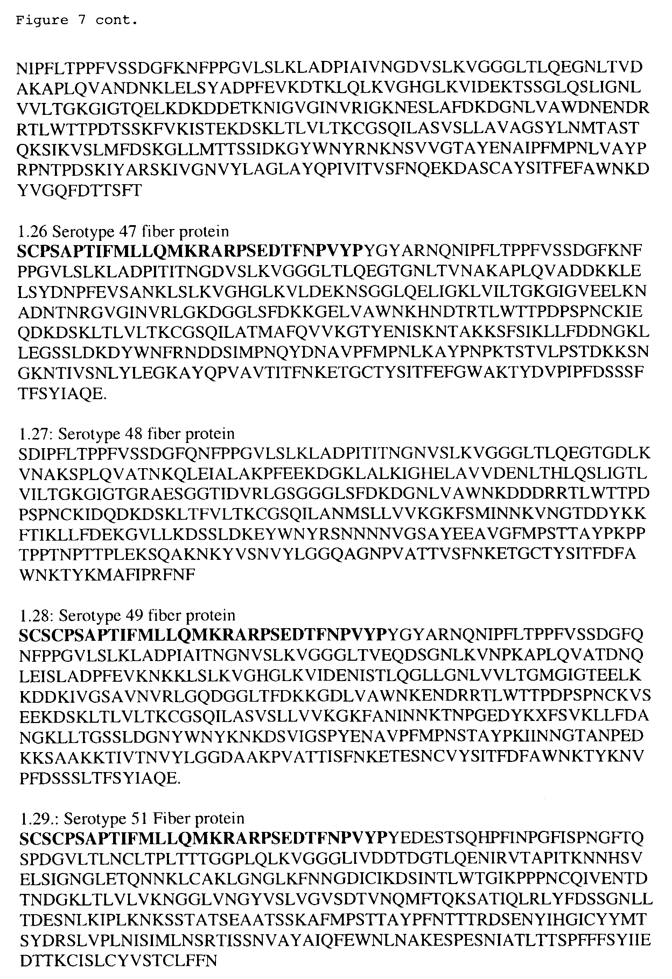

In another aspect the invention provides a capsid protein derived from a subgroup D and/or a subgroup F adenovirus or a functional part, derivative and/or analogue thereof. Preferably, said protein is a fiber protein. The invention further provides a nucleic acid encoding a capsid protein of the invention. Preferably, said nucleic acid comprises a fiber sequence from a subgroup D and/or a subgroup F as depicted in figure 7.

### Detailed description.

It has been demonstrated in mice that upon *in vivo* systemic delivery of recombinant adenovirus serotype 5 for gene therapy purposes approximately 99% of the virus is trapped in the liver (Herz et al, 1993). Therefore, alteration of the adenovirus serotype 5 host cell range to be able to target other organs *in vivo* is a major interest of the invention.

The initial step for successful infection is binding of adenovirus to its target cell, a process generally thought to be mediated through fiber protein. The fiber protein has a trimeric structure (Stouten et al, 1992) with different lengths depending on the virus serotype (Signas et al 1985; Kidd et all 1993). Different serotypes have polypeptides with structurally similar N and C termini, but different middle stem regions. N-terminally, the first 30 amino acids are involved in anchoring of the fiber to the penton base (Chroboczek et al, 1995), especially the conserved FNPVYP region in the tail (Arnberg et al 1997). The C-terminus, or knob, is generally thought to be responsible for initial interaction with the cellular adenovirus receptor. After this initial binding secondary binding between the capsid penton base and cell-surface integrins is proposed to lead to internalisation of viral particles in coated pits and endocytosis (Morgan et al, 1969; Svensson et al, 1984; Varga et al, 1992; Greber et al, 1993; Wickham et al, 1994).

Integrins are αβ-heterodimers of which at least 14 α-subunits and 8 β-subunits have been identified (Hynes et al, 1992). The array of integrins expressed in cells is complex and will vary between cell types and cellular environment. Although the knob contains some conserved regions, between serotypes, knob proteins show a high degree of variability, indicating that different adenovirus receptors might exist. For instance, it has been demonstrated that adenoviruses of subgroup C (Ad2, Ad5) and adenoviruses of subgroup B (Ad3) bind to different receptors (Defner et al, 1990). By using baculovirus produced soluble CAR as well as adenovirus serotype 5 knob protein, Roelvink et all concluded via interference studies that all adenovirus serotypes, except serotypes of subgroup B, enter cells via CAR (Roelvink et al, 1998). The latter, which is now generally accepted in the field, if valid should thus limit the complexity of using different serotypes for gene therapy purposes.

Besides the involvement in cell binding, the fiber protein also contains the type specific γ-antigen, which together with the ε-antigen of the hexon determines the serotype specificity. The γ-antigen is localised on the fiber and it is known that it consists of 17 amino acids (Eiz et al, 1997). The anti-fiber antibodies of the host are therefore directed to the trimeric structure of the knob. To obtain re-directed infection of recombinant adenovirus serotype 5, several approaches have been or still are under investigation. Wickham et al has altered the RGD (Arg, Gly, Asp) motif in the penton base which is believed to be responsible for the αᵥβ₃ and αᵥβ₅ integrin binding to the penton base. They have replaced this RGD motif by another peptide motif which is specific for the α₄β₁ receptor. In this way targeting the adenovirus to a specific target cell could be accomplished (Wickham et al, 1995, 1996). Krasnykh et al has made use of the HI loop available in the knob. This loop is, based on X-ray crystallographics, located on the outside of the knob trimeric structure and therefore is thought not to contribute to the intramolecular interactions in the knob (Krasnykh et al, 1998). However, complete CAR independent infection was not observed.

It is an object of the present invention to provide a method and means by which an adenovirus can infect cells negative for the CAR protein. Therefore, the generation of chimaeric adenoviruses based on adenovirus serotype 5 with a modified fiber gene is described. For this purpose, two or three plasmids, which together contain the complete adenovirus serotype 5 genome, were constructed. From a plasmid the DNA encoding the adenovirus serotype 5 fiber protein was essentially removed and replaced by linker DNA sequences which facilitate easy cloning. This plasmid subsequently served as template for the insertion of DNA encoding for fiber protein derived from different adenovirus serotypes (human or animal). The DNAs derived from the different serotypes were obtained using the polymerase chain reaction technique in combination with (degenerate) oligonucleotides. At the former E1 location in the genome of adenovirus serotype 5, any gene of interest can be cloned. A single transfection procedure of the two or three plasmids together resulted in the formation of a recombinant chimaeric adenovirus. Although successful introduction of changes in the adenovirus serotype 5 fiber and penton-base have been reported, the complex structure of knob and the limited knowledge of the precise amino acids interacting with CAR render such targeting approaches laborious and difficult. To overcome the limitations described above we used pre-existing adenovirus fibers to maximise the chance of obtaining recombinant adenovirus which can normally assemble in the nucleus of a producer cell and which can be produced on pre-existing packaging cells. By generating a chimaeric adenovirus serotype 5 based fiber library containing fiber proteins of all other human adenovirus serotypes, we have developed a technology which enables rapid screening for a recombinant adenoviral vector with preferred infection characteristics.

In one aspect this invention describes chimaeric adenoviruses and methods to generate these viruses that have an tropism different from that of adenovirus serotype 5. This chimaeric adenovirus serotype 5 is able to infect cell types which do not express the CAR protein much more efficiently both *in vitro* and *in vivo* than the adenovirus serotype 5. Such cells include but are not limited to endothelial cells, smooth muscle cells, dendritic cells, neuronal cells, glial cells, synovical cells, primary fibroblasts, cells from the amniotic fluid, hemopoietic stem cells, and monocytic/ macrophage cells etc.

In another aspect the invention describes the construction and use of plasmids consisting of distinct parts of adenovirus serotype 5 in which the gene encoding for fiber protein has been replaced with DNA derived from alternative human or animal serotypes. This set of constructs, in total encompassing the complete adenovirus genome, allows for the construction of unique chimaeric adenoviruses customised for transduction of particular cell types or organ(s).

In all aspects of the invention the chimaeric adenoviruses may, or may not, contain deletions in the E1 region and insertions of heterologous genes linked either or not to a promoter. Furthermore, chimaeric adenoviruses may, or may not, contain deletions in the E3 region and insertions of heterologous genes linked to a promoter. Furthermore, chimaeric adenoviruses may, or may not, contain deletions in the E2 and/ or E4 region and insertions of heterologous genes linked to a promoter. In the latter case E2 and/ or E4 complementing cell lines are required to generated recombinant adenoviruses.

### Example 1: Generation of adenovirus serotype 5 genomic plasmid clones

The complete genome of adenovirus serotype 5 has been cloned into various plasmids or cosmids to allow easy modification of parts of the adenovirus serotype 5 genome, while still retaining the capability to produce recombinant virus. For this purpose the following plasmids were generated:

### 1. pBr/Ad.Bam-rITR (ECACC deposit P97082122)

In order to facilitate blunt end cloning of the ITR sequences, wild-type human adenovirus type 5 (Ad5) DNA was treated with Klenow enzyme in the presence of excess dNTPs. After inactivation of the Klenow enzyme and purification by phenol/chloroform extraction followed by ethanol precipitation, the DNA was digested with BamHI. This DNA preparation was used without further purification in a ligation reaction with pBr322 derived vector DNA prepared as follows: pBr322 DNA was digested with EcoRV and BamHI, dephosphorylated by treatment with TSAP enzyme (Life Technologies) and purified on LMP agarose gel (SeaPlaque GTG). After transformation into competent *E.coli* DH5a (Life Techn.) and analysis of ampicilline resistant colonies, one clone was selected that showed a digestion pattern as expected for an insert extending from the BamHI site in Ad5 to the right ITR.
Sequence analysis of the cloning border at the right ITR revealed that the most 3' G residue of the ITR was missing, the remainder of the ITR was found to be correct. Said missing G residue is complemented by the other ITR during replication.

### 2. pBr/Ad.Sal-rITR (ECACC deposit P97082119)

pBr/Ad.Bam-rITR was digested with BamHI and SalI. The vector fragment including the adenovirus insert was isolated in LMP agarose (SeaPlaque GTG) and ligated to a 4.8 kb SalI-BamHI fragment obtained from wt Ad5 DNA and purified with the Geneclean II kit (Bio 101, Inc.). One clone was chosen and the integrity of the Ad5 sequences was determined by restriction enzyme analysis. Clone pBr/Ad.Sal-rITR contains adeno type 5 sequences from the SalI site at bp 16746 up to and including the rITR (missing the most 3' G residue).

### 3. pBr/Ad.Cla-Bam (ECACC deposit P97082117)

wt Adeno type 5 DNA was digested with ClaI and BamHI, and the 20.6 kb fragment was isolated from gel by electro-elution. pBr322 was digested with the same enzymes and purified from agarose gel by Geneclean. Both fragments were ligated and transformed into competent DH5a. The resulting clone pBr/Ad.Cla-Bam was analysed by restriction enzyme digestion and shown to contain an insert with adenovirus sequences from bp 919 to 21566.

### 4. pBr/Ad.AflII-Bam (ECACC deposit P97082114)

Clone pBr/Ad.Cla-Bam was linearised with EcoRI (in pBr322) and partially digested with AflII. After heat inactivation of AflII for 20' at 65°C the fragment ends were filled in with Klenow enzyme. The DNA was then ligated to a blunt double stranded oligo linker containing a PacI site (5'-AATTGTCTTAATTAACCGCTTAA-3'). This linker was made by annealing the following two oligonucleotides: 5'-AATTGTCTTAATTAACCGC-3' and 5'-AATTGCGGTTAATTAAGAC-3', followed by blunting with Klenow enzyme. After precipitation of the ligated DNA to change buffer, the ligations were digested with an excess PacI enzyme to remove concatameres of the oligo. The 22016 bp partial fragment containing Ad5 sequences from bp 3534 up to 21566 and the vector sequences, was isolated in LMP agarose (SeaPlaque GTG), religated and transformed into competent DH5a. One clone that was found to contain the PacI site and that had retained the large adeno fragment was selected and sequenced at the 5' end to verify correct insertion of the PacI linker in the (lost) AflII site.

### 5. pBr/Ad.Bam-rITRpac#2 (ECACC deposit P97082120) and pBr/Ad.Sam-rITR#8 (ECACC deposit P97082121)

To allow insertion of a PacI site near the ITR of Ad5 in clone pBr/Ad.Bam-rITR about 190 nucleotides were removed between the ClaI site in the pBr322 backbone and the start of the ITR sequences. This was done as follows: pBr/Ad.Bam-rITR was digested with ClaI and treated with nuclease Bal31 for varying lengths of time (2', 5', 10' and 15'). The extent of nucleotide removal was followed by separate reactions on pBr322 DNA (also digested at the ClaI site), using identical buffers and conditions. Bal31 enzyme was inactivated by incubation at 75°C for 10 minutes, the DNA was precipitated and resuspended in a smaller volume of TE buffer. To ensure blunt ends, DNAs were further treated with T4 DNA polymerase in the presence of excess dNTPs. After digestion of the (control) pBr322 DNA with SalI, satisfactory degradation (^{∼}150 bp) was observed in the samples treated for 10' or 15'. The 10' or 15' treated pBr/Ad.Bam-rITR samples were then ligated to the above described blunted PacI linkers (See pBr/Ad.AflII-Bam). Ligations were purified by precipitation, digested with excess PacI and separated from the linkers on an LMP agarose gel. After religation, DNAs were transformed into competent DH5a and colonies analysed. Ten clones were selected that showed a deletion of approximately the desired length and these were further analysed by T-track sequencing (T7 sequencing kit, Pharmacia Biotech). Two clones were found with the PacI linker inserted just downstream of the rITR. After digestion with PacI, clone #2 has 28 bp and clone #8 has 27 bp attached to the ITR.

### pWE/Ad.AflII-rITR (ECACC deposit P97082116)

Cosmid vector pWE15 (Clontech) was used to clone larger Ad5 inserts. First, a linker containing a unique PacI site was inserted in the EcoRI sites of pWE15 creating pWE.pac. To this end, the double stranded PacI oligo as described for pBr/Ad.AflII-BamHI was used but now with its EcoRI protruding ends. The following fragments were then isolated by electro-elution from agarose gel: pWE.pac digested with PacI, pBr/AflII-Bam digested with PacI and BamHI and pBr/Ad.Bam-rITR#2 digested with BamHI and PacI. These fragments were ligated together and packaged using 1 phage packaging extracts (Stratagene) according to the manufacturers protocol. After infection into host bacteria, colonies were grown on plates and analysed for presence of the complete insert. pWE/Ad.AflII-rITR contains all adenovirus type 5 sequences from bp 3534 (AflII site) up to and including the right ITR (missing the most 3' G residue).

### pBr/Ad.lITR-Sal(9.4) (ECACC deposit P97082115)

Adeno 5 wt DNA was treated with Klenow enzyme in the presence of excess dNTPs and subsequently digested with SalI. Two of the resulting fragments, designated left ITR-Sal(9.4) and Sal(16.7)-right ITR, respectively, were isolated in LMP agarose (Seaplaque GTG). pBr322 DNA was digested with EcoRV and SalI and treated with phosphatase (Life Technologies). The vector fragment was isolated using the Geneclean method (BIO 101, Inc.) and ligated to the Ad5 SalI fragments. Only the ligation with the 9.4 kb fragment gave colonies with an insert. After analysis and sequencing of the cloning border a clone was chosen that contained the full ITR sequence and extended to the SalI site at bp 9462.

### pBr/Ad.lITR-Sal(16.7) (ECACC deposit P97082118)

pBr/Ad.lITR-Sal(9.4) is digested with SalI and dephosphorylated (TSAP, Life Technologies). To extend this clone up to the third SalI site in Ad5, pBr/Ad.Cla-Bam was linearised with BamHI and partially digested with SalI. A 7.3 kb SalI fragment containing adenovirus sequences from 9462-16746 was isolated in LMP agarose gel and ligated to the SalI-digested pBr/Ad.lITR-Sal (9.4) vector fragment.

### pWE/Ad.AflII-EcoRI

pWE.pac was digested with ClaI and 5' protruding ends were filled using Klenow enzyme. The DNA was then digested with PacI and isolated from agarose gel. pWE/AflII-rITR was digested with EcoRI and after treatment with Klenow enzyme digested with PacI. The large 24 kb fragment containing the adenoviral sequences was isolated from agarose gel and ligated to the ClaI-digested and blunted pWE.pac vector using the Ligation Express™ kit from Clontech. After transformation of Ultracompetent XL10-Gold cells from Stratagene, clones were identified that contained the expected insert. pWE/AflII-EcoRI contains Ad5 sequences from bp 3534-27336.

### Construction of new adapter plasmids

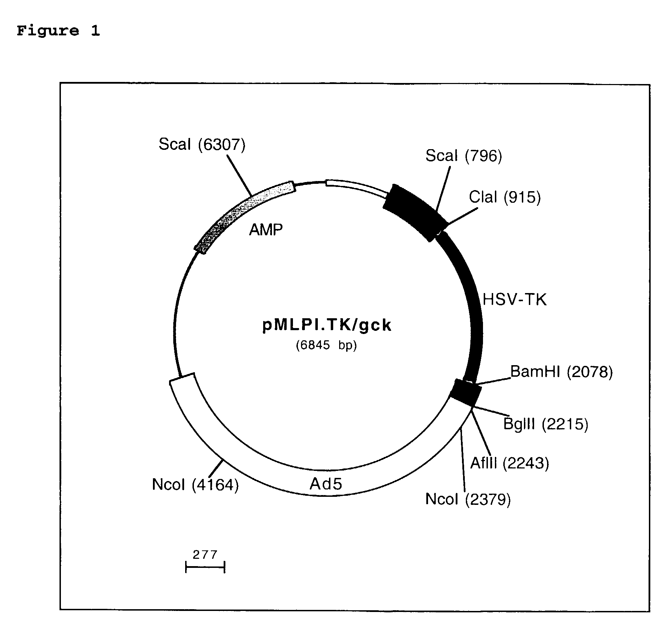

The absence of sequence overlap between the recombinant adenovirus and E1 sequences in the packaging cell line is essential for safe, RCA-free generation and propagation of new recombinant viruses. The adapter plasmid pMLPI.TK (figure. 1) is an example of an adapter plasmid designed for use according to the invention in combination with the improved packaging cell lines of the invention. This plasmid was used as the starting material to make a new vector in which nucleic acid molecules comprising specific promoter and gene sequences can be easily exchanged.

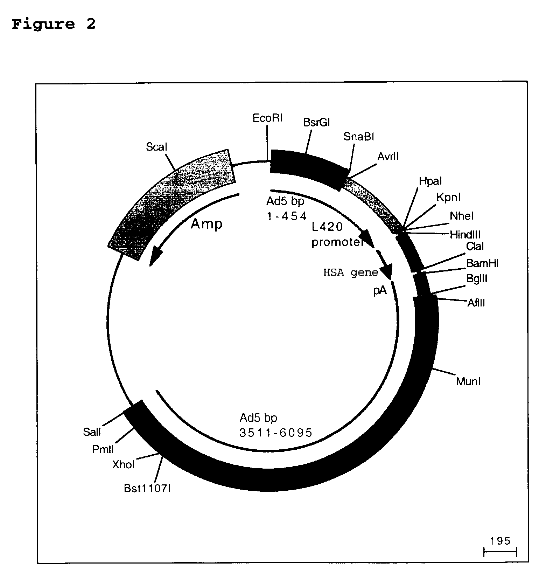

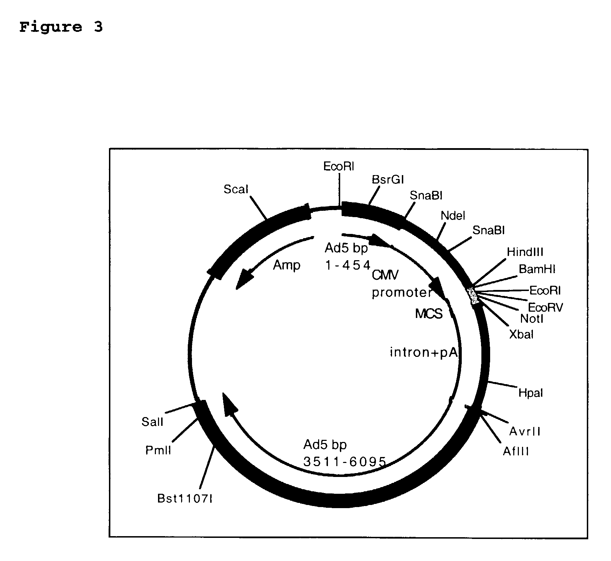

First, a PCR fragment was generated from pZipΔMo+PyF101(N⁻) template DNA (described in PCT/NL96/00195) with the following primers: LTR-1: 5'-CTG TAC GTA CCA GTG CAC TGG CCT AGG CAT GGA AAA ATA CAT AAC TG-3' and LTR-2: 5'-GCG GAT CCT TCG AAC CAT GGT AAG CTT GGT ACC GCT AGC GTT AAC CGG GCG ACT CAG TCA ATC G-3'. Pwo DNA polymerase (Boehringer Mannheim) was used according to manufacturers protocol with the following temperature cycles: once 5' at 95°C; 3' at 55°C; and 1' at 72°C, and 30 cycles of 1' at 95°C, 1' at 60°C, 1' at 72°C, followed by once 10' at 72°C. The PCR product was then digested with BamHI and ligated into pMLP10 (Levrero *et al*., 1991) vector digested with PvuII and BamHI, thereby generating vector pLTR10. This vector contains adenoviral sequences from bp 1 up to bp 454 followed by a promoter consisting of a part of the Mo-MuLV LTR having its wild-type enhancer sequences replaced by the enhancer from a mutant polyoma virus (PyF101). The promoter fragment was designated L420. Next, the coding region of the murine HSA gene was inserted. pLTR10 was digested with BstBI followed by Klenow treatment and digestion with NcoI. The HSA gene was obtained by PCR amplification on pUC18-HSA (Kay *et al.,* 1990) using the following primers: HSA1, 5'-GCG CCA CCA TGG GCA GAG CGA TGG TGG C-3' and HSA2, 5'-GTT AGA TCT AAG CTT GTC GAC ATC GAT CTA CTA ACA GTA GAG ATG TAG AA-3'. The 269 bp amplified fragment was subcloned in a shuttle vector using the NcoI and BglII sites. Sequencing confirmed incorporation of the correct coding sequence of the HSA gene, but with an extra TAG insertion directly following the TAG stop codon. The coding region of the HSA gene, including the TAG duplication was then excised as a NcoI (sticky)-SalI (blunt) fragment and cloned into the 3.5 kb NcoI(sticky)/BstBI(blunt) fragment from pLTR10, resulting in pLTR-HSA10.
Finally, pLTR-HSA10 was digested with EcoRI and BamHI after which the fragment containing the left ITR, packaging signal, L420 promoter and HSA gene was inserted into vector pMLPI.TK digested with the same enzymes and thereby replacing the promoter and gene sequences. This resulted in the new adapter plasmid pAd/L420-HSA (figure. 2) that contains convenient recognition sites for various restriction enzymes around the promoter and gene sequences. SnaBI and AvrII can be combined with HpaI, NheI, KpnI, HindIII to exchange promoter sequences, while the latter sites can be combined with the ClaI or BamHI sites 3' from HSA coding region to replace genes in this construct.
Another adapter plasmid that was designed to allow easy exchange of nucleic acid molecules was made by replacing the promoter, gene and poly A sequences in pAd/L420-HSA with the CMV promoter, a multiple cloning site, an intron and a poly-A signal. For this purpose, pAd/L420-HSA was digested with AvrII and BglII followed by treatment with Klenow to obtain blunt ends. The 5.1 kb fragment with pBr322 vector and adenoviral sequences was isolated and ligated to a blunt 1570 bp fragment from pcDNA1/amp (Invitrogen) obtained by digestion with HhaI and AvrII followed by treatment with T4 DNA polymerase. This adapter plasmid was named pCLIP (figure. 3).

### Generation of recombinant adenoviruses

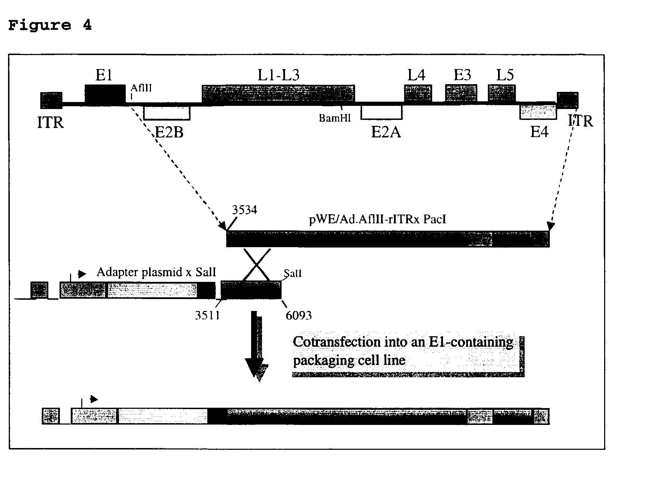

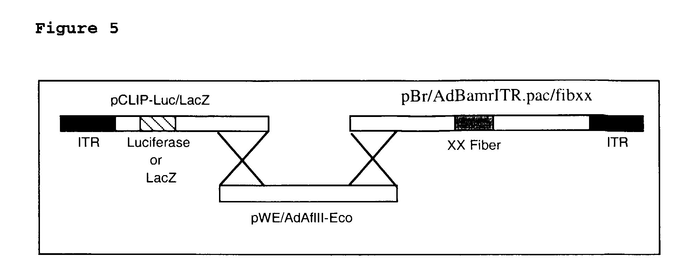

To generate E1 deleted recombinant adenoviruses with the new plasmid-based system, the following constructs are prepared:
a) An adapter construct containing the expression cassette with the gene of interest linearised with a restriction enzyme that cuts at the 3' side of the overlapping adenoviral genome fragment, preferably not containing any pBr322 vector sequences, and
b) A complementing adenoviral genome construct pWE/Ad.AflII-rITR digested with PacI.
These two DNA molecules are further purified by phenol/ chloroform extraction and EtOH precipitation. Co-transfection of these plasmids into an adenovirus packaging cell line, preferably a cell line according to the invention, generates recombinant replication deficient adenoviruses by a one-step homologous recombination between the adapter and the complementing construct (figure. 4) .
Alternatively, in stead of pWE/Ad.AflII-rITR other fragments can be used, e.g., pBr/Ad.Cla-Bam digested with EcoRI and BamHI or pBr/Ad.AflII-BamHI digested with PacI and BamHI can be combined with pBr/Ad.Sal-rITR digested with SalI. In this case, three plasmids are combined and two homologous recombinations are needed to obtain a recombinant adenovirus (figure. 5). It is to be understood that those skilled in the art may use other combinations of adapter and complementing plasmids without departing from the present invention. A general protocol as outlined below and meant as a nonlimiting example of the present invention has been performed to produce several recombinant adenoviruses using various adapter plasmids and the Ad.AflII-rITR fragment. Adenovirus packaging cells (PER.C6) were seeded in ^{∼}25 cm² flasks and the next day when they were at ^{∼}80% confluency, transfected with a mixture of DNA and lipofectamine agent (Life Techn.) as described by the manufacturer. Routinely, 40 µl lipofectamine, 4 µg adapter plasmid and 4 µg of the complementing adenovirus genome fragment AflII- rITR (or 2 µg of all three plasmids for the double homologous recombination) are used. Under these conditions transient transfection efficiencies of ^{∼}50% (48 hrs post transfection) are obtained as determined with control transfections using a pAd/CMV-LacZ adapter. Two days later, cells are passaged to ^{∼} 80 cm² flasks and further cultured. Approximately five (for the single homologous recombination) to eleven days (for the double homologous recombination) later a cytopathogenic effect (CPE) is seen, indicating that functional adenovirus has formed. Cells and medium are harvested upon full CPE and recombinant virus is released by freeze-thawing. An extra amplification step in an 80 cm² flask is routinely performed to increase the yield since at the initial stage the titers are found to be variable despite the occurrence of full CPE. After amplification, viruses are harvested and plaque purified on PER.C6 cells. Individual plaques are tested for viruses with active transgenes.

Besides replacements in the E1 region it is possible to delete or replace (part of) the E3 region in the adenovirus because E3 functions are not necessary for the replication, packaging and infection of the (recombinant) virus. This creates the opportunity to use a larger insert or to insert more than one gene without exceeding the maximum package size (approximately 105% of wt genome length). This can be done, e.g., by deleting part of the E3 region in the pBr/Ad.Bam-rITR clone by digestion with XbaI and religation. This removes Ad5 wt sequences 28592-30470 including all known E3 coding regions. Another example is the precise replacement of the coding region of gpl9K in the E3 region with a polylinker allowing insertion of new sequences. This, 1) leaves all other coding regions intact and 2) obviates the need for a heterologous promoter since the transgene is driven by the E3 promoter and pA sequences, leaving more space for coding sequences.
To this end, the 2.7 kb EcoRI fragment from wt Ad5 containing the 5' part of the E3 region was cloned into the EcoRI site of pBluescript (KS⁻) (Stratagene). Next, the HindIII site in the polylinker was removed by digestion with EcoRV and HincII and subsequent religation. The resulting clone pBS.Eco-Eco/ad5DHIII was used to delete the gp19K coding region. Primers 1 (5'-GGG TAT TAG GCC AA AGG CGC A-3') and 2 (5'-GAT CCC ATG GAA GCT TGG GTG GCG ACC CCA GCG-3') were used to amplify a sequence from pBS.Eco-Eco/Ad5DHIII corresponding to sequences 28511 to 28734 in wt Ad5 DNA. Primers 3 (5'-GAT CCC ATG GGG ATC CTT TAC TAA GTT ACA AAG CTA-3') and 4 (5'-GTC GCT GTA GTT GGA CTG G-3') were used on the same DNA to amplify Ad5 sequences from 29217 to 29476. The two resulting PCR fragments were ligated together by virtue of the new introduced NcoI site and subsequently digested with XbaI and MunI. This fragment was then ligated into the pBS.Eco-Eco/ad5 ΔHIII vector that was digested with XbaI (partially) and MunI generating pBS.Eco-Eco/ad5ΔHIII.Δgp19K. To allow insertion of foreign genes into the HindIII and BamHI site, an XbaI deletion was made in pBS.Eco-Eco/ad5ΔHIII.Δgp19K to remove the BamHI site in the Bluescript polylinker. The resulting plasmid pBS.Eco-Eco/ad5ΔHIIIΔgp19KΔXbaI, contains unique HindIII and BamHI sites corresponding to sequences 28733 (HindIII) and 29218 (BamHI) in Ad5. After introduction of a foreign gene into these sites, either the deleted XbaI fragment is re-introduced, or the insert is recloned into pBS.Eco-Eco/ad5ΔHIII.Δgp19K using HindIII and for example MunI. Using this procedure, we have generated plasmids expressing HSV-TK, hIL-1a, rat IL-3, luciferase or LacZ. The unique SrfI and NotI sites in the pBS.Eco-Eco/ad5ΔHIII.Δgp19K plasmid (with or without inserted gene of interest) are used to transfer the region comprising the gene of interest into the corresponding region of pBr/Ad.Bam-rITR, yielding construct pBr/Ad.Bam-rITRΔgp19K (with or without inserted gene of interest). This construct is used as described *supra* to produce recombinant adenoviruses. In the viral context, expression of inserted genes is driven by the adenovirus E3 promoter.

Recombinant viruses that are both E1 and E3 deleted are generated by a double homologous recombination procedure as described above for E1-replacement vectors using a plasmid-based system consisting of:
a) an adapter plasmid for E1 replacement according to the invention, with or without insertion of a first gene of interest,
b) the pWE/Ad.AflII-EcoRI fragment, and
c) the pBr/Ad.Bam-rITRΔgp19K plasmid with or without insertion of a second gene of interest.
In addition to manipulations in the E3 region, changes of (parts of) the E4 region can be accomplished easily in pBr/Ad.Bam-rITR. Generation and propagation of such a virus, however, in some cases demands complementation *in trans.*

### Example 2: Generation of adenovirus serotype 5 based viruses with chimaeric fiber proteins

The method described *infra* to generate recombinant adenoviruses by co-transfection of two, or more separate cloned adenovirus sequences. One of these cloned adenovirus sequences was modified such that the adenovirus serotype 5 fiber DNA was deleted and substituted for unique restriction sites thereby generating ìtemplate clonesî which allow for the easy introduction of DNA sequences encoding for fiber protein derived from other adenovirus serotypes.

### Generation of adenovirus template clones lacking DNA encoding for fiber

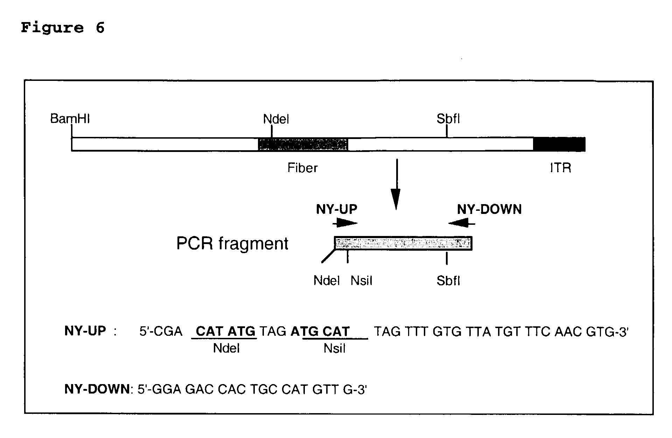

The fiber coding sequence of adenovirus serotype 5 is located between nucleotides 31042 and 32787. To remove the adenovirus serotype 5 DNA encoding fiber we started with construct pBr/Ad.Bam-rITR. First a NdeI site was removed from this construct. For this purpose, pBr322 plasmid DNA was digested with NdeI after which protruding ends were filled using Klenow enzyme. This pBr322 plasmid was then re-ligated, digested with NdeI and transformed into *E.coli* DH5α. The obtained pBr/ΔNdeI plasmid was digested with ScaI and SalI and the resulting 3198 bp vector fragment was ligated to the 15349 bp ScaI-SalI fragment derived from pBr/Ad.BamrITR, resulting in plasmid pBr/Ad.Bam-rITRΔNdeI which hence contained a unique NdeI site. Next a PCR was performed with oligonucleotides NY-up: 5'- CGA **CAT ATG** TAG ATG CAT TAG TTT GTG TTA TGT TTC AAC GTG-3'
And NY-down:5'-GGA GAC CAC TGC CAT GTT-3'(figure 6). During amplification, both a NdeI (bold face) and a NsiI restriction site (underlined) were introduced to facilitate cloning of the amplified fiber DNAs. Amplification consisted of 25 cycles of each 45 sec. at 94°C, 1 min. at 60°C, and 45 sec. at 72°C. The PCR reaction contained 25 pmol of oligonucleotides NY-up or NY-down, 2mM dNTP, PCR buffer with 1.5 mM MgCl₂, and 1 unit of Elongase heat stable polymerase (Gibco, The Netherlands). One-tenth of the PCR product was run on an agarose gel which demonstrated that the expected DNA fragment of ± 2200 bp was amplified. This PCR fragment was subsequently purified using Geneclean kit system (Bio101 Inc.). Then, both the construct pBr/Ad.Bam-rITRANdeI as well as the PCR product were digested with restriction enzymes NdeI and SbfI. The PCR fragment was subsequently cloned using T4 ligase enzyme into the NdeI and SbfI digested pBr/Ad.Bam-rITRΔNdeI, generating pBr/Ad.BamRΔFib. This plasmid allows insertion of any PCR amplified fiber sequence through the unique NdeI and NsiI sites that are inserted in place of the removed fiber sequence. Viruses can be generated by a double homologous recombination in packaging cells described *infra* using an adapter plasmid, construct pBr/Ad.AflII-EcoRI digested with PacI and EcoRI and a pBr/Ad.BamRΔFib construct in which heterologous fiber sequences have been inserted. To increase the efficiency of virus generation, the construct pBr/Ad.BamRΔFib was modified to generate a PacI site flanking the right ITR. Hereto, pBr/Ad.BamRΔFib was digested with AvrII and the 5 kb adeno fragment was isolated and introduced into the vector pBr/Ad.Bam-rITR.pac#8 replacing the corresponding AvrII fragment. The resulting construct was named pBr/Ad.BamRΔFib.pac. Once a heterologous fiber sequence is introduced in pBr/Ad.BamRΔFib.pac, the fiber modified right hand adenovirus clone may be introduced into a large cosmid clone as described for pWE/Ad.AflII-rITR in example 1. Such a large cosmid clone allows generation of adenovirus by only one homologous recombination making the process extremely efficient.

### Amplification of fiber sequences from adenovirus serotypes

To enable amplification of the DNAs encoding fiber protein derived from alternative serotypes degenerate oligonucleotides were synthesised. For this purpose, first known DNA sequences encoding for fiber protein of alternative serotypes were aligned to identify conserved regions in both the tail-region as well as the knob-region of the fiber protein. From the alignment, which contained the nucleotide sequence of 19 different serotypes representing all 6 subgroups, (degenerate) oligonucleotides were synthesised (see table 2). Also shown in table 3 is the combination of oligonucleotides used to amplify the DNA encoding fiber protein of a specific serotype. The amplification reaction (50 µl) contained 2 mM dNTPs, 25 pmol of each oligonucleotide, standard 1x PCR buffer, 1,5 mM MgCl₂, and 1 Unit Pwo heat stable polymerase (Boehringer) per reaction. The cycler program contained 20 cycles, each consisting of 30 sec. 94°C, 60 sec. 60-64°C, and 120 sec. At 72°C. One-tenth of the PCR product was run on an agarose gel which demonstrated that a DNA fragment was amplified. Of each different template, two independent PCR reactions were performed after which the independent PCR fragments obtained were sequenced to determine the nucleotide sequence. From 11 different serotypes, the nucleotide sequence could be compared to sequences present in Genbank. Of all other serotypes, the DNA encoding fiber protein was previously unknown and was therefore aligned with known sequences from other subgroup members to determine homology i.e. sequence divergence. Of the 51 human serotypes known to date, all fiber sequences, except for serotypes 1, 6, and 26, have been amplified and sequenced. The protein sequences of the fiber from different adenovirus serotypes is given in figure 7.

### Generation of fiber chimaeric adenoviral DNA constructs

All amplified fiber DNAs as well as the vector (pBr/Ad.BamRΔ Fib) were digested with NdeI and NsiI. The digested DNAs was subsequently run on a agarose gel after which the fragments were isolated from the gel and purified using the Geneclean kit (Bio101 Inc). The PCR fragments were then cloned into the NdeI and NsiI sites of pBr/AdBamRAFib, thus generating pBr/AdBamRFibXX (where XX stands for the serotype number of which the fiber DNA was isolated). So far the fiber sequence of serotypes 5/ 7/ 8/ 9/ 10/ 11/ 12/ 13/ 14/ 16/ 17/ 19/ 21/ 24/ 27/ 28/ 29/ 30/ 32/ 33/ 34/ 35/ 36/ 37/ 38/ 40-S/ 40-L/ 41-S/ 42/45/ 47/ 49/ 51 have been cloned into pBr/AdBamRFibXX. From pBr/AdBamRFibXX (where XX is 5/ 8/ 9/ 10/ 11/ 13/ 16/ 17/ 24/ 27/ 30/ 32/ 33/ 34/ 35/ 38/ 40-S/ 40-L/ 45/ 47/ 49/ 51) an 6 kb AvrII fragment encompassing the fiber sequence was isolated via gelelectrophoresis and Geneclean. This AvrII fragment was subsequently cloned in plasmid pBr/Ad.Bam-rITR.pac (see example 1) which was digested to completion with AvrII and dephosphorylated as described previously, leading to the generation of the plasmid pBr/Ad.Bam-rITR.pac.fibXX. This plasmid was subsequently used to generate a cosmid clone with a modified fiber using the constructs pWE.pac, pBr/AflII-Bam and pBr/Ad.Bam-rITR.pac.fibXX. This cosmid cloning resulted in the formation of construct pWE/Ad.AflII-rITR/FibXX (where XX stands for the serotype number of which the fiber DNA was isolated).

### Generation of pAd5/L420.HSA, pAd5/Clip and pAd5/Clipsal

pMLPI.TK was used to make a new vector in which nucleic acid molecules comprising specific promoter and gene sequences can be easily exchanged.
First, a PCR fragment was generated from pZipΔMo+PyF101(N⁻) template DNA (described in PCT/NL96/00195) with the following primers: LTR-1: 5'-CTG TAC GTA CCA GTG CAC TGG CCT AGG CAT GGA AAA ATA CAT AAC TG-3' and LTR-2: 5'-GCG GAT CCT TCG AAC CAT GGT AAG CTT GGT ACC GCT AGC GTT AAC CGG GCG ACT CAG TCA ATC G-3'. Pwo DNA polymerase (Boehringer Mannheim) was used according to manufacturers protocol with the following temperature cycles: once 5' at 95°C; 3' at 55°C; and 1' at 72°C, and 30 cycles of 1' at 95°C, 1' at 60°C, 1' at 72°C, followed by once 10' at 72°C. The PCR product was then digested with BamHI and ligated into pMLP10 (Levrero et al., 1991; Gene 101, 195-202) digested with PvuII and BamHI, thereby generating vector pLTR10. This vector contains adenoviral sequences from bp 1 up to bp 454 followed by a promoter consisting of a part of the Mo-MuLV LTR having its wild-type enhancer sequences replaced by the enhancer from a mutant polyoma virus (PyF101). The promoter fragment was designated L420. Sequencing confirmed correct amplification of the LTR fragment however the most 5' bases in the PCR fragment were missing so that the PvuII site was not restored. Next, the coding region of the murine HSA gene was inserted. pLTR10 was digested with BstBI followed by Klenow treatment and digestion with NcoI. The HSA gene was obtained by PCR amplification on pUC18-HSA (Kay et al., 1990; J. Immunol. 145, 1952-1959) using the following primers: HSA1, 5'-GCG CCA CCA TGG GCA GAG CGA TGG TGG C-3' and HSA2, 5'-GTT AGA TCT AAG CTT GTC GAC ATC GAT CTA CTA ACA GTA GAG ATG TAG AA-3'. The 269 bp amplified fragment was subcloned in a shuttle vector using the NcoI and BglII sites. Sequencing confirmed incorporation of the correct coding sequence of the HSA gene, but with an extra TAG insertion directly following the TAG stop codon. The coding region of the HSA gene, including the TAG duplication was then excised as a NcoI(sticky)-SalI(blunt) fragment and cloned into the 3.5 kb NcoI(sticky)/BstBI(blunt) fragment from pLTR10, resulting in pLTR-HSA10.
Finally, pLTR-HSA10 was digested with EcoRI and BamHI after which the fragment containing the left ITR, packaging signal, L420 promoter and HSA gene was inserted into vector pMLPI.TK digested with the same enzymes and thereby replacing the promoter and gene sequences. This resulted in the new adapter plasmid pAd5/L420-HSA that contains convenient recognition sites for various restriction enzymes around the promoter and gene sequences. SnaBI and AvrII can be combined with HpaI, NheI, KpnI, HindIII to exchange promoter sequences, while the latter sites can be combined with the ClaI or BamHI sites 3' from HSA coding region to replace genes in this construct.

Another adapter plasmid that was designed to allow easy exchange of nucleic acid molecules was made by replacing the promoter, gene and polyA sequences in pAd5/L420-HSA with the CMV promoter, a multiple cloning site, an intron and a polyA signal. For this purpose, pAd5/L420-HSA was digested with AvrII and BglII followed by treatment with Klenow to obtain blunt ends. The 5.1 kb fragment with pBr322 vector and adenoviral sequences was isolated and ligated to a blunt 1570 bp fragment from pcDNA1/amp (Invitrogen) obtained by digestion with HhaI and AvrII followed by treatment with T4 DNA polymerase. This adapter plasmid was named pAd5/Clip. To enable removal of vector sequences from the adenoviral fragment pAd5/Clip was partially digested with EcoRI and the linear fragment was isolated. An oligo of the sequence 5' TTAAGTCGAC-3' was annealed to itself resulting in a linker with a SalI site and EcoRI overhang. The linker was ligated to the partially digested pAd5/Clip vector and clones were selected that had the linker inserted in the EcoRI site 23 bp upstream of the left adenovirus ITR in pAd5/Clip resulting in pAd5/Clipsal.

### Generation of pAd5ClipLacZ, PAd5Clip.Luc, pAd5Clip.TK and pAd5Clipsal.Luc

The adapter plasmid pAd5/Clip.LacZ was generated as follows: The E.coli LacZ gene was amplified from the plasmid pMLP.nlsLacZ (EP 95-202 213) by PCR with the primers
5'GGGGTGGCCAGGGTACCTCTAGGCTTTTGCAA and
5'GGGGGGATCCATAAACAAGTTCAGAATCC. The PCR reaction was performed Ex Taq (Takara) according to the suppliers protocol at the following amplification program: 5 minutes 94°C, 1 cycle; 45 seconds 94°C and 30 seconds 60°C and 2 minutes 72°C, 5 cycles; 45 seconds 94°C and 30 seconds 65°C and 2 minutes 72°C, 25 cycles; 10 minutes 72; 45 seconds 94°C and 30 seconds 60°C and 2 minutes 72°C, 5 cycles, I cycle. The PCR product was subsequently digested with Kpnl and BamHI and the digested DNA fragment was ligated into KpnI/BamHI digested pcDNA3 (Invitrogen), giving rise to pcDNA3.nlsLacZ. Next, the plasmid pAd5/Clip was digested with SpeI. The large fragment containing part of the 5' part CMV promoter and the adenoviral sequences was isolated. The plasmid pcDNA3.nlsLacZ was digested with SpeI and the fragment containing the 3'part of the CMV promoter and the lacZ gene was isolated. Subsequently, the fragments were ligated, giving rise to pAd/Clip.LacZ. The reconstitution of the CMV promoter was confirmed by restriction digestion.

The adapter plasmid pAd5/Clip.Luc was generated as follows: The plasmid pCMV.Luc (EP 95-202 213) was digested with HindIII and BamHI. The DNA fragment containing the luciferase gene was isolated. The adapter plasmid pAd5/Clip was digested with HindIII and BamHI, and the large fragment was isolated. Next, the isolated DNA fragments were ligated, giving rise to pAd5/Clip.Luc. The adapter pClipsal.Luc was generated in the same way but using the adapter pClipsal digested with HIII and BamHI as vector fragment. Likewise, the TK containing HIII-BamHI fragment from pCMV.TK (EP 95-202 213) was inserted in pClipsal to generate pAd5/Clip.TK. The presence of the SalI site just upstream of the left ITR enables liberation of vector sequences from the adeno insert. Removal of these vector sequences enhances frequency of vector generation during homologous recombination in PER.C6.

### Generation of recombinant adenovirus chimaeric for fiber protein

To generate recombinant Ad 5 virus carrying the fiber of serotype 12, 16, 28, 40-L, 51, and 5, three constructs, pCLIP.Luc, pWE/AdAflII-Eco and pBr/AdBamrITR.pac/fibXX (XX = 12, 16, 28, 40-L, 51, and 5) were transfected into adenovirus producer cells. To generate recombinant Ad 5 virus carrying the fiber of 5/ 7/ 8/ 9/ 10/ 11/ 12/ 13/ 14/ 16/ 17/ 19/ 21/ 24/ 27/ 28/ 29/ 30/ 32/ 33/ 34/ 35/ 36/ 37/ 38/ 40-S/ 40-L/ 41-S/ 42/45/ 47/ 49/ 51, two constructs pCLIP.Luc and pWE/Ad.AflII-rITR/FibXX were transfected into adenovirus producer cells.
For transfection, 2 µg of pCLIP.Luc, and 4 µg of both pWE/AdAflII-Eco and pBr/AdBamrITR.pac/fibXX (or in case of cosmids: 4 µg of pCLIP.Luc plus 4 µg of pWE/Ad.AflII-rITR/FibXX) were diluted in serum free DMEM to 100 µl total volume. To this DNA suspension 100 µl 1x diluted lipofectamine (Gibco) was added. After 30 minutes at room temperature the DNA-lipofectamine complex solution was added to 2.5 ml of serum-free DMEM which was subsequently added to a T25 cm² tissue culture flask. This flask contained 2x10⁶ PER.C6 cells that were seeded 24-hours prior to transfection. Two hours later, the DNA-lipofectamine complex containing medium was diluted once by the addition of 2.5 ml DMEM supplemented with 20% foetal calf serum. Again 24 hours later the medium was replaced by fresh DMEM supplemented with 10% foetal calf serum. Cells were cultured for 6-8 days, subsequently harvested, and freeze/thawed 3 times. Cellular debris was removed by centrifugation for 5 minutes at 3000 rpm room temperature. Of the supernatant (12.5 ml) 3-5 ml was used to infect again infect PER.C6 cells (T80 cm² tissue culture flasks). This re-infection results in full cytopathogenic effect (CPE) after 5-6 days after which the adenovirus is harvested as described above.

### Example 3: Production, purification, and titration of fiber chimaeric adenoviruses

Of the supernatant obtained from transfected PER.C6 cells typically 10 ml was used to inoculate a 1 litre fermentor which contained 1 - 1.5 x 10⁶ cells/ ml PER.C6 that were specifically adapted to grow in suspension. Three days after inoculation, the cells were harvested and pelleted by centrifuging for 10 min at 1750 rpm at room temperature. The chimaeric adenoviruses present in the pelleted cells were subsequently extracted and purified using the following downstream processing protocol. The pellet was dissolved in 50 ml 10 mM NaPO₄⁻ and frozen at -20°C. After thawing at 37°C, 5.6 ml deoxycholate (5% w/v) was added after which the solution was homogenated. The solution was subsequently incubated for 15 minutes at 37°C to completely crack the cells. After homogenising the solution, 1875 µl (1M) MgCl₂⁻ was added and 5 ml 100% glycerol. After the addition of 375 µl DNase (10 mg/ ml) the solution was incubated for 30 minutes at 37°C. Cell debris was removed by centrifugation at 1880xg for 30 minutes at room temperature without the brake on. The supernatant was subsequently purified from proteins by loading on 10 ml of freon. Upon centrifugation for 15 minutes at 2000 rpm without brake at room temperature three bands are visible of which the upper band represents the adenovirus. This band was isolated by pipetting after which it was loaded on a Tris/HCl (1M) buffered caesiumchloride blockgradient (range: 1.2 to 1.4 gr./ml). Upon centrifugation at 21000 rpm for 2.5 hours at 10°C the virus was purified from remaining protein and cell debris since the virus, in contrast to the other components, does not migrate into the 1.4 gr./ ml caesiumchloride solution. The virus band is isolated after which a second purification using a Tris/ HCl (1M) buffered continues gradient of 1.33 gr./ml of caesiumchloride is performed. After virus loading on top of this gradient the virus is centrifuged for 17 hours at 55000 rpm at 10°C. Subsequently the virus band is isolated and after the addition of 30 µl of sucrose (50 w/v) excess caesiumchloride is removed by three rounds of dialysis, each round comprising of 1 hour. For dialysis the virus is transferred to dialysis slides (Slide-a-lizer, cut off 10000 kDa, Pierce, USA). The buffers used for dialysis are PBS which are supplemented with an increasing concentration of sucrose (round 1 to 3: 30 ml, 60 ml, and 150 ml sucrose (50% w/v)/ 1.5 litre PBS, all supplemented with 7.5 ml 2% (w/v) CaMgCl₂). After dialysis, the virus is removed from the slide-a-lizer after which it is aliquoted in portions of 25 and 100 µl upon which the virus is stored at -85°C.

To determine the number of virus particles per millilitre, 100 µl of the virus batch is run on an high pressure liquid chromatograph (HPLC). The adenovirus is bound to the column (anion exchange) after which it is eluted using a NaCl gradient (range 300-600 mM). By determining the area under the virus peak the number of virus particles can be calculated. To determine the number of infectious units (IU) per ml present in a virus batch, titrations are performed on 911 cells. For this purpose, 4x10⁴ 911 cells are seeded per well of 96-well plates in rows B, D, and F in a total volume of 100 µl per well. Three hours after seeding the cells are attached to the plastic support after which the medium can be removed. To the cells a volume of 200 µl is added, in duplicate, containing different dilutions of virus (range: 10² times diluted to 2x10⁹). By screening for CPE the highest virus dilution which still renders CPE after 14 days is considered to contain at least one infectious unit. Using this observation, together with the calculated amount of virus volume present in these wells renders the number of infectious units per ml of a given virus batch. The production results i.e. virus particles per ml and IU per ml or those chimaeric adenoviruses that were produced so far, are shown in table 3.

### Example 4: Presence of Ad5 Receptor molecules on human cells

To investigate the importance of the presence of CAR on target cells for infection with chimaeric adenoviruses, a panel of human cell lines and primary cells were tested for the presence and/ or absence of CAR, MHC class I, and integrins (αvβ3, αvβ5). For this purpose, 1x10⁵ target cells or were transferred to tubes (4 tubes per cell type) designed for flow cytometry. Cells were washed once with PBS/ 0.5% BSA after which the cells were pelleted by centrifugation for 5 minutes at 1750 rpm at room temperature. Subsequently, 10 µl of a 100 times diluted αᵥβ3 antibody (Mab 1961, Brunswick chemie, Amsterdam, The Netherlands), a 100 times diluted antibody αᵥβ5 (antibody (Mab 1976, Brunswick chemie, Amsterdam, The Netherlands), or 2000 times diluted CAR antibody was a kind gift of Dr. Bergelson, Harvard Medical School, Boston, USA (Hsu et al) was added to the cell pellet after which the cells were incubated for 30 minutes at 4°C in a dark environment. After this incubation, cells were washed twice with PBS/0.5% BSA and again pelleted by centrifugation for 5 minutes at 1750 rpm room temperature. To label the cells, 10 µl of rat anti mouse IgGl labelled with phycoerythrin (PE) was added to the cell pellet upon which the cells were again incubated for 30 minutes at 4°C in a dark environment. Finally the cells were washed twice with PBS/0.5% BSA and analysed on a flow cytometer. The results of flow cytometric analysis of these experiments are shown in table 4. These results show that human erythroid leukemia cells (K562, ATCC: CCL-243), human primary fibroblasts (GM09503), human primary smooth muscle cells, and primary human synoviocytes do not express detectable levels of the CAR receptor. In contrast, human lung carcinoma cells (A549, ATCC: CCL-1185), human lymphoblast cells (SupT1 (B and T cell hybrid, ATCC, CRL-1991), and human liver cells (HEPG2, ATCC, HB8065) express high amounts of CAR protein. Human lymphoblast cells (CEM, ATCC: CRL-1992), primary human umbilical vein endothelial cells (HUVEC), and human primary chorion villi express low amounts of CAR protein.

### Example 5: Infection of CAR negative cells with fiber chimaeric adenovirus

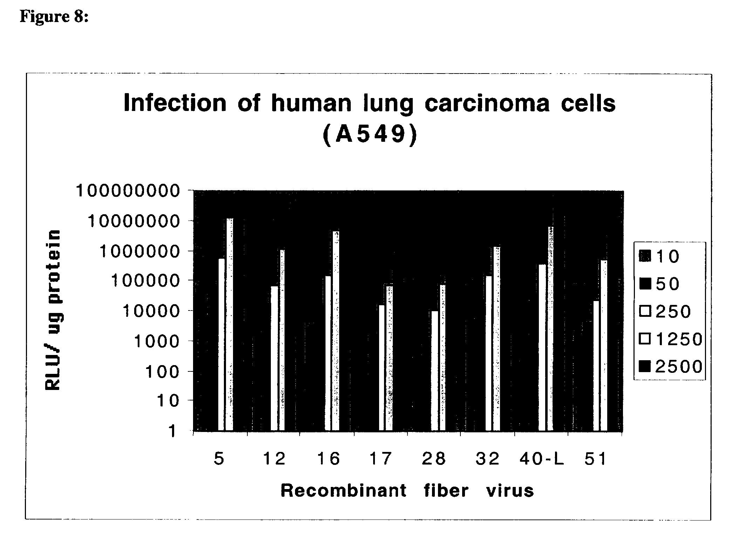

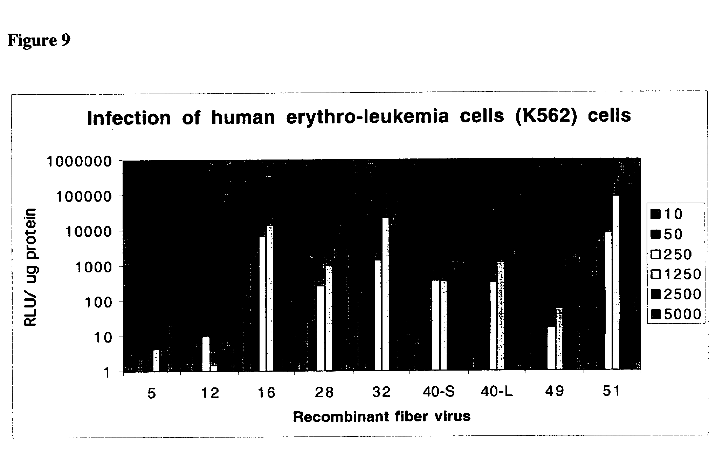

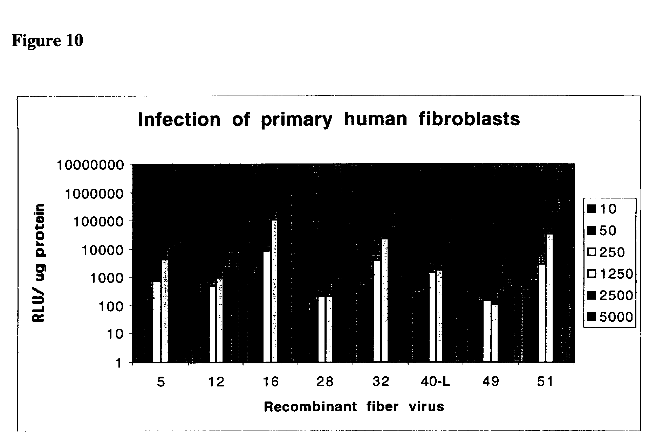

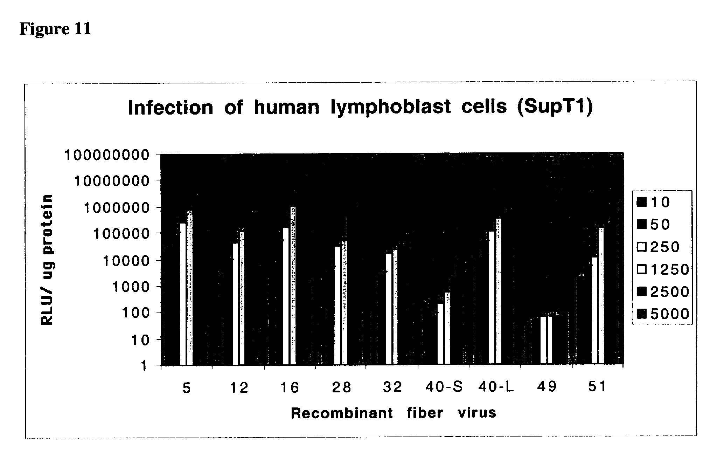

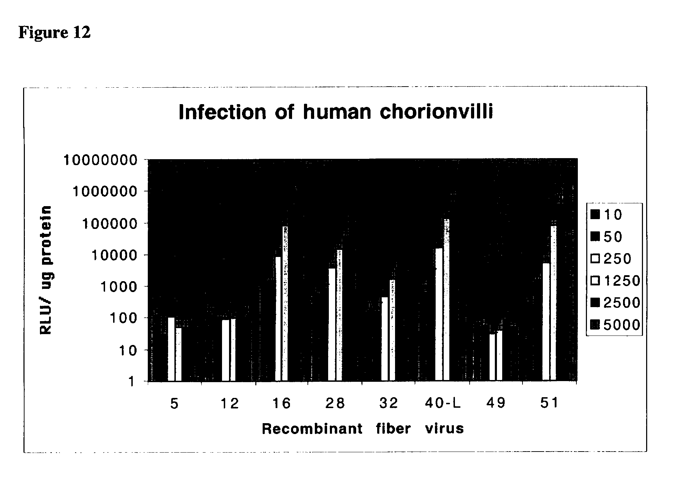

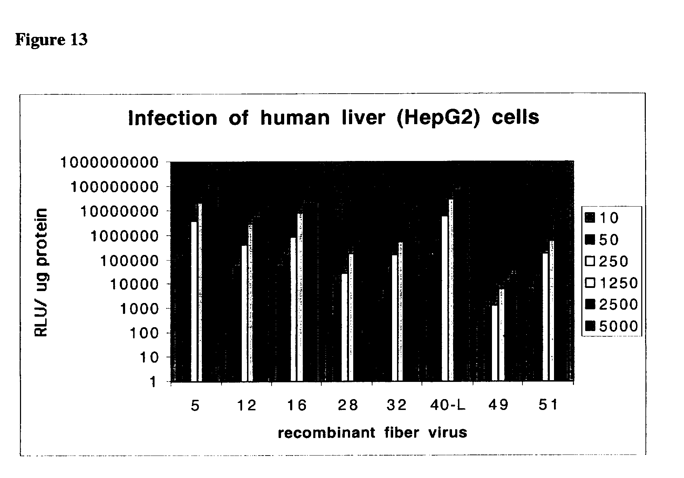

Several of the cell types described in example 4, i.e. A549, K562, GM09503, SupT1, chorion villi, and HepG2 were infected with a panel of chimaeric adenoviruses. This panel consists of adenovirus serotype 5 (subgroup C), and of adenovirus serotype 5 containing the fiber of serotypes 16 and 51 (subgroup B), of 28, 32, and 49 (subgroup D), of 12 (subgroup A), and of 40 (40-S and/or 40-L: subgroup F). For this purpose target cells are seeded at a concentration of 10⁵ cells per well of 6-well plates in 2 ml Dulbecco's modified Eagles medium (DMEM, Life Technologies, The Netherlands) supplemented with 10% Foetal calf serum. Twenty-four hours later the medium is replaced by fresh medium containing the different chimaeric adenoviruses at an increasing MOI of 0, 10, 50, 250, 1250, 2500, 5000 (MOI based on virus particles per millilitre). Approximately 2 hours after the addition of virus the medium containing the virus is discarded, cells are washed once with PBS, and subsequently 2 ml of fresh medium (not containing virus) is added to each well. Forthy-eight hours later cells are harvested, washed and pelleted by centrifuging 5 minutes at 1500 rpm. Cells are subsequently lysed in 0,1 ml lysis buffer (1% Triton-X-100, 15% Glycerol, 2 mM EDTA, 2 mM DTT, and 25 mM MgCl₂ in Tris-phosphate buffer pH 7.8) after which the total protein concentration of the lysate is measured (Biorad, protein standard II). To determine marker gene expression (luciferase activity) 20 µl of the protein sample is mixed with 100 µl of a luciferase substrate (Luciferine, Promega, The Netherlands) and subsequently measured on a Lumat LB 9507 apparatus (EG & G Berthold, The Netherlands). The results of these infection experiments, given as the amount of luciferase activity (RLU) per µg protein, are shown in figures 8-14. From these infection experiments several conclusions can be drawn. The infection of A549 cells (figure 8) demonstrates that all chimaeric adenoviruses tested infect with relative high efficiency these cells. The infection of K562 cells (figure 9) demonstrates that these cells cannot be transduced with adenovirus serotype 5 (subgroup C) or the fiber chimera 12 (subgroup A). All other chimaeric adenoviruses (16/ 51: subgroup B; 28/ 32/ 49: subgroup D; 40-L: subgroup F) are able to infect these cells with different efficiencies. The infection of GM09503 primary human fibroblasts (figure 10) demonstrates that these cells can be transduced with all fiber chimeras including Adenovirus serotype 5 albeit with different efficiencies. The infection of SupT1 cells (figure 11) demonstrates that these cells can be transduced with all fiber chimeras albeit with different efficiencies except for fiber chimera 49 which does not infect these human lymphoblast cells. The infection of human chorion villi cells (figure 12) shows a similar transduction pattern as observed with K562 cells except for adenovirus chimera 49 which does not infect these cells. The infection of HEPG2 cells (figure 13) shows a similar transduction pattern as observed with A549 cells. Linking the CAR expression data of these cells to the infection efficiency data obtained, several conclusions can be drawn. 1) Infection of adenovirus serotype 5 is correlated with the presence of CAR (figure 8-13). 2) In the absence of CAR but in the presence of high amount of MHC class I, poor infection is observed using adenovirus serotype 5, indicating that MHC class I is a worse receptor for adenovirus serotype 5 as compared to CAR (figure 10). 3) In the absence of CAR adenovirus fiber chimeras 16 and 51 (subgroup B) as well as chimeras 28 and 32 (subgroup D) as well as chimera 40-L (subgroup F) can infect cells with high efficiency, indicating that these viruses can utilise receptors other than CAR (figures 9 and 12). 4) A comparison of the infection data of the chimaeric adenoviruses carrying the fiber of 28, 32, and 49 teaches that within an adenovirus subgroup differences in transduction efficiencies exist, indicating that adenovirus members of one subgroup either have different affinities for the same receptor, or that different adherence molecules can be used (figures 8-13) by members of an adenovirus subgroup.

### Example 6: Complexity of receptor recognition of adenovirus serotypes

To investigate the complexity and/or the number of different adherence molecules which can be used by human adenoviruses from different subgroups or between members within one subgroup the following strategies are designed.

### 1) Interference studies with total chimaeric viruses

Via infection experiments described in example 5, cell lines are identified that are poorly transducible with a chimaeric viruses carrying the fiber protein of for example serotype 49 (subgroup D) indicating that such a cell expresses low levels of the adherence molecule required for D group adenovirus infection. Next, chimaeric adenoviruses carrying the fiber protein of other members of subgroup D are mixed in different concentrations with the fiber 49 chimaeric adenovirus and subsequently added to the cells. Since the fiber 49 chimaeric adenovirus carries a transgene other than the other subgroup D chimaeric adenoviruses (including but not limited to LacZ, Green Fluorescent Protein Yellow Fluorescent Protein, luciferase etc) interference of infection can be visualised. As a positive control two fiber 49 chimaeric adenoviruses carrying different marker genes is used. Identical to the example for subgroup D described above experiments are conducted with different members of subgroup A, B, C, E, and F. These experiments show if the fiber protein of members of the same adenovirus subgroup recognise the same adherence molecules on a cell membrane. Naturally, this approach is also used to investigate inter-subgroup variation for example usage of adherence molecules by subgroup D and B members

### 2) Interference studies with fiber protein derived peptides

Peptides of 6-12 amino acids are synthetically synthesised which together form the complete knob domain of a fiber from a subgroup D, for example 49. Next, one or more peptides are mixed in various concentrations with the fiber 49 chimaeric adenovirus after which the mixture is added to the cells. Using this approach one or more peptides are identified which block, at a certain concentration, the infection of the fiber 49 chimaeric adenovirus. This peptide or these peptides are subsequently used to investigate whether the infection of other subgroup D members is blocked by addition of the peptide(s) and whether inhibition of infection occurs using the same concentration of peptide. Identical to the example for subgroup D described above peptides are synthesised using the knob domain of a member of subgroup A, B, C, E, and F. These experiments show not only which adherence molecules are used but also which part of the fiber protein is directly involved in binding to target cells. Naturally these peptides are also used to investigate inter-subgroup variation.

### 3) Interference studies with baculovirus produced recombinant knob proteins

Of each adenovirus subgroup, the knob region of one member is amplified by PCR. The forward oligonucleotide hybridises to the final repeat of the shaft part of the fiber just upstream of the start of the knob protein. This oligonucleotide contains a restriction site to facilitate cloning, a Histidine (6x) tag for purification after production, and a mutation thereby introducing a Methionine start codon. The reverse oligonucleotide hybridises after the polyA signal and contains a restriction site to facilitate cloning into a baculovirus expression construct. After generation of recombinant baculovirus, insect cells for instance Sf9, are infected. 4-6 days after infection cells are cracked by 3 cycles of freeze/ thaw. Recombinant knob protein is purified from the supernatant using an antibody specifically recognising the His tag. The recombinant knobs are subsequently used in interference studies to investigate the complexity of adenovirus binding between members of different subgroups as well as members within one subgroup.

### Example 7: identification of adherence molecules involved in adenovirus subgroup B, D, and F binding and internalisation

To investigate what adherence molecules are involved in binding and internalisation of adenovirus serotypes from different subgroups in particular subgroups B, D, and F, the following strategies are designed.

### 1) Phage display libraries

Phage display libraries, containing random 6-12 amino acids peptides are ìmixedî with synthetically synthesised peptides which have identified to block infection of one or more members of either subgroup B, D, and/ or F. Mixing of phages with peptide(s) is performed in an ELISA setting in which the peptide(s) are coated to a plastic support. Several rounds of mixing, washing and elution are performed to obtain an enrichment for phages that truly and specifically bind to the peptide(s). Finally the phages retrieved are amplified and plaque purified after which approximately 20 are sequenced to establish the nature of the peptide insert of the phages. From the consensus sequence of all 20 phages, a (degenerate) oligonucleotide is synthesised which together with a polyA hybridising oligonucleotide is used for the amplification of cDNA sequences both from cells which can or cannot (negative control) be infected with a subgroup B, D, and/or F chimaeric adenovirus. Amplified cDNAs are cloned, sequenced and aligned, amongst others, against existing Genbank sequences.

### 2) cDNA expression library screening

cDNA libraries, either commercially available or generated using a CAR-negative cell line which is highly transducible with chimaeric adenoviruses carrying the fiber protein of members of for example subgroup D or subgroup F, are used for expression library screening using either radiolabelled adenovirus or recombinant produced knob proteins as probes. Clones or plaques which bind to the probe are picked, amplified and re-tested for enrichment of probe binding. Finally phages are picked after which the cDNA content is elucidated by sequence analysis. Retrieved cDNAs are cloned, sequenced and aligned, amongst others, against existing Genbank sequences.

### 3) Peptidase treatment of cells after adenovirus binding

Cells which are highly transducible with chimaeric adenoviruses carrying the fiber protein of members of for example subgroup D, are treated with different peptidases after binding of the chimaeric adenovirus. The panel of peptidases suited is first tested on the chimaeric adenovirus only to ensure that capsid proteins of the chimaeric virus is not cleaved. Peptidae treated cells are spun down after which the supernatant is added to 24-well plates precoated with anti-adenovirus hexon and/ or penton antibodies. After binding of adenovirus to the precoated plastic support, wells are washed extensively with PBS. Upon washing, the adenovirus is harvested after which either protein gel electrophoresis or Malditoff is used to identify whether parts of a cellular protein is bound to the fiber protein or whether extra protein bands are visible as compared to protein gel electrophoresis or Malditoff of a purified batch of adenovirus only. As a negative control for the above described experiments cells negative for infection with a chimaeric adenovirus carrying a fiber of a member of subgroup D can be used. Alternatively, cells which are highly transducible with chimaeric adenoviruses carrying the fiber protein of members of for example subgroup D, are first treated with peptidases after which the medium is incubated with adenoviruses bound to a plastic support.

The above described examples encompasses the construction of recombinant adenoviral vectors chimaeric for the fiber protein which results in an altered infection hostrange. The alteration of the infection host range results in highly efficient infection of cells negative for the CAR protein which is the protein required by adenovirus serotype 5 for efficient infection. These vectors are generated for the purpose of gene transfer and recombinant DNA vaccines. These vectors are thus ideally suited for gene transfer to tissues, and/or organs of which de cells do not express detectable levels of CAR.
Figure and table legends
Table 1: Association of human adenovirus serotypes with human disease.
Table 2: Oligonucleotides and degenerate oligonucleotides used for the amplification of DNA encoding for fiber protein derived from alternative human adenovirus serotypes. Bold letters in oligonucleotides A-E represent an NdeI restriction site. Bold letters in oligonucleotides 1-6 and 8 represent an NsiI restriction site. Bold letters in oligonucleotide 7 represents a PacI restriction site.
Table 3: Production results of fiber chimaeric adenoviruses. The number of virus particles per ml were determined using HPLC. The number of infectious units (IU) per millilitre were determined through titration on human 911 cells. For infection experiments, the number of virus particles per millilitre is taken from all chimaeric adenoviruses since IU/ ml reflects a receptor mediated process.
Table 4: Flow cytometric results on expression of integrins αᵥβ3 and αᵥβ5, the Coxsacki adenovirus receptor (CAR), and MHC class I on the membranes of human cell lines and human primary cells. A549: Human lung carcinoma cell line (ATCC, CCL-1185). K562: Human erythroid leukemia (ATCC, CCL-243). SupT1: Human Lymphoblast hybrid B and T (ATCC, CRL-1991). GM09503: Human primary fibroblasts. HEPG2: Human liver carcinoma (ATCC, HB8065). CEM: human lymphoblast cells (ATCC, CRL-1992). HeLa: Human cervix carcinoma (ATCC, CCL-2). Primary amniocytes and chorion villi cells were obtained from department of antropogenetics, Leiden, The Netherlands. Primary Smooth muscle cells, Human umbilical vein endothelial cells, and synoviocytes were obtained from TNO-PG, Leiden, The Netherlands. Shown is the percentage of cells expressing either molecule on their membrane. ND: not determined. 0% means undetectable expression of the molecule on the membrane of the cell using flow cytometry. 100% means high expression of the molecule on the cell membrane.

Figure 1: Schematic presentation of adapter plasmid pMLPI.TK.

Figure 2: Schematic presentation of adapter plasmid pAd/L420-HAS.

Figure 3: Schematic presentation of adapter plasmid pAd5/CLIP

Figure 4: Schematic presentation of plasmid system which requires only one recombinational event to generate recombinant adenoviruses.

Figure 5: Schematic presentation of plasmid system which requires two recombinational events to generate recombinant adenoviruses.

Figure 6: Schematic presentation of generation of plasmid pBr/AdBamRDeltaFib in which the Adenovirus type 5 fiber DNA is replaced by a short DNA stretch containing an unique NsiI site.

Figure 7: Fiber protein sequences of adenovirus serotypes 8, 9, 13, 14,20, 23, 24, 25, 27, 28, 29, 30, 32, 33, 34, 35, 36, 37, 38, 39, 42, 43, 44, 45, 46, 47, 48, 49, and 51. Bold letters represent part of the tail of adenovirus serotype 5. If bold letters not present it means that a PCR fragment was sequenced which does not contain the Ad5 tail. An X, present in the sequence means unidentified amino acid due to unidentified nucleotide. At the end of the sequence the stop codon of the fiber is presented by a dot.

Figure 8: Transduction of human lung carcinoma cells (A549) with a panel of chimaeric adenoviruses carrying the fiber of adenovirus 12, 16, 17, 28, 32, 40-L, or 51. Adenovirus 5 served as reference. Cells were infected with increasing MOI based on virus particles per cell: 10, 50, 250, 1250, 2500 (see legend on the right of graph). Luciferase transgene expression is expressed as relative light units (RLU) per µg of protein.

Figure 9: Transduction of human erythroid leukemia cells (K562) with a panel of chimaeric adenoviruses carrying the fiber of adenovirus 12, 16, 28, 32, 40-S, 40-L, 49, or 51. Adenovirus 5 served as reference. Cells were infected with increasing MOI based on virus particles per cell: 10, 50, 250, 1250, 2500, 5000 (see legend on the right of graph). Luciferase transgene expression is expressed as relative light units (RLU) per µg of protein. Error bars represent SD.

Figure 10: Transduction of human primary fibroblasts (GM09503) with a panel of chimaeric adenoviruses carrying the fiber of adenovirus 12, 16, 28, 32, 40-L, 49, or 51. Adenovirus 5 served as reference. Cells were infected with increasing MOI based on virus particles per cell: 10, 50, 250, 1250, 2500, 5000 (see legend on the right of graph). Luciferase transgene expression is expressed as relative light units (RLU) per µg of protein. Error bars represent SD.

Figure 11: Transduction of human lymphoblast cells (SupT1) with a panel of chimaeric adenoviruses carrying the fiber of adenovirus 12, 16, 28, 32, 40-S, 40-L, 49, or 51. Adenovirus 5 served as reference. Cells were infected with increasing MOI based on virus particles per cell: 10, 50, 250, 1250, 2500, 5000 (see legend on the right of graph). Luciferase transgene expression is expressed as relative light units (RLU) per µg of protein. Error bars represent SD.

Figure 12: Transduction of human chorion villi cells with a panel of chimaeric adenoviruses carrying the fiber of adenovirus 12, 16, 28, 32, 40-L, 49, or 51. Adenovirus 5 served as reference. Cells were infected with increasing MOI based on virus particles per cell: 10, 50, 250, 1250, 2500, 5000 (see legend on the right of graph). Luciferase transgene expression is expressed as relative light units (RLU) per µg of protein. Error bars represent SD.

Figure 13: Transduction of human hepatic cells (HEPG2) with a panel of chimaeric adenoviruses carrying the fiber of adenovirus 12, 16, 28, 32, 40-S, 40-L, 49, or 51. Adenovirus 5 served as reference. Cells were infected with increasing MOI based on virus particles per cell: 10, 50, 250, 1250, 2500, 5000 (see legend on the right of graph). Luciferase transgene expression is expressed as relative light units (RLU) per µg of protein. Error bars represent SD.

### REFERENCES

Arnberg N., Mei Y. and Wadell G., 1997. Fiber genes of adenoviruses with tropism for the eye and the genital tract. Virology 227: 239-244.

Bout A., 1997. Gene therapy, p. 167-182. In: D.J.A. Crommelin and R.D. Sindelar (ed.), Pharmaceutical biotechnology , Harwood Academic Publishers.

Bout, A. 1996. Prospects for human Gene therapy. Eur. J. drug Met. And Pharma. 2, 175-179.

Blaese et al., Cancer Gene Ther., 2 (1995):291-297).

Brody and Crystal, Ann. N. Y. Acad. Sci. 716(1994):90-101.

Chroboczek J., Ruigrok R.W.H., and Cusack S., 1995. Adenovirus fiber, p. 163-200. In: W. Doerfler and P. Bohm (ed.), The molecular repertoire of adenoviruses, I. Springer-Verlag, Berlin.

Defer C., Belin M., Caillet-Boudin M. and Boulanger P., 1990. Human adenovirus-host cell interactions; comparative study with members of subgroup B and C. Journal of Virology 64 (8): 3661-3673.

De Jong, J.C., Wermenbol, A.G., Verweij-Uijterwaal, M.W., Slaterus, K.W., Wertheim-van Dillen, P., van Doornum, G.J.J., Khoo, S.H., and Hierholzer, J.C. (1998) Adenoviruses from HIV-infected patients, including two new candidate serotypes Ad50 and Ad51 of Subgenus D and B1 respectively. In preparation.

Eisenlohr, L.C., Gerard, W., and Hackett, C.J. (1987). Role of receptor-binding activity of the viral hemagglutin molecule in the presentation of influenza virus antigens to helper T-cells. J. Virol 61, 1375-1383

Eiz B and Pring-⁰kerblom P., 1997. Molecular characterization of the type-specific g-determinant located on the adenovirus fiber. Journal of Virology 71: 6576-6581.

Francki, R.I.B., Fauquet, C.M., Knudson, D.L. and Brown, F. (1991) Classification and nomenclature of viruses. Fifth report of the international Committee on taxonomy of viruses. Arch. Virol. Suppl. 2, 140-144

GahÈry-SÈgard H., Farace F., Godfrin D., Gaston J., Lengagne R., Tursz T., Boulanger P. and Guillet J., 1998.Immune response to recombinant capsid proteins of adenovirus in humans: antifiber and anti-penton base antibodies have a synergistic effect on neutralizing activity. Journal of Virology 72: 2388-2397.

Gall J., Kass-Eisler A., Leinwand L. and Falck-Pedersen E., 1996. Adenovirus type 5 and 7 capsid chimera: fiber replacement alters receptor tropism without affecting primary immune neutralisation epitopes. Journal of Virology 70 (4): 2116-2123.

Greber, U.F., Willets, M., Webster, P., and Helenius, A. (1993). Stepwise dismanteling of adenovirus 2 during entry into cells. Cell 75, 477-486.

Hynes, R.O. (1992) Integrins: versatility, modulation and signalling in cell adhesion. Cell 69, 11-25

Herz and Gerard, Proc. Natl. Acad. Sci. U.S.A., 96 (1993):2812-2816

Hierholzer, J.C. (1992) Adenovirus in the immunocompromised host. Clin. Microbiol Rev. 5, 262-274.

Hierholzer, J.C., Wigand, R., Anderson, L.J., Adrian, T., and Gold, J.W.M. (1988) Adenoviruses from patients with AIDS: a plethora of serotypes and a description of five new serotypes of subgenus D (types43-47). J. Infect. Dis. 158, 804-813.

Ishibashi, M. and Yasue (1983) in Adenoviruses of Animals, Chapter 12, p497-561

Kay, R., Takei, F., and Humphries, R.K. (1990). Expression cloning of a cDNA encoding M1/69-J11d heat-stable antigens. J. Immunol. 145 (6), 1952-1959

Khoo, S.H., Bailey, A.S., De Jong, J.C., and Mandal, B.K. (1995). Adenovirus infections in human immunodeficiency virus-positive patients: Clinical features and molecular epidemiology. J. Infect. Dis 172, 629-637

Kidd, A.H., Chrboczek, J., Cusack, S., and Ruigrok, R.W.|H. (1993) Adenovirus type 40 virions containtwo distinct fibers. Virology 192, 73-84.

Krasnykh V.N., Mikheeva G.V., Douglas J.T. and Curiel D.T., 1996.Generation of recombinant adenovirus vectors with modified fibers for altering viral tropism. Journal of Virology **70**(10): 6839-6846.

Krasnykh V., Dmitriev I., Mikheeva G., Miller C.R., Belousova N. and Curiel D.T.,1998.
Characterization of an adenovirus vector containing a heterologous peptide epitope in the HI loop of the fiber knob. Journal of Virology **72**(3): 1844-1852.

Leopold, P.L., Ferris, B., Grinberg, I., Worgall, S., Hackett, N.R., and Crystal, R.G. (1998). Fluorescent virions: Dynamic tracking of the pathway of adenoviral vectors in living cells. Hum. Gene Ther. 9, 367-378.

Levrero, M., Barban, V., Manteca, S., Ballay, A., Balsamo, C., Avantaggiata, M.L., Natoli, G., Skellekens, H., Tiollais, P., and Perricaudet, M. (1991(. Defective and non-defective adenovirus vectors for expression foreign genes in vitro and in vivo. Gene 101, 195-202.

Matlin, K.S., Reggio, H., Helenius, A., and Simons, K. (1981). Infectious entry pathway of influenza virus in a canine kidney cell line. J. Cell Biol. 91, 601-613

Morgan, C., Rozenkrantz, H.S., and Mednis, B. (1969( Structure and development of viruses as observed in the electron microscope.X. Entry and uncoating of adenovirus. J.Virol 4, 777-796.

Roelvink, P.W., Lizonova, A.,Lee, J.G.M., Li, Y., Bergelson, J.M. Finberg, R.W., Brough, D.E., Kovesdi, I., and Wickham, T.J. (1998). The Coacksackievirus-adenovirus receptor protein can function as a cellular attachment protein for adenovirus serotypes from subgroups A,C,D,E, and F. J. Virology 72 (No. 10), 7909-7915

Richman, D.D., Hostetler, K.Y., Yazaki, P.J., and Clark, S. (1986). Fate of influenza A virion proteins after entry into subcellular fractions of LLC cells and the effect of amantadine. Virology 151, 200-210

Stevenson S.C., Rollence M., White B., Weaver L. and McClelland A., 1995.

Human adenovirus serotypes 3 and 5 bind to two different cellular receptors via the fiber head domain. Journal of Virology 69(5): 2850-2857.

Stevenson S.C., Rollence M., Marshall-Neff J. and McClelland A., 1997.Selective targeting of human cells by a chimaeric adenovirus vector containing a modified fiber protein. Journal of Virology 71(6): 4782-4790.

Signas, G., Akusjarvi, G., and Petterson, U. (1985). Adenovirus 3 fiberpolypeptide gene: Complications for the structure of the fiber protein. J. Virol. 53, 672-678.

Stouten, P.W.F., Sander, C., Ruigrok, R.W.H., and Cusack, S. (1992) New triple helical model for the shaft of the adenovirus fiber. J. Mol. Biol. 226, 1073-1084.

Schulick, A.H., Vassalli, G., Dunn, P.F., Dong, G., Rade, J.J., Zamarron, C. and Dichek, D.A. (1997). Established immunity precludes adenovirus-mediated gene transfer inrat carotid arteries.

Schnurr, D and Dondero, M.E. (1993) Two new candidate adenovirus serotypes Intervirol. 36, 79-83

Svensson, V. and Persson, R. (1984). Entry of adenovirus 2 into Hela cells. J. Virol. 51, 687-694.

Varga, M.J., Weibull, C., and Everitt, E. (1991). Infectious entry pathway of adenovirus type 2. J. Virol 65, 6061-6070.

Wickham T.J., Carrion M.E. and Kovesdi I., 1995. Targeting of adenovirus penton base to new receptors through replacement of its RGD motif with other receptor-specific peptide motifs. Gene Therapy **2**: 750-756.

Wickham T.J., Segal, D.M., Roelvink, P.W., Carrion M.E., Lizonova, A., Lee, G-M., and Kovesdi, I. (1996). Targeted adenovirus gene transfer to endothelial and smooth muscle cells by using bispecific antibodies. J. Virol. 70 (10), 6831-6838

Wickham, T.J., Mathias, P., Cherish, D.A., and Nemerow, G.R. (1993) Integrins avb3 and avb5 promote adenovirus internalisation but not virus attachment. Cell 73, 309-319.

### Tables and figures

**Table 1**

| **Syndrom** | **Subgenus** | **Serotype** |
|---|---|---|
| Respiratory illness | A | 31 |
| | B | 3, 7, 11, 14, 21, 34, 35, 51 |
| | C | 1,2,5,6 |
| | D | 39, 42-48 |
| | E | 4 |
| Keratoconjunctivitis (eye) | B | 11 |
| | D | 8, 19, 37, 50 |
| Hemorrhagic cystitis (Kidney) | B | 7, 11, 14, 16, 21, 34, 35 |
| And urogenital tract infections | C | 5 |
| | D | 39, 42-48 |
| Sexual transmission | C | 2 |
| | D | 19, 37 |
| Gastroenteritis | A | 31 |
| | B | 3 |
| | C | 1, 2, 5 |
| | D | 28 |
| | F | 40, 41 |
| CNS disease | A | 12, 31 |
| | B | 3, 7 |
| | C | 2, 5, 6 |
| | D | 32, 49 |
| Hepatitis | A | 31 |
| | C | 1,2,5 |
| Disseminated | A | 31 |
| | B | 3, 7, 11, 21 |
| | D | 30, 43-47 |
| None (???) | A | 18 |
| | D | 9, 10, 13, 15 17, 20, 22-29, 33, 36, 38 |

**Table 2**

| Serotype | Tail oligonucleotide | Knob oligonucleotide |
|---|---|---|
| 4 | A | 1 |
| 8 | B | 2 |
| 9 | B | 2 |
| 12 | E | 3 |
| 16 | C | 4 |
| 19p | B | 2 |
| 28 | B | 2 |
| 32 | B | 2 |
| 36 | B | 2 |
| 37 | B | 2 |
| 40-1 | D | 5 |
| 40-2 | D | 6 |
| 41-s | D | 5 |
| 41-1 | D | 7 |
| 49 | B | 2 |
| 50 | B | 2 |
| 51 | C | 8 |
| A: 5'- CCC GTG TAT CC**A TAT G**AT GCA GAC AAC GAC CGA CC- 3' | | |
| B: 5'- CCC GTC TAC CC**A TAT G**GC TAC GCG CGG- 3' | | |
| C: 5'- CCK GTS TAC CC**A TAT G**AA GAT GAA AGC- 3' | | |
| D: 5'- CCC GTC TAC CC**A TAT G**AC ACC TYC TCA ACT C- 3' | | |
| E: 5'- CCC GTT TAC CC**A TAT G**AC CCA TTT GAC ACA TCA GAC- 3' | | |
| 1: 5''- CCG **ATG CAT** TTA TTG TTG GGC TAT ATA GGA - 3' | | |
| 2: 5'- CCG **ATG CAT** TYA TTC TTG GGC RAT ATA GGA - 3' | | |
| 3: 5'- CCG **ATG CAT** TTA TTC TTG GGR AAT GTA WGA AAA GGA - 3' | | |
| 4: 5'- CCG **ATG CAT** TCA GTC ATC TTC TCT GAT ATA - 3' | | |
| 5: 5'- CCG **ATG CAT** TTA TTG TTC AGT TAT GTA GCA - 3' | | |
| 6: 5'- GCC **ATG CAT** TTA TTG TTC TGT TAC ATA AGA - 3' | | |
| 7: 5' - CCG **TTA ATT AA**G CCC TTA TTG TTC TGT TAC ATA AGA A - 3' | | |
| 8: 5'- CCG **ATG CAT** TCA GTC ATC YTC TWT AAT ATA - 3' | | |

**Table 3**

| Adenovirus | Virus particles/ ml | Infectious units/ ml |
|---|---|---|
| Ad5Fib5 | 2.2 x 10¹² | 6.8 x 10¹¹ |
| Ad5Fib12 | 4.4 x 10¹² | 1.9 x 10¹² |
| Ad5Fib16 | 1.4 x 10¹² | 3.0 x 10¹⁰ |
| Ad5Fib17 | 9.3 x 10¹¹ | 9.5 x 10⁹ |
| Ad5Fib28 | 5.4 x 10¹⁰ | 2.8 x 10⁸ |
| Ad5Fib32 | 2.0 x 10¹² | 1.1 x 10¹² |
| Ad5Fib40-S | 3.2 x 10¹⁰ | 1.0 x 10¹⁰ |
| Ad5Fib40-L | 2.0 x 10¹² | 6.4 x 10¹¹ |
| Ad5Fib49 | 1.2 x 10¹² | 4.3 x 10¹¹ |
| Ad5Fib51 | 5.1 x 10¹² | 1.0 x 10¹² |

**Table 4**

| Cell line | αᵥβ3 | αᵥβ5 | CAR | MHC class I |
|---|---|---|---|---|
| A549 | 17% | 98% | 100% | ND |
| K562 | 12% | 55% | 0% | 15% |
| GM09503 | 20% | 50% | 0% | 100% |
| CEM | 0% | 0% | 3% | 100% |
| SupT1 | 5% | 1% | 70% | 100% |
| Smooth muscle cells | 100% | 70% | 0% | 15% |
| HUVEC | 100% | 15% | 10% | 90% |
| Synoviocytes | 30% | 40% | 0% | 100% |
| 1⁰chorionvilli | 100% | 0% | 12% | 100% |
| HepG2 | 0% | 10% | 100% | 80% |

## Claims

1. A method for delivering a nucleic acid of interest to a host cell by means of a gene delivery vehicle based on adenoviral material, whereby said gene delivery vehicle delivers the nucleic acid to the host cell by associating with a binding site and/or a receptor present on CAR-negative cells, said binding site and/or receptor being a binding site and/or a receptor for adenovirus subgroups D and/or F.

2. Use of a gene delivery vehicle comprising a nucleic acid of interest and comprising adenoviral material involved in binding to a host cell, said material being from a subgroup D and/or F adenovirus, in delivering said nucleic acid of interest to a CAR-negative cell.

3. A gene delivery vehicle being a chimaera based on at least two adenoviruses, whereby a cell recognising element of said gene delivery vehicle is based on adenoviral material from a subgroup D and/or F adenovirus, which material confers the capability of infecting CAR negative cells.

4. A gene delivery vehicle according to claim 3, wherein said adenoviral material is based on a fiber, a penton and/or a hexon protein of a subgroup D and/or subgroup F adenovirus.

5. A gene delivery vehicle according to claim 3 or 4, further comprising an element from adenovirus 35, responsible for at least partially avoiding an immune response against adenovirus 35.

6. A gene delivery vehicle according to any one of claims 3-5, which comprises an element of adenovirus 16 or a functional analogue thereof, which element confers said virus with an enhanced capability to infect smooth muscle cells and/or synoviocytes.

7. A gene delivery vehicle according to any one of claims 3-6, comprising a nucleic acid derived from an adenovirus.

8. A gene delivery vehicle according to any one of claims 3-7, comprising a nucleic acid derived from at least two different adenoviruses.

9. A gene delivery vehicle according to claim 7 or claim 8, wherein said nucleic acid comprises at least one sequence encoding a capsid protein comprising at least a tissue tropism determining fragment of a subgroup D and/or subgroup F adenovirus capsid protein.

10. A gene delivery vehicle according to any one of claims 7-9, wherein said nucleic acid derived from adenovirus is modified such that the capacity of said nucleic acid to replicate in a target cell has been reduced or disabled.

11. A gene delivery vehicle according to any one of claims 7-10, wherein said nucleic acid derived from adenovirus is modified such that the capacity of a host immune system to mount an immune response against adenovirus proteins encoded by said nucleic acid derived from adenovirus has been reduced or disabled.

12. A gene delivery vehicle according to anyone of claims 7-11, comprising a minimal adenovirus vector or an integrating adenovirus such as an Ad/AAV chimaeric vector, a retro-adenovirus or a transposon-adenovirus.

13. A gene delivery vehicle according to anyone of the claims 1-12, further comprising at least one non-adenovirus nucleic acid.

14. A gene delivery vehicle according to anyone of claims 7-13, wherein said nucleic acid derived from adenovirus is produced by welding together through homologous recombination two nucleic acid molecules comprising partially overlapping sequences wherein said overlapping sequences allow essentially only one homologous recombination which leads to the generation of a physically linked nucleic acid comprising at least two functional adenovirus inverted terminal repeats, a functional encapsulation signal, a nucleic acid of interest, or functional parts, derivatives and/or analogues thereof.

15. A cell for the production of a gene delivery vehicle according to anyone of the claims 3-14, comprising means for the assembly of said gene delivery vehicle wherein said means includes a means for the production of an adenovirus capsid protein, wherein said capsid protein comprises at least a receptor and/or binding site binding fragment of a subgroup D and/or subgroup F adenovirus capsid protein.

16. A cell according to claim 15, wherein said cell is or is derived from a PER.C6 cell (ECACC deposit number 96022940).

17. The use of a gene delivery vehicle according to anyone of the claims 1-14 as a pharmaceutical.

18. A receptor and/or a binding site for adenoviruses type D and/or F, present on or associated with CAR negative cells.

19. A receptor and/or a binding site according to claim 18, present on K562 cells, amniotic fluid cells and/or primary fibroblast cells.

20. A capsid protein derived from a subgroup D and/or a subgroup F adenovirus or a functional part, derivative and/or analogue thereof.

21. A capsid protein according to claim 20, wherein said protein is a fiber protein.

22. An isolate and/or recombinant nucleic acid encoding a capsid protein according to claim 20 or claim 21.

23. An isolate and/or recombinant nucleic acid according to claim 22, wherein said nucleic acid comprises a sequence as depicted in figure 7.


### Annex to the application documents - subsequently filed sequences listing

SEQUENCE LISTING

<110> Introgene BV

<120> Infection with chimaeric adenoviruses of cells negative
      for the adenovirus serotype 5 Coxsacki adenovirus
      receptor (CAR).

<130> P50220EP00

<140> 99202234.3
<141> 1999-07-08

<160> 58

<170> PatentIn Ver. 2.1

<210> 1
<211> 23
<212> DNA
<213> Artificial Sequence

<220>
<223> Description of Artificial Sequence: "oligo linker"

<220>
<221> misc_feature
<222> (1)..(23)
<223> /note="Linker containing a PacI site"

<400> 1
aattgtctta attaaccgct taa                                          23


<210> 2
<211> 19
<212> DNA
<213> Artificial Sequence

<220>
<223> Description of Artificial Sequence: "oligo linker"

<220>
<221> misc_feature
<222> (1)..(19)
<223> /note="Linker containing a PacI site"

<400> 2
aattgtctta attaaccgc                                               19


<210> 3
<211> 19
<212> DNA
<213> Artificial Sequence

<220>
<223> Description of Artificial Sequence: "oligo linker"

<220>
<221> misc_feature

<222> (1)..(19)
<223> /note="Linker containing a PacI site"

<400> 3
aattgcggtt aattaagac                                                    19


<210> 4
<211> 47
<212> DNA
<213> Artificial Sequence

<220>
<223> Description of Artificial Sequence: "primer"

<220>
<221> primer_bind
<222> (1)..(47)
<223> /note="Primer LTR-1"

<400> 4
ctgtacgtac cagtgcactg gcctaggcat ggaaaaatac ataactg                     47


<210> 5
<211> 64
<212> DNA
<213> Artificial Sequence

<220>
<223> Description of Artificial Sequence:"primer"

<220>
<221> primer_bind
<222> (1)..(64)
<223> /note="Primer LTR-2"

<400> 5
gcggatcctt cgaaccatgg taagcttggt accgctagcg ttaaccgggc gactcagtca 60
atcg                                                                    64


<210> 6
<211> 28
<212> DNA
<213> Artificial Sequence

<220>
<223> Description of Artificial Sequence:"primer"

<220>
<221> primer_bind
<222> (1)..(28)
<223> /note="Primer HSA1"

<400> 6
gcgccaccat gggcagagcg atggtggc                                          28


<210> 7
<211> 50

<212> DNA
<213> Artificial Sequence

<220>
<223> Description of Artificial Sequence:"primer"

<220>
<221> primer_bind
<222> (1)..(50)
<223> /note="Primer HSA2"

<400> 7
gttagatcta agcttgtcga catcgatcta ctaacagtag agatgtagaa          50


<210> 8
<211> 21
<212> DNA
<213> Artificial Sequence

<220>
<223> Description of Artificial Sequence:"primer"

<220>
<221> primer_bind
<222> (1)..(21)
<223> /note="Primer 1"

<400> 8
gggtattagg ccaaaggcgc a                                         21


<210> 9
<211> 33
<212> DNA
<213> Artificial Sequence

<220>
<223> Description of Artificial Sequence:"primer"

<220>
<221> primer_bind
<222> (1)..(33)
<223> /note="Primer 2"

<400> 9
gatcccatgg aagcttgggt ggcgacccca gcg                            33


<210> 10
<211> 36
<212> DNA
<213> Artificial Sequence

<220>
<223> Description of Artificial Sequence:"primer"

<220>
<221> primer_bind
<222> (1)..(36)
<223> /note="Primer 3"

<400> 10
gatcccatgg ggatccttta ctaagttaca aagcta                                36

<210> 11
<211> 19
<212> DNA
<213> Artificial Sequence

<220>
<223> Description of Artificial Sequence:"primer"

<220>
<221> primer_bind
<222> (1)..(19)
<223> /note="Primer 4"

<400> 11
gtcgctgtag ttggactgg                                                   19

<210> 12
<211> 42
<212> DNA
<213> Artificial Sequence

<220>
<223> Description of Artificial Sequence:"primer"

<220>
<221> primer_bind
<222> (1)..(42)
<223> /note="Primer NY-up"

<400> 12
cgacatatgt agatgcatta gtttgtgtta tgtttcaacg tg                         42

<210> 13
<211> 19
<212> DNA
<213> Artificial Sequence

<220>
<223> Description of Artificial Sequence:"primer"

<220>
<221> primer_bind
<222> (1)..(19)
<223> /note="Primer NY-down"

<400> 13
ggagaccact gccatgttg                                                   19

<210> 14
<211> 10
<212> DNA
<213> Artificial Sequence

<220>
<223> Description of Artificial Sequence:"oligo linker"

<220>
<221> misc_feature
<222> (1)..(10)
<223> /note="Linker with  SalI and EcoRI-site"

<400> 14
ttaagtcgac                                                              10


<210> 15
<211> 32
<212> DNA
<213> Artificial Sequence

<220>
<223> Description of Artificial Sequence:"primer"

<220>
<221> primer_bind
<222> (1)..(32)
<223> /note="LacZ primer 1"

<400> 15
ggggtggcca gggtacctct aggcttttgc aa                                     32


<210> 16
<211> 29
<212> DNA
<213> Artificial Sequence

<220>
<223> Description of Artificial Sequence:"primer"

<220>
<221> primer_bind
<222> (1)..(29)
<223> /note="LacZ primer 2"

<400> 16
gggggatcc ataaacaagt tcagaatcc                                          29


<210> 17
<211> 35
<212> DNA
<213> Artificial Sequence

<220>
<223> Description of Artificial Sequence:"oligonucleotide"

<220>
<221> misc_feature
<222> (1)..(35)
<223> /note="Tail oligonucleotide"

<400> 17
cccgtgtatc catatgatgc agacaacgac cgacc                                  35

<210> 18
<211> 27
<212> DNA
<213> Artificial Sequence

<220>
<223> Description of Artificial Sequence:"oligonucleotide"

<220>
<221> misc_feature
<222> (1)..(27)
<223> /note="Tail oligonucleotide"

<400> 18
cccgtctacc catatggcta cgcgcgg                                          27


<210> 19
<211> 27
<212> DNA
<213> Artificial Sequence

<220>
<223> Description of Artificial Sequence:"oligonucleotide"

<220>
<221> misc_feature
<222> (1)..(27)
<223> /note="Tail oligonucleotide"

<400> 19
cckgtstacc catatgaaga tgaaagc                                          27


<210> 20
<211> 31
<212> DNA
<213> Artificial Sequence

<220>
<223> Description of Artificial Sequence:"oligonucleotide"

<220>
<221> misc_feature
<222> (1)..(31)
<223> /note="Tail oligonucleotide"

<400> 20
cccgtctacc catatgacac ctyctcaact c                                     31


<210> 21
<211> 36
<212> DNA
<213> Artificial Sequence

<220>
<223> Description of Artificial Sequence:"oligonucleotide"

<220>
<221> misc_feature
<222> (1)..(36)
<223> /note="Tail oligonucleotide"

<400> 21
cccgtttacc catatgaccc atttgacaca tcagac                                36


<210> 22
<211> 30
<212> DNA
<213> Artificial Sequence

<220>
<223> Description of Artificial Sequence:"oligonucleotide"

<220>
<221> misc_feature
<222> (1)..(30)
<223> /note="Knob oligonucleotide"

<400> 22
ccgatgcatt tattgttggg ctatatagga                                       30


<210> 23
<211> 30
<212> DNA
<213> Artificial Sequence

<220>
<223> Description of Artificial Sequence:"oligonucleotide"

<220>
<221> misc_feature
<222> (1)..(30)
<223> /note="Knob oligonucleotide"

<400> 23
ccgatgcatt yattcttggg cratatagga                                       30


<210> 24
<211> 36
<212> DNA
<213> Artificial Sequence

<220>
<223> Description of Artificial Sequence:"oligonucleotide"

<220>
<221> misc_feature
<222> (1)..(36)
<223> /note="Knob oligonucleotide"

<400> 24
ccgatgcatt tattcttggg raatgtawga aaagga                                36


<210> 25

<211> 30
<212> DNA
<213> Artificial Sequence

<220>
<223> Description of Artificial Sequence:"oligonucleotide"

<220>
<221> misc_feature
<222> (1)..(30)
<223> /note="Knob oligonucleotide"

<400> 25
ccgatgcatt cagtcatctt ctctgatata                                        30


<210> 26
<211> 30
<212> DNA
<213> Artificial Sequence

<220>
<223> Description of Artificial Sequence:"oligonucleotide"

<220>
<221> misc_feature
<222> (1)..(30)
<223> /note="Knob oligonucleotide"

<400> 26
ccgatgcatt tattgttcag ttatgtagca                                        30


<210> 27
<211> 30
<212> DNA
<213> Artificial Sequence

<220>
<223> Description of Artificial Sequence:"oligonucleotide"

<220>
<221> misc_feature
<222> (1)..(30)
<223> /note="Knob oligonucleotide"

<400> 27
gccatgcatt tattgttctg ttacataaga                                        30


<210> 28
<211> 37
<212> DNA
<213> Artificial Sequence

<220>
<223> Description of Artificial Sequence:"oligonucleotide"

<220>
<221> misc_feature
<222> (1)..(37)

<223> /note="Knob oligonucleotide"

<400> 28
ccgttaatta agcccttatt gttctgttac ataagaa                                37

<210> 29
<211> 30
<212> DNA
<213> Artificial Sequence

<220>
<223> Description of Artificial Sequence:"oligonucleotide"

<220>
<221> misc_feature
<222> (1)..(30)
<223> /note="Knob oligonucleotide"

<400> 29
ccgatgcatt cagtcatcyt ctwtaatata                                        30

<210> 30
<211> 377
<212> PRT
<213> adenoviridae

<220>
<221> VARIANT
<222> (1)..(377)
<223> /note="Serotype 8 fiber protein"

<400> 30
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asn Gly Phe Gln Asn Phe Pro Pro Gly Val Leu Ser Leu Lys
    50                  55                  60

Leu Ala Asp Pro Ile Thr Ile Asn Asn Gln Asn Val Ser Leu Lys Val
65                  70                  75                  80

Gly Gly Gly Leu Thr Leu Gln Glu Glu Thr Gly Lys Leu Thr Val Asn
                85                  90                  95

Thr Glu Pro Pro Leu His Leu Thr Asn Asn Lys Leu Gly Ile Ala Leu
            100                 105                 110

Asp Ala Pro Phe Asp Val Ile Asp Asn Lys Leu Thr Leu Leu Ala Gly
        115                 120                 125

His Gly Leu Ser Ile Ile Thr Lys Glu Thr Ser Thr Leu Pro Gly Leu
    130                 135                 140

Val Asn Thr Leu Val Val Leu Thr Gly Lys Gly Ile Gly Thr Asp Leu
145                 150                 155                 160

Ser Asn Asn Gly Gly Asn Ile Cys Val Arg Val Gly Glu Gly Gly Gly
                165                 170                 175

Leu Ser Phe Asn Asp Asn Gly Asp Leu Val Ala Phe Asn Lys Lys Glu
            180                 185                 190

Asp Lys Arg Thr Leu Trp Thr Thr Pro Asp Thr Ser Pro Asn Cys Arg
        195                 200                 205

Ile Asp Gln Asp Lys Asp Ser Lys Leu Thr Leu Val Leu Thr Lys Cys
    210                 215                 220

Gly Ser Gln Ile Leu Ala Asn Val Ser Leu Ile Val Val Ala Gly Arg
225                 230                 235                 240

Tyr Lys Ile Ile Asn Asn Asn Thr Asn Pro Ala Leu Lys Gly Phe Thr
                245                 250                 255

Ile Lys Leu Leu Phe Asp Lys Asn Gly Val Leu Met Glu Ser Ser Asn
            260                 265                 270

Leu Gly Lys Ser Tyr Trp Asn Phe Arg Asn Gln Asn Ser Ile Met Ser
        275                 280                 285

Thr Ala Tyr Glu Lys Ala Ile Gly Phe Met Pro Asn Leu Val Ala Tyr
    290                 295                 300

Pro Lys Pro Thr Thr Gly Ser Lys Lys Tyr Ala Arg Asp Ile Val Tyr
305                 310                 315                 320

Gly Asn Ile Tyr Leu Gly Gly Lys Pro His Gln Pro Val Thr Ile Lys
                325                 330                 335

Thr Thr Phe Asn Gln Glu Thr Gly Cys Glu Tyr Ser Ile Thr Phe Asp
            340                 345                 350

Phe Ser Trp Ala Lys Thr Tyr Val Asn Val Glu Phe Glu Thr Thr Ser
        355                 360                 365

Phe Thr Phe Ser Tyr Ile Ala Gln Glu
    370                 375

<210> 31
<211> 377
<212> PRT
<213> adenoviridae

<220>
<221> VARIANT
<222> (1)..(377)
<223> /note="Serotype 9 fiber protein"

<400> 31
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asp Gly Phe Gln Asn Phe Pro Pro Gly Val Leu Ser Leu Lys
    50                  55                  60

Leu Ala Asp Pro Ile Ala Ile Val Asn Gly Asn Val Ser Leu Lys Val
65                  70                  75                  80

Gly Gly Gly Leu Thr Leu Gln Asp Gly Thr Gly Lys Leu Thr Val Asn
                85                  90                  95

Ala Asp Pro Pro Leu Gln Leu Thr Asn Asn Lys Leu Gly Ile Ala Leu
            100                 105                 110

Asp Ala Pro Phe Asp Val Ile Asp Asn Lys Leu Thr Leu Leu Ala Gly
        115                 120                 125

His Gly Leu Ser Ile Ile Thr Lys Glu Thr Ser Thr Leu Pro Gly Leu
    130                 135                 140

Ile Asn Thr Leu Val Val Leu Thr Gly Lys Gly Ile Gly Thr Glu Ser
145                 150                 155                 160

Thr Asp Asn Gly Gly Ser Val Cys Val Arg Val Gly Glu Gly Gly Gly
                165                 170                 175

Leu Ser Phe Asn Asn Asp Gly Asp Leu Val Ala Phe Asn Lys Lys Glu
            180                 185                 190

Asp Lys Arg Thr Leu Trp Thr Thr Pro Asp Thr Ser Pro Asn Cys Lys
        195                 200                 205

Ile Asp Gln Asp Lys Asp Ser Lys Leu Thr Leu Val Leu Thr Lys Cys
    210                 215                 220

Gly Ser Gln Ile Leu Ala Asn Val Ser Leu Ile Val Val Ala Gly Lys
225                 230                 235                 240

Tyr Lys Ile Ile Asn Asn Asn Thr Gln Pro Ala Leu Lys Gly Phe Thr
                245                 250                 255

Ile Lys Leu Leu Phe Asp Glu Asn Gly Val Leu Met Glu Ser Ser Asn
            260                 265                 270

Leu Gly Lys Ser Tyr Trp Asn Phe Arg Asn Glu Asn Ser Ile Met Ser
        275                 280                 285

Thr Ala Tyr Glu Lys Ala Ile Gly Phe Met Pro Asn Leu Val Ala Tyr
    290                 295                 300

Pro Lys Pro Thr Ala Gly Ser Lys Lys Tyr Ala Arg Asp Ile Val Tyr
305                 310                 315                 320

Gly Asn Ile Tyr Leu Gly Gly Lys Pro Asp Gln Pro Val Thr Ile Lys
                325                 330                 335

Thr Thr Phe Asn Gln Glu Thr Gly Cys Glu Tyr Ser Ile Thr Phe Asp

340                    345                    350

Phe Ser Trp Ala Lys Thr Tyr Val Asn Val Glu Phe Glu Thr Thr Ser
        355                 360                 365

Phe Thr Phe Ser Tyr Ile Ala Gln Glu
    370                 375

<210> 32
<211> 391
<212> PRT
<213> adenoviridae

<220>
<221> VARIANT
<222> (1)..(391)
<223> /note="Serotype 13 fiber protein"

<400> 32
Xaa Xaa Xaa Xaa Xaa Ser Ala Pro Thr Ile Phe Met Leu Leu Gln Met
1               5                   10                  15

Lys Arg Ala Arg Ser Ser Xaa Asp Thr Phe Asn Pro Val Tyr Pro Tyr
            20                  25                  30

Gly Tyr Ala Arg Asn Gln Asn Ile Xaa Phe Xaa Thr Pro Pro Phe Val
        35                  40                  45

Xaa Ser Asp Gly Phe Lys Asn Phe Pro Pro Gly Val Leu Ser Leu Lys
    50                  55                  60

Leu Ala Asp Pro Ile Thr Ile Ala Asn Gly Asp Val Ser Leu Lys Val
65                  70                  75                  80

Gly Gly Gly Leu Thr Leu Gln Glu Gly Ser Leu Thr Val Asp Pro Lys
                85                  90                  95

Ala Pro Leu Gln Leu Ala Asn Asp Lys Lys Leu Glu Leu Val Tyr Asp
            100                 105                 110

Asp Pro Phe Glu Val Ser Thr Asn Lys Leu Ser Leu Lys Val Gly His
        115                 120                 125

Gly Leu Lys Val Leu Asp Asp Lys Ser Ala Gly Gly Leu Lys Asp Leu
    130                 135                 140

Ile Gly Lys Leu Val Val Leu Thr Gly Lys Gly Ile Gly Ile Glu Asn
145                 150                 155                 160

Leu Gln Asn Asp Asp Gly Ser Ser Arg Gly Val Gly Ile Asn Val Arg
                165                 170                 175

Leu Gly Thr Asp Gly Gly Leu Ser Phe Asp Arg Lys Gly Glu Leu Val
            180                 185                 190

Ala Trp Asn Arg Lys Asp Asp Arg Arg Thr Leu Trp Thr Thr Pro Asp
        195                 200                 205

Pro Ser Pro Asn Cys Lys Ala Glu Thr Glu Lys Asp Ser Lys Leu Thr

210                 215                 220

Leu Val Leu Thr Lys Cys Gly Ser Gln Ile Leu Ala Thr Val Ser Ile
225                 230                 235                 240

Ile Val Leu Lys Gly Lys Tyr Glu Phe Val Lys Lys Glu Thr Glu Pro
                245                 250                 255

Lys Ser Phe Asp Val Lys Leu Leu Phe Asp Ser Lys Gly Val Leu Leu
            260                 265                 270

Pro Thr Ser Asn Leu Ser Lys Glu Tyr Trp Asn Tyr Arg Ser Tyr Asp
        275                 280                 285

Asn Asn Ile Gly Thr Pro Tyr Glu Asn Ala Val Pro Phe Met Pro Asn
    290                 295                 300

Leu Lys Ala Tyr Pro Lys Pro Thr Lys Thr Ala Ser Asp Lys Ala Glu
305                 310                 315                 320

Asn Lys Ile Ser Ser Ala Lys Asn Lys Ile Val Ser Asn Phe Tyr Phe
                325                 330                 335

Gly Gly Gln Ala Tyr Gln Pro Gly Thr Ile Ile Ile Lys Phe Asn Glu
            340                 345                 350

Glu Ile Asp Glu Thr Cys Ala Tyr Ser Ile Thr Phe Asn Phe Gly Trp
        355                 360                 365

Gly Lys Val Tyr Asp Asn Pro Phe Pro Phe Asp Thr Thr Ser Phe Thr
    370                 375                 380

Xaa Ser Tyr Ile Ala Gln Glu
385                 390



<210> 33
<211> 290
<212> PRT
<213> adenoviridae

<220>
<221> VARIANT
<222> (1)..(290)
<223> /note="Serotype 14 fiber protein"

<400> 33
His Pro Phe Ile Asn Pro Gly Phe Ile Ser Pro Asn Gly Phe Thr Gln
1               5                   10                  15

Ser Pro Asp Gly Val Leu Thr Leu Lys Cys Leu Thr Pro Leu Thr Thr
            20                  25                  30

Thr Gly Gly Ser Leu Gln Leu Lys Val Gly Gly Gly Leu Thr Val Asp
        35                  40                  45

Asp Thr Asp Gly Thr Leu Gln Glu Asn Ile Gly Ala Thr Thr Pro Leu
    50                  55                  60

Val Lys Thr Gly His Ser Ile Gly Leu Ser Leu Gly Ala Gly Leu Gly

65                  70                  75                  80

Thr Asp Glu Asn Lys Leu Cys Thr Lys Leu Gly Glu Gly Leu Thr Phe
                85                  90                  95

Asn Ser Asn Asn Ile Cys Ile Asp Asp Asn Ile Asn Thr Leu Trp Thr
            100                 105                 110

Gly Val Asn Pro Thr Glu Ala Asn Cys Gln Met Met Asp Ser Ser Glu
        115                 120                 125

Ser Asn Asp Cys Lys Leu Ile Leu Thr Leu Val Lys Thr Gly Ala Leu
    130                 135                 140

Val Thr Ala Phe Val Tyr Val Ile Gly Val Ser Asn Asn Phe Asn Met
145                 150                 155                 160

Leu Thr Thr Tyr Arg Asn Ile Asn Phe Thr Ala Glu Leu Phe Phe Asp
                165                 170                 175

Ser Ala Gly Asn Leu Leu Thr Ser Leu Ser Ser Leu Lys Thr Pro Leu
            180                 185                 190

Asn His Lys Ser Gly Gln Thr Trp Leu Leu Val Pro Leu Leu Met Leu
        195                 200                 205

Lys Val Ser Cys Pro Ala Gln Leu Leu Ile Leu Ser Ile Ile Ile Leu
    210                 215                 220

Glu Lys Asn Lys Thr Thr Phe Thr Glu Leu Val Thr Thr Gln Leu Val
225                 230                 235                 240

Ile Thr Leu Leu Phe Pro Leu Thr Ile Ser Val Met Leu Asn Gln Arg
                245                 250                 255

Ala Ile Arg Ala Asp Thr Ser Tyr Cys Ile Arg Ile Thr Trp Ser Trp
            260                 265                 270

Asn Thr Gly Asp Ala Pro Glu Gly Gln Thr Ser Ala Thr Thr Leu Val
        275                 280                 285

Thr Ser
    290

<210> 34
<211> 345
<212> PRT
<213> adenoviridae

<220>
<221> VARIANT
<222> (1)..(345)
<223> /note="Serotype 20 fiber protein"

<400> 34
Ile Gln Asn Ile Pro Phe Leu Thr Pro Pro Phe Val Ser Ser Asp Gly
1               5                   10                  15

Leu Gln Asn Phe Pro Pro Gly Val Leu Ser Leu Lys Leu Ala Asp Pro

20                  25                  30

Ile Ala Ile Val Asn Gly Asn Val Ser Leu Lys Val Gly Gly Gly Ile
        35                  40                  45

Thr Val Glu Gln Asp Ser Gly Gln Leu Ile Ala Asn Pro Lys Ala Pro
    50                  55                  60

Leu Gln Val Ala Asn Asp Lys Leu Glu Leu Ser Tyr Ala Tyr Pro Phe
65                  70                  75                  80

Glu Thr Ser Ala Asn Lys Leu Ser Leu Lys Val Gly Gln Gly Leu Lys
                85                  90                  95

Val Leu Asp Glu Lys Asp Ser Gly Gly Leu Gln Asn Leu Leu Gly Lys
            100                 105                 110

Leu Val Val Leu Thr Gly Lys Gly Ile Gly Val Glu Glu Leu Lys Asn
        115                 120                 125

Pro Asp Asn Thr Asn Arg Gly Val Gly Ile Asn Val Arg Leu Gly Lys
    130                 135                 140

Asp Gly Gly Leu Ser Phe Asn Lys Asn Gly Glu Leu Val Ala Trp Asn
145                 150                 155                 160

Lys His Asn Asp Thr Gly Thr Leu Trp Thr Thr Pro Asp Pro Ser Pro
                165                 170                 175

Asn Cys Lys Ile Glu Glu Val Lys Asp Ser Lys Leu Thr Leu Val Leu
            180                 185                 190

Thr Lys Cys Gly Ser Gln Ile Leu Ala Thr Met Ala Phe Gln Val Val
        195                 200                 205

Lys Gly Thr Tyr Glu Asn Ile Ser Lys Asn Thr Ala Lys Asn Ser Phe
    210                 215                 220

Ser Ile Lys Leu Leu Phe Asp Asp Asn Gly Lys Leu Leu Glu Gly Ser
225                 230                 235                 240

Ser Leu Asp Lys Asp Tyr Trp Asn Phe Arg Ser Asp Asp Ser Ile Ile
                245                 250                 255

Pro Asn Gln Tyr Asp Asn Ala Val Pro Phe Met Pro Asn Leu Lys Ala
            260                 265                 270

Tyr Pro Lys Pro Ser Thr Val Leu Pro Ser Thr Asp Lys Asn Ser Asn
        275                 280                 285

Gly Lys Asn Thr Ile Val Ser Asn Leu Tyr Leu Glu Gly Lys Ala Tyr
    290                 295                 300

Gln Pro Val Ala Val Thr Ile Thr Phe Asn Lys Glu Ile Gly Cys Thr
305                 310                 315                 320

Tyr Ser Ile Thr Phe Asp Phe Gly Trp Ala Lys Thr Tyr Asp Val Pro
                325                 330                 335

Ile Pro Phe Asp Ser Ser Ser Phe Thr
            340                 345

<210> 35
<211> 346
<212> PRT
<213> adenoviridae

<220>
<221> VARIANT
<222> (1)..(346)
<223> /note="Serotype 23 fiber protein"

<400> 35
Gln Asn Ile Pro Phe Leu Thr Pro Pro Phe Val Ser Ser Asp Gly Phe
1               5                   10                  15

Gln Asn Phe Pro Pro Gly Val Leu Ser Leu Lys Leu Ala Asp Pro Ile
            20                  25                  30

Ala Ile Thr Asn Gly Asp Val Ser Leu Lys Val Gly Gly Gly Leu Thr
        35                  40                  45

Val Glu Gln Asp Ser Gly Asn Leu Lys Val Asn Thr Lys Ala Pro Leu
    50                  55                  60

Gln Val Ala Ala Asp Lys Gln Leu Glu Ile Ala Leu Ala Asp Pro Phe
65                  70                  75                  80

Glu Val Ser Lys Gly Arg Leu Gly Ile Lys Ala Gly His Gly Leu Lys
                85                  90                  95

Val Ile Asp Asn Ser Ile Ser Gly Leu Glu Gly Leu Val Gly Thr Leu
            100                 105                 110

Val Val Leu Thr Gly His Gly Ile Gly Thr Glu Asn Leu Leu Asn Asn
        115                 120                 125

Asp Gly Ser Ser Arg Gly Val Gly Ile Asn Val Arg Leu Gly Lys Asp
    130                 135                 140

Gly Gly Leu Ser Phe Asp Lys Lys Gly Asp Leu Val Ala Trp Asn Lys
145                 150                 155                 160

Lys Tyr Asp Thr Arg Thr Leu Trp Thr Thr Pro Asp Pro Ser Pro Asn
                165                 170                 175

Cys Lys Val Ile Glu Ala Lys Asp Ser Lys Leu Thr Leu Val Leu Thr
            180                 185                 190

Lys Cys Gly Ser Gln Ile Leu Ala Asn Met Ser Leu Leu Ile Leu Lys
        195                 200                 205

Gly Thr Tyr Glu Tyr Ile Ser Asn Ala Ile Ala Asn Lys Ser Phe Thr
    210                 215                 220

Ile Lys Leu Leu Phe Asn Asp Lys Gly Val Leu Met Asp Gly Ser Ser
225                 230                 235                 240

Leu Asp Lys Asp Tyr Trp Asn Tyr Lys Ser Asp Asp Ser Val Met Ser
                245                 250                 255

Lys Ala Tyr Glu Asn Ala Val Pro Phe Met Pro Asn Leu Lys Ala Tyr
            260                 265                 270

Pro Asn Pro Thr Thr Ser Thr Thr Asn Pro Ser Thr Asp Lys Lys Ser
        275                 280                 285

Asn Gly Lys Asn Ala Ile Val Ser Asn Val Tyr Leu Glu Gly Arg Ala
    290                 295                 300

Tyr Gln Pro Val Ala Ile Thr Ile Thr Phe Asn Lys Glu Thr Gly Cys
305                 310                 315                 320

Thr Tyr Ser Met Thr Phe Asp Phe Gly Trp Ser Lys Val Tyr Asn Asp
                325                 330                 335

Pro Ile Pro Phe Asp Thr Ser Ser Leu Thr
            340                 345

<210> 36
<211> 390
<212> PRT
<213> adenoviridae

<220>
<221> VARIANT
<222> (1)..(390)
<223> /note="Serotype 24 fiber protein"

<400> 36
Ser Cys Ser Cys Pro Ser Ala Pro Thr Ile Phe Met Leu Leu Gln Met
  1               5                  10                  15

Lys Arg Ala Arg Pro Ser Glu Asp Thr Phe Asn Pro Val Tyr Pro Tyr
             20                  25                  30

Gly Tyr Ala Arg Asn Gln Asn Ile Pro Phe Leu Thr Pro Pro Phe Val
         35                  40                  45

Ser Ser Asp Gly Phe Gln Asn Phe Pro Pro Gly Val Leu Ser Leu Lys
     50                  55                  60

Leu Ala Asp Pro Ile Ala Ile Thr Asn Gly Asp Val Ser Leu Lys Val
 65                  70                  75                  80

Gly Gly Gly Leu Thr Val Glu Lys Asp Ser Gly Asn Leu Lys Val Asn
                 85                  90                  95

Pro Lys Ala Pro Leu Gln Val Thr Thr Asp Lys Gln Leu Glu Ile Ala
            100                 105                 110

Leu Ala Tyr Pro Phe Glu Val Ser Asn Gly Lys Leu Gly Ile Lys Ala
        115                 120                 125

Gly His Gly Leu Lys Val Ile Asp Lys Ile Ala Gly Leu Glu Gly Leu
    130                 135                 140

Ala Gly Thr Leu Val Val Leu Thr Gly Lys Gly Ile Gly Thr Glu Asn
145                 150                 155                 160

Leu Glu Asn Ser Asp Gly Ser Ser Arg Gly Val Gly Ile Asn Val Arg
                165                 170                 175

Leu Ala Lys Asp Gly Gly Leu Ser Phe Asp Lys Lys Gly Asp Leu Val
            180                 185                 190

Ala Trp Asn Lys His Asp Asp Arg Arg Thr Leu Trp Thr Thr Pro Asp
        195                 200                 205

Pro Ser Pro Asn Cys Thr Ile Asp Gln Glu Arg Asp Ser Lys Leu Thr
    210                 215                 220

Leu Val Leu Thr Lys Cys Gly Ser Gln Ile Leu Ala Asn Val Ser Leu
225                 230                 235                 240

Leu Val Val Lys Gly Lys Phe Ser Asn Ile Asn Asn Asn Thr Asn Pro
                245                 250                 255

Thr Asp Lys Lys Ile Thr Val Lys Leu Leu Phe Asn Glu Lys Gly Val
            260                 265                 270

Leu Met Asp Ser Ser Thr Leu Lys Lys Glu Tyr Trp Asn Tyr Arg Asn
        275                 280                 285

Asp Asn Ser Thr Val Ser Gln Ala Tyr Asp Asn Ala Val Pro Phe Met
    290                 295                 300

Pro Asn Ile Lys Ala Tyr Pro Lys Pro Thr Thr Asp Thr Ser Ala Lys
305                 310                 315                 320

Pro Glu Asp Lys Lys Ser Ala Ala Lys Arg Tyr Ile Val Ser Asn Val
                325                 330                 335

Tyr Ile Gly Gly Leu Pro Asp Lys Thr Val Val Ile Thr Ile Lys Phe
            340                 345                 350

Asn Ala Glu Thr Glu Cys Ala Tyr Ser Ile Thr Phe Glu Phe Thr Trp
        355                 360                 365

Ala Lys Thr Phe Glu Asp Val Gln Phe Asp Ser Ser Ser Phe Thr Phe
    370                 375                 380

Ser Tyr Ile Ala Gln Glu
385                 390

<210> 37
<211> 375
<212> PRT
<213> adenoviridae

<220>
<221> VARIANT
<222> (1)..(375)
<223> /note="Serotype 25 fiber protein"

<400> 37
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asp Gly Phe Gln Asn Phe Pro Pro Gly Val Leu Ser Leu Lys
    50                  55                  60

Leu Ala Asp Pro Ile Thr Ile Ser Asn Gly Asp Val Ser Leu Lys Val
65                  70                  75                  80

Gly Gly Gly Leu Thr Val Glu Gln Asp Ser Gly Asn Leu Ser Val Asn
                85                  90                  95

Pro Lys Ala Pro Leu Gln Val Gly Thr Asp Lys Lys Leu Glu Leu Ala
            100                 105                 110

Leu Ala Pro Pro Phe Asn Val Lys Asp Asn Lys Leu Asp Leu Leu Val
        115                 120                 125

Gly Asp Gly Leu Lys Val Ile Asp Lys Ser Ile Ser Xaa Leu Pro Gly
    130                 135                 140

Leu Leu Asn Tyr Leu Val Val Leu Thr Gly Lys Gly Ile Gly Asn Glu
145                 150                 155                 160

Glu Leu Lys Asn Asp Asp Gly Ser Asn Lys Gly Val Gly Leu Cys Val
                165                 170                 175

Arg Ile Gly Glu Gly Gly Gly Leu Thr Phe Asp Asp Lys Gly Tyr Leu
            180                 185                 190

Val Ala Trp Asn Lys Lys His Asp Ile Arg Thr Leu Trp Thr Thr Leu
        195                 200                 205

Asp Pro Ser Pro Asn Cys Arg Ile Asp Val Asp Lys Asp Ser Lys Leu
    210                 215                 220

Thr Leu Val Leu Thr Lys Cys Gly Ser Gln Ile Leu Ala Asn Val Ser
225                 230                 235                 240

Leu Leu Val Val Lys Gly Arg Phe Gln Asn Leu Asn Tyr Lys Thr Asn
                245                 250                 255

Pro Asn Leu Pro Lys Thr Phe Thr Ile Lys Leu Leu Phe Asp Glu Asn
            260                 265                 270

Gly Ile Leu Lys Asp Ser Ser Asn Leu Asp Lys Asn Tyr Trp Asn Tyr
        275                 280                 285

Arg Asn Gly Asn Ser Ile Leu Ala Glu Gln Tyr Lys Asn Ala Val Gly
    290                 295                 300

Phe Met Pro Asn Leu Ala Ala Tyr Pro Lys Ser Thr Thr Thr Gln Ser
305                 310                 315                 320

Lys Leu Tyr Ala Arg Asn Thr Ile Phe Gly Asn Ile Tyr Leu Asp Ser
                325                 330                 335

Gln Ala Tyr Asn Pro Val Val Ile Lys Ile Thr Phe Asn Gln Glu Ala
            340                 345                 350

Asp Ser Ala Tyr Ser Ile Thr Leu Asn Tyr Ser Trp Gly Lys Asp Tyr
        355                 360                 365

Glu Asn Ile Pro Phe Asp Ser
    370                 375

<210> 38
<211> 335
<212> PRT
<213> adenoviridae

<220>
<221> VARIANT
<222> (1)..(335)
<223> /note="Serotype 27 fiber protein"

<400> 38
Ile Pro Phe Leu Thr Pro Pro Phe Val Ser Ser Asp Gly Phe Lys Asn
 1               5                  10                  15

Phe Pro Pro Gly Val Leu Ser Leu Lys Leu Ala Asp Pro Ile Thr Ile
            20                  25                  30

Thr Asn Gly Asp Val Ser Leu Lys Val Gly Gly Gly Leu Val Val Glu
        35                  40                  45

Lys Glu Ser Gly Lys Leu Ser Val Asp Pro Lys Thr Pro Leu Gln Val
    50                  55                  60

Ala Ser Asp Asn Lys Leu Glu Leu Ser Tyr Asn Ala Pro Phe Lys Val
65                  70                  75                  80

Glu Asn Asp Lys Leu Ser Leu Asp Val Gly His Gly Leu Lys Val Ile
                85                  90                  95

Gly Asn Glu Val Ser Ser Leu Pro Gly Leu Ile Asn Lys Leu Val Val
            100                 105                 110

Leu Thr Gly Lys Gly Ile Gly Thr Glu Glu Leu Lys Glu Gln Asn Ser
        115                 120                 125

Asp Lys Ile Ile Gly Val Gly Ile Asn Val Arg Ala Arg Gly Gly Leu
    130                 135                 140

Ser Phe Asp Asn Asp Gly Tyr Leu Val Ala Trp Asn Pro Lys Tyr Asp
145                 150                 155                 160

Thr Arg Thr Leu Trp Thr Thr Pro Asp Thr Ser Pro Asn Cys Lys Met
                165                 170                 175

Leu Thr Lys Lys Asp Ser Lys Leu Thr Leu Thr Leu Thr Lys Cys Gly
            180                 185                 190

Ser Gln Ile Leu Gly Asn Val Ser Leu Leu Ala Val Ser Gly Lys Tyr
        195                 200                 205

Leu Asn Met Thr Lys Asp Glu Thr Gly Val Lys Ile Ile Leu Leu Phe
    210                 215                 220

Asp Arg Asn Gly Val Leu Met Gln Glu Ser Ser Leu Asp Lys Glu Tyr
225                 230                 235                 240

Trp Asn Tyr Arg Asn Asp Asn Asn Val Ile Gly Thr Pro Tyr Glu Asn
                245                 250                 255

Ala Val Gly Phe Met Pro Asn Leu Val Ala Tyr Pro Lys Pro Thr Ser
            260                 265                 270

Ala Asp Ala Lys Asn Tyr Ser Arg Ser Lys Ile Ile Ser Asn Val Tyr
        275                 280                 285

Leu Lys Gly Leu Ile Tyr Gln Pro Val Ile Ile Ile Ala Ser Phe Asn
    290                 295                 300

Gln Glu Thr Thr Asn Gly Cys Val Tyr Ser Ile Ser Phe Asp Phe Thr
305                 310                 315                 320

Cys Ser Lys Asp Tyr Thr Gly Gln Gln Phe Asp Val Thr Ser Phe
                325                 330                 335

<210> 39
<211> 374
<212> PRT
<213> adenoviridae

<220>
<221> VARIANT
<222> (1)..(374)
<223> /note="Serotype 28 fiber protein"

<400> 39
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asp Gly Phe Gln Asn Phe Pro Pro Gly Val Leu Ser Leu Lys
    50                  55                  60

Leu Ala Asp Pro Ile Thr Ile Ala Asn Gly Asp Val Ser Leu Lys Leu
65                  70                  75                  80

Gly Gly Gly Leu Thr Val Glu Lys Glu Ser Gly Asn Leu Thr Val Asn
                85                  90                  95

Pro Lys Ala Pro Leu Gln Val Ala Ser Gly Gln Leu Glu Leu Ala Tyr
            100                 105                 110

Tyr Ser Pro Phe Asp Val Lys Asn Asn Met Leu Thr Leu Lys Ala Gly
        115                 120                 125

His Gly Leu Ala Val Val Thr Lys Asp Asn Thr Asp Leu Gln Pro Leu
    130                 135                 140

Met Gly Thr Leu Val Val Leu Thr Gly Lys Gly Ile Gly Thr Gly Thr
145                 150                 155                 160

Ser Ala His Gly Gly Thr Ile Asp Val Arg Ile Gly Lys Asn Gly Ser
                165                 170                 175

Leu Ala Phe Asp Lys Asn Gly Asp Leu Val Ala Trp Asp Lys Glu Asn
            180                 185                 190

Asp Arg Arg Thr Leu Trp Thr Thr Pro Asp Thr Ser Pro Asn Cys Lys
        195                 200                 205

Met Ser Glu Val Lys Asp Ser Lys Leu Thr Leu Ile Leu Thr Lys Cys
    210                 215                 220

Gly Ser Gln Ile Leu Gly Ser Val Ser Leu Leu Ala Val Lys Gly Glu
225                 230                 235                 240

Tyr Gln Asn Met Thr Ala Ser Thr Asn Lys Asn Val Lys Ile Thr Leu
                245                 250                 255

Leu Phe Asp Ala Asn Gly Val Leu Leu Glu Gly Ser Ser Leu Asp Lys
            260                 265                 270

Glu Tyr Trp Asn Phe Arg Asn Asn Asp Ser Thr Val Ser Gly Lys Tyr
        275                 280                 285

Glu Asn Ala Val Pro Phe Met Pro Asn Ile Thr Ala Tyr Lys Pro Val
    290                 295                 300

Asn Ser Lys Ser Tyr Ala Arg Ser His Ile Phe Gly Asn Val Tyr Ile
305                 310                 315                 320

Asp Ala Lys Pro Tyr Asn Pro Val Val Ile Lys Ile Ser Phe Asn Gln
                325                 330                 335

Glu Thr Gln Asn Asn Cys Val Tyr Ser Ile Ser Phe Asp Tyr Thr Cys
            340                 345                 350

Ser Lys Glu Tyr Thr Gly Met Gln Phe Asp Val Thr Ser Phe Thr Phe
        355                 360                 365

Ser Tyr Ile Ala Gln Glu
    370

<210> 40
<211> 343
<212> PRT
<213> adenoviridae

<220>
<221> VARIANT
<222> (1)..(343)
<223> /note="Serotype 29 fiber protein"

<400> 40

Gln Asn Ile Pro Phe Leu Thr Pro Pro Phe Val Ser Ser Asp Gly Phe
1               5                   10                  15

Lys Asn Phe Pro Pro Gly Val Leu Ser Leu Lys Leu Ala Asp Pro Ile
            20                  25                  30

Ala Ile Thr Asn Gly Asp Val Ser Leu Lys Val Gly Gly Gly Leu Thr
        35                  40                  45

Val Glu Gln Asp Ser Gly Asn Leu Ser Val Asn Pro Lys Ala Pro Leu
    50                  55                  60

Gln Val Gly Thr Asp Lys Lys Leu Glu Leu Ala Leu Ala Pro Pro Phe
65                  70                  75                  80

Asp Val Arg Asp Asn Lys Leu Ala Ile Leu Val Gly Asp Gly Leu Lys
                85                  90                  95

Val Ile Asp Arg Ser Ile Ser Asp Leu Pro Gly Leu Leu Asn Tyr Leu
            100                 105                 110

Val Val Leu Thr Gly Lys Gly Ile Gly Asn Glu Glu Leu Lys Asn Asp
        115                 120                 125

Asp Gly Ser Asn Lys Gly Val Gly Leu Cys Val Arg Ile Gly Glu Gly
    130                 135                 140

Gly Gly Leu Thr Phe Asp Asp Lys Gly Tyr Leu Val Ala Trp Asn Asn
145                 150                 155                 160

Lys His Asp Ile Arg Thr Leu Trp Thr Thr Leu Asp Pro Ser Pro Asn
                165                 170                 175

Cys Lys Ile Asp Ile Glu Lys Asp Ser Lys Leu Thr Leu Val Leu Thr
            180                 185                 190

Lys Cys Gly Ser Gln Ile Leu Ala Asn Val Ser Leu Ile Ile Val Asn
        195                 200                 205

Gly Lys Phe Lys Ile Leu Asn Asn Lys Thr Asp Pro Ser Leu Pro Lys
    210                 215                 220

Ser Phe Asn Ile Lys Leu Leu Phe Asp Gln Asn Gly Val Leu Leu Glu
225                 230                 235                 240

Asn Ser Asn Ile Glu Lys Gln Tyr Leu Asn Phe Arg Ser Gly Asp Ser
                245                 250                 255

Ile Leu Pro Glu Pro Tyr Lys Asn Ala Ile Gly Phe Met Pro Asn Leu
            260                 265                 270

Leu Ala Tyr Ala Lys Ala Thr Thr Asp Gln Ser Lys Ile Tyr Ala Arg
        275                 280                 285

Asn Thr Ile Tyr Gly Asn Ile Tyr Leu Asp Asn Gln Pro Tyr Asn Pro
    290                 295                 300

Val Val Ile Lys Ile Thr Phe Asn Asn Glu Ala Asp Ser Ala Tyr Ser
305                 310                 315                 320

Ile Thr Phe Asn Tyr Ser Trp Thr Lys Asp Tyr Asp Asn Ile Pro Phe

                325                 330                 335

Asp Ser Thr Ser Phe Thr Ser
            340

<210> 41
<211> 386
<212> PRT
<213> adenoviridae

<220>
<221> VARIANT
<222> (1)..(386)
<223> /note="Serotype 30 fiber protein"

<400> 41
Ser Cys Ser Cys Pro Ser Ala Pro Thr Ile Phe Met Leu Leu Gln Met
1               5                   10                  15

Lys Arg Ala Arg Pro Ser Xaa Asp Thr Phe Asn Pro Val Tyr Pro Tyr
            20                  25                  30

Gly Tyr Ala Arg Asn Gln Asn Ile Pro Phe Xaa Thr Pro Pro Phe Val
        35                  40                  45

Xaa Ser Asp Gly Phe Lys Asn Phe Pro Pro Gly Val Leu Ser Leu Lys
    50                  55                  60

Leu Ala Asp Pro Ile Ala Ile Thr Asn Gly Asp Val Ser Leu Lys Val
65                  70                  75                  80

Gly Gly Gly Leu Thr Val Glu Gln Asp Ser Gly Asn Leu Ser Val Asn
                85                  90                  95

Xaa Lys Ala Pro Leu Gln Val Gly Thr Asp Lys Lys Leu Glu Leu Ala
            100                 105                 110

Leu Ala Pro Pro Phe Asp Val Arg Asp Asn Lys Leu Ala Ile Leu Val
        115                 120                 125

Gly Asp Gly Leu Lys Val Ile Asp Arg Ser Ile Ser Asp Leu Pro Gly
    130                 135                 140

Leu Leu Asn Tyr Leu Val Val Xaa Thr Gly Lys Gly Ile Gly Asn Glu
145                 150                 155                 160

Glu Leu Lys Asn Asp Asp Gly Ser Asn Lys Gly Val Gly Leu Cys Val
                165                 170                 175

Arg Ile Gly Glu Gly Gly Gly Leu Thr Xaa Asp Asp Lys Gly Tyr Leu
            180                 185                 190

Val Ala Trp Asn Asn Lys His Asp Ile Arg Thr Leu Trp Thr Thr Leu
        195                 200                 205

Asp Pro Ser Pro Asn Cys Lys Ile Asp Ile Glu Lys Asp Ser Lys Leu
    210                 215                 220

Thr Leu Val Leu Thr Lys Cys Gly Ser Gln Ile Leu Ala Asn Val Ser

225                 230                 235                 240

Leu Ile Ile Val Asn Gly Lys Phe Lys Ile Leu Asn Asn Lys Thr Asp
                245                 250                 255

Pro Ser Leu Pro Lys Ser Phe Asn Ile Lys Leu Leu Phe Asp Gln Asn
            260                 265                 270

Gly Val Leu Leu Glu Asn Ser Asn Ile Glu Lys Gln Tyr Leu Asn Phe
        275                 280                 285

Arg Ser Gly Asp Ser Ile Leu Pro Glu Pro Tyr Lys Asn Ala Ile Gly
    290                 295                 300

Phe Met Pro Asn Leu Leu Ala Tyr Ala Lys Ala Thr Thr Asp Gln Ser
305                 310                 315                 320

Lys Ile Tyr Ala Arg Asn Thr Ile Tyr Gly Asn Ile Tyr Leu Asp Asn
                325                 330                 335

Gln Pro Tyr Asn Pro Val Val Ile Lys Ile Thr Phe Asn Asn Glu Ala
            340                 345                 350

Asp Ser Ala Tyr Ser Ile Thr Phe Asn Tyr Ser Trp Thr Lys Asp Tyr
        355                 360                 365

Asp Asn Ile Pro Phe Asp Ser Thr Ser Phe Thr Phe Ser Tyr Ile Ala
    370                 375                 380

Gln Glu
385

<210> 42
<211> 391
<212> PRT
<213> adenoviridae

<220>
<221> VARIANT
<222> (1)..(391)
<223> /note="Serotype 32 fiber protein"

<400> 42
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asp Gly Phe Gln Asn Phe Pro Pro Gly Val Leu Ser Leu Lys
    50                  55                  60

Leu Ala Asp Pro Ile Thr Ile Ala Asn Gly Asn Val Ser Leu Lys Val
65                  70                  75                  80

Gly Gly Gly Leu Thr Leu Glu Gln Asp Ser Gly Lys Leu Ile Val Asn

85                  90                  95

Pro Lys Ala Pro Leu Gln Val Ala Asn Asp Lys Leu Glu Leu Ser Tyr
            100                 105                 110

Ala Asp Pro Phe Glu Thr Ser Ala Asn Lys Leu Ser Leu Lys Val Gly
        115                 120                 125

His Gly Leu Lys Val Leu Asp Glu Lys Asn Ala Gly Gly Leu Lys Asp
    130                 135                 140

Leu Ile Gly Thr Leu Val Val Leu Thr Gly Lys Gly Ile Gly Val Glu
145                 150                 155                 160

Glu Leu Lys Asn Ala Asp Asn Thr Asn Arg Gly Val Gly Ile Asn Val
                165                 170                 175

Arg Leu Gly Lys Asp Gly Gly Leu Ser Phe Asp Lys Lys Gly Asp Leu
            180                 185                 190

Val Ala Trp Asn Lys His Asp Asp Arg Arg Thr Leu Trp Thr Thr Pro
        195                 200                 205

Asp Pro Ser Pro Asn Cys Thr Ile Asp Glu Glu Arg Asp Ser Lys Leu
    210                 215                 220

Thr Leu Val Leu Thr Lys Cys Gly Ser Gln Ile Leu Ala Asn Val Ser
225                 230                 235                 240

Leu Leu Val Val Lys Gly Lys Phe Ser Asn Ile Asn Asn Asn Thr Asn
                245                 250                 255

Pro Thr Asp Lys Lys Ile Thr Val Lys Leu Leu Phe Asn Glu Lys Gly
            260                 265                 270

Val Leu Met Asp Ser Ser Ser Leu Lys Lys Glu Tyr Trp Asn Tyr Arg
        275                 280                 285

Asn Asp Asn Ser Thr Val Ser Gln Ala Tyr Asp Asn Ala Val Pro Phe
    290                 295                 300

Met Pro Asn Ile Lys Ala Tyr Pro Lys Pro Thr Thr Asp Thr Ser Ala
305                 310                 315                 320

Lys Pro Glu Asp Lys Lys Ser Ala Ala Lys Arg Tyr Ile Val Ser Asn
                325                 330                 335

Val Tyr Ile Gly Gly Leu Pro Asp Lys Thr Val Val Ile Thr Ile Lys
            340                 345                 350

Leu Asn Ala Glu Thr Glu Ser Ala Tyr Ser Met Thr Phe Glu Phe Thr
        355                 360                 365

Trp Ala Lys Thr Phe Glu Asn Leu Gln Phe Asp Ser Ser Ser Phe Thr
    370                 375                 380

Phe Ser Tyr Ile Ala Gln Glu
385                 390

<210> 43
<211> 390
<212> PRT
<213> adenoviridae

<220>
<221> VARIANT
<222> (1)..(390)
<223> /note="Serotype 33 fiber protein"

<400> 43
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asp Gly Phe Lys Asn Phe Pro Pro Gly Val Leu Ser Leu Lys
    50                  55                  60

Leu Ala Asp Pro Ile Thr Ile Thr Asn Gly Asp Val Ser Leu Lys Val
65                  70                  75                  80

Gly Gly Gly Leu Thr Leu Gln Glu Gly Ser Leu Thr Val Asn Pro Lys
                85                  90                  95

Ala Pro Leu Gln Leu Ala Asn Asp Lys Lys Leu Glu Leu Val Tyr Asp
            100                 105                 110

Asp Pro Phe Glu Val Ser Thr Asn Lys Leu Ser Leu Lys Val Gly His
        115                 120                 125

Gly Leu Lys Val Leu Asp Asp Lys Ser Ala Gly Gly Leu Gln Asp Leu
    130                 135                 140

Ile Gly Lys Leu Val Val Leu Thr Gly Lys Gly Ile Gly Ile Glu Asn
145                 150                 155                 160

Leu Gln Asn Asp Asp Gly Ser Ser Arg Gly Val Gly Ile Asn Val Arg
                165                 170                 175

Leu Gly Thr Asp Gly Gly Leu Ser Phe Asp Arg Lys Gly Glu Leu Val
            180                 185                 190

Ala Trp Asn Arg Lys Asp Asp Arg Arg Thr Leu Trp Thr Thr Pro Asp
        195                 200                 205

Pro Ser Pro Asn Cys Lys Ala Glu Thr Glu Lys Asp Ser Lys Leu Thr
    210                 215                 220

Leu Val Leu Thr Lys Cys Gly Ser Gln Ile Leu Ala Thr Val Ser Ile
225                 230                 235                 240

Ile Val Leu Lys Gly Lys Tyr Glu Phe Val Lys Lys Glu Thr Glu Pro
                245                 250                 255

Lys Ser Phe Asp Val Lys Leu Leu Phe Asp Ser Lys Gly Val Leu Leu
            260                 265                 270

Pro Thr Ser Asn Leu Ser Lys Glu Tyr Trp Asn Tyr Arg Ser Tyr Asp
        275                 280                 285

Asn Asn Ile Gly Thr Pro Tyr Glu Asn Ala Val Pro Phe Met Pro Asn
    290                 295                 300

Leu Lys Ala Tyr Pro Lys Pro Thr Lys Thr Ala Ser Asp Lys Ala Glu
305                 310                 315                 320

Asn Lys Ile Ser Ser Ala Lys Asn Lys Ile Val Ser Asn Phe Tyr Phe
                325                 330                 335

Gly Gly Gln Ala Tyr Gln Pro Gly Thr Ile Ile Ile Lys Phe Asn Glu
            340                 345                 350

Glu Ile Asp Glu Thr Cys Ala Tyr Ser Ile Thr Phe Asn Phe Gly Trp
        355                 360                 365

Gly Lys Val Tyr Asp Asn Pro Phe Pro Phe Asp Thr Thr Ser Phe Thr
    370                 375                 380

Phe Ser Tyr Ile Ala Gln Glu
385                 390

<210> 44
<211> 337
<212> PRT
<213> adenoviridae

<220>
<221> VARIANT
<222> (1)..(337)
<223> /note="Serotype 34 fiber protein"

<400> 44
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Glu Asp Glu Ser Thr Ser Gln His Pro Phe Ile Asn Pro Gly Phe Ile
        35                  40                  45

Ser Pro Asn Gly Phe Thr Gln Ser Pro Asp Gly Val Leu Thr Leu Lys
    50                  55                  60

Cys Leu Thr Pro Leu Thr Thr Thr Gly Gly Ser Leu Gln Leu Lys Val
65                  70                  75                  80

Gly Gly Gly Leu Thr Val Asp Asp Thr Asp Gly Thr Leu Gln Lys Asn
                85                  90                  95

Ile Arg Ala Thr Thr Pro Ile Thr Lys Asn Asn His Ser Val Glu Leu
            100                 105                 110

Thr Ile Gly Asn Gly Leu Glu Thr Gln His Asn Lys Leu Cys Ala Lys
        115                 120                 125

Leu Gly Asn Gly Leu Lys Phe Asn Asn Gly Asp Ile Cys Ile Lys Asp
    130                 135                 140

Ser Ile Asn Thr Leu Trp Thr Gly Ile Asn Pro Pro Pro Asn Cys Gln
145                 150                 155                 160

Ile Val Glu Asn Thr Asn Thr Asn Asp Gly Lys Leu Thr Leu Val Leu
                165                 170                 175

Val Lys Asn Gly Gly Leu Val Asn Gly Tyr Val Ser Leu Val Gly Val
            180                 185                 190

Ser Asp Thr Val Asn Gln Met Phe Thr Gln Lys Thr Ala Asn Ile Gln
        195                 200                 205

Leu Arg Leu Tyr Phe Asp Ser Ser Gly Asn Leu Leu Thr Asp Glu Ser
    210                 215                 220

Asp Leu Lys Ile Pro Leu Lys Asn Lys Ser Ser Thr Ala Thr Ser Glu
225                 230                 235                 240

Thr Val Ala Ser Ser Lys Ala Phe Met Pro Ser Thr Thr Ala Tyr Pro
                245                 250                 255

Phe Asn Thr Thr Thr Arg Asp Ser Glu Asn Tyr Ile His Gly Ile Cys
            260                 265                 270

Tyr Tyr Met Thr Ser Tyr Asp Arg Ser Leu Phe Pro Leu Asn Ile Ser
        275                 280                 285

Ile Met Leu Asn Ser Arg Met Ile Ser Ser Asn Val Ala Tyr Ala Ile
    290                 295                 300

Gln Phe Glu Trp Asn Leu Asn Ala Ser Glu Ser Pro Glu Lys Gln His
305                 310                 315                 320

Met Thr Leu Thr Thr Ser Pro Phe Phe Phe Ser Tyr Ile Ile Glu Asp
                325                 330                 335

Asp Asn

<210> 45
<211> 337
<212> PRT
<213> adenoviridae

<220>
<221> VARIANT
<222> (1)..(337)
<223> /note="Serotype 35 fiber protein"

<400> 45
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Glu Asp Glu Ser Thr Ser Gln His Pro Phe Ile Asn Pro Gly Phe Ile
        35                  40                  45

Ser Pro Asn Gly Phe Thr Gln Ser Pro Asp Gly Val Leu Thr Leu Lys
    50                  55                  60

Cys Leu Thr Pro Leu Thr Thr Thr Gly Gly Ser Leu Gln Leu Lys Val
65                  70                  75                  80

Gly Gly Gly Leu Thr Val Asp Asp Thr Asp Gly Thr Leu Gln Glu Asn
                85                  90                  95

Ile Arg Ala Thr Ala Pro Ile Thr Lys Asn Asn His Ser Val Glu Leu
            100                 105                 110

Ser Ile Gly Asn Gly Leu Glu Thr Gln Asn Asn Lys Leu Cys Ala Lys
        115                 120                 125

Leu Gly Asn Gly Leu Lys Phe Asn Asn Gly Asp Ile Cys Ile Lys Asp
    130                 135                 140

Ser Ile Asn Thr Leu Trp Thr Gly Ile Asn Pro Pro Pro Asn Cys Gln
145                 150                 155                 160

Ile Val Glu Asn Thr Asn Thr Asn Asp Gly Lys Leu Thr Leu Val Leu
                165                 170                 175

Val Lys Asn Gly Gly Leu Val Asn Gly Tyr Val Ser Leu Val Gly Val
            180                 185                 190

Ser Asp Thr Val Asn Gln Met Phe Thr Gln Lys Thr Ala Asn Ile Gln
        195                 200                 205

Leu Arg Leu Tyr Phe Asp Ser Ser Gly Asn Leu Leu Thr Glu Glu Ser
    210                 215                 220

Asp Leu Lys Ile Pro Leu Lys Asn Lys Ser Ser Thr Ala Thr Ser Glu
225                 230                 235                 240

Thr Val Ala Ser Ser Lys Ala Phe Met Pro Ser Thr Thr Ala Tyr Pro
                245                 250                 255

Phe Asn Thr Thr Thr Arg Asp Ser Glu Asn Tyr Ile His Gly Ile Cys
            260                 265                 270

Tyr Tyr Met Thr Ser Tyr Asp Arg Ser Leu Phe Pro Leu Asn Ile Ser
        275                 280                 285

Ile Met Leu Asn Ser Arg Met Ile Ser Ser Asn Val Ala Tyr Ala Ile
    290                 295                 300

Gln Phe Glu Trp Asn Leu Asn Ala Ser Glu Ser Pro Glu Ser Asn Ile
305                 310                 315                 320

Met Thr Leu Thr Thr Ser Pro Phe Phe Phe Ser Tyr Ile Thr Glu Asp
                325                 330                 335

Asp Asn

<210> 46
<211> 392
<212> PRT
<213> adenoviridae

<220>
<221> VARIANT
<222> (1)..(392)
<223> /note="Serotype 36 fiber protein"

<400> 46
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asp Gly Phe Lys Asn Phe Pro Pro Gly Val Leu Ser Leu Lys
    50                  55                  60

Leu Ala Asp Pro Ile Ala Ile Val Asn Gly Asp Val Ser Leu Lys Val
65                  70                  75                  80

Gly Gly Gly Leu Thr Val Glu Gln Asp Ser Gly Lys Leu Lys Val Asn
                85                  90                  95

Pro Lys Ile Pro Leu Gln Val Val Asn Asp Gln Leu Glu Leu Ala Thr
            100                 105                 110

Asp Lys Pro Phe Lys Ile Glu Asn Asn Lys Leu Ala Leu Asp Val Gly
        115                 120                 125

His Gly Leu Lys Val Ile Asp Lys Thr Ile Ser Asp Leu Gln Gly Leu
    130                 135                 140

Val Gly Lys Leu Val Val Leu Thr Gly Val Gly Ile Gly Thr Glu Thr
145                 150                 155                 160

Leu Lys Asp Lys Asn Asp Lys Val Ile Gly Ser Ala Val Asn Val Arg
                165                 170                 175

Leu Gly Lys Asp Gly Gly Leu Asp Phe Asn Lys Lys Gly Asp Leu Val
            180                 185                 190

Ala Trp Asn Arg Tyr Asp Asp Arg Arg Thr Leu Trp Thr Thr Pro Asp
        195                 200                 205

Pro Ser Pro Asn Cys Lys Val Ser Glu Ala Lys Asp Ser Lys Leu Thr
    210                 215                 220

Leu Val Leu Thr Lys Cys Gly Ser Gln Ile Leu Ala Ser Val Ala Leu
225                 230                 235                 240

Leu Ile Val Lys Gly Lys Tyr Gln Thr Ile Ser Glu Ser Thr Ile Pro
                245                 250                 255

Lys Asp Gln Arg Asn Phe Ser Val Lys Leu Met Phe Asp Glu Lys Gly
            260                 265                 270

Lys Leu Leu Asp Lys Ser Ser Leu Asp Lys Glu Tyr Trp Asn Phe Arg
        275                 280                 285

Ser Asn Asp Ser Val Val Gly Thr Ala Tyr Asp Asn Ala Val Pro Phe
    290                 295                 300

Met Pro Asn Leu Lys Ala Tyr Pro Lys Asn Thr Thr Thr Ser Ser Thr
305                 310                 315                 320

Asn Pro Asp Asp Lys Ile Ser Ala Gly Lys Lys Asn Ile Val Ser Asn
                325                 330                 335

Val Tyr Leu Glu Gly Arg Val Tyr Gln Pro Val Ala Leu Thr Val Lys
            340                 345                 350

Phe Asn Ser Glu Asn Asp Cys Ala Tyr Ser Ile Thr Phe Asp Phe Val
        355                 360                 365

Trp Ser Lys Thr Tyr Glu Ser Pro Val Ala Phe Asp Ser Ser Ser Phe
    370                 375                 380

Thr Phe Ser Tyr Ile Ala Gln Glu
385                 390

<210> 47
<211> 380
<212> PRT
<213> adenoviridae

<220>
<221> VARIANT
<222> (1)..(380)
<223> /note="Serotype 37 fiber protein"

<400> 47
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asp Gly Phe Lys Asn Phe Pro Pro Gly Val Leu Ser Leu Lys
    50                  55                  60

Leu Ala Asp Pro Ile Thr Ile Thr Asn Gly Asp Val Ser Leu Lys Val
65                  70                  75                  80

Gly Gly Gly Leu Thr Leu Gln Asp Gly Ser Leu Thr Val Asn Pro Lys
                85                  90                  95

Ala Pro Leu Gln Val Asn Thr Asp Lys Lys Leu Glu Leu Ala Tyr Asp
            100                 105                 110

Asn Pro Phe Glu Ser Ser Ala Asn Lys Leu Ser Leu Lys Val Gly His
        115                 120                 125

Gly Leu Lys Val Leu Asp Glu Lys Ser Ala Ala Gly Leu Lys Asp Leu
    130                 135                 140

Ile Gly Lys Leu Val Val Leu Thr Gly Lys Gly Ile Gly Thr Glu Asn
145                 150                 155                 160

Leu Glu Asn Thr Asp Gly Ser Ser Arg Gly Ile Gly Ile Asn Val Arg
                165                 170                 175

Ala Arg Glu Gly Leu Thr Phe Asp Asn Asp Gly Tyr Leu Val Ala Trp
            180                 185                 190

Asn Pro Lys Tyr Asp Leu Arg Thr Leu Trp Thr Thr Pro Asp Thr Ser
        195                 200                 205

Pro Asn Cys Thr Ile Ala Gln Asp Lys Asp Ser Lys Leu Thr Leu Val
    210                 215                 220

Leu Thr Lys Cys Gly Ser Gln Ile Leu Ala Asn Val Ser Leu Ile Val
225                 230                 235                 240

Val Ala Gly Lys Tyr His Ile Ile Asn Asn Lys Thr Asn Pro Lys Ile
                245                 250                 255

Lys Ser Phe Thr Ile Lys Leu Leu Phe Asn Lys Asn Gly Val Leu Leu
            260                 265                 270

Asp Asn Ser Asn Leu Gly Lys Ala Tyr Trp Asn Phe Arg Ser Gly Asn
        275                 280                 285

Ser Asn Val Ser Thr Ala Tyr Glu Lys Ala Ile Gly Phe Met Pro Asn
    290                 295                 300

Leu Val Ala Val Ser Lys Pro Ser Asn Ser Lys Lys Tyr Ala Arg Asp
305                 310                 315                 320

Ile Val Tyr Gly Asn Ile Tyr Leu Gly Gly Lys Pro Asp Gln Pro Gly
                325                 330                 335

Val Ile Lys Thr Thr Phe Asn Gln Glu Thr Gly Cys Glu Tyr Ser Ile
            340                 345                 350

Thr Phe Asn Phe Ser Trp Ser Lys Thr Tyr Glu Asn Val Glu Phe Glu
        355                 360                 365

Thr Thr Ser Phe Thr Phe Ser Tyr Ile Ala Gln Glu
    370                 375                 380

<210> 48
<211> 391
<212> PRT
<213> adenoviridae

<220>
<221> VARIANT
<222> (1)..(391)

<223> /note="Serotype 38 fiber protein"

<400> 48
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Xaa Thr Pro Pro Phe Val
        35                  40                  45

Xaa Ser Asp Gly Phe Gln Asn Phe Pro Pro Gly Val Leu Ser Leu Lys
    50                  55                  60

Leu Ala Asp Pro Ile Thr Ile Ala Asn Gly Asn Val Ser Leu Lys Val
65                  70                  75                  80

Gly Gly Gly Leu Thr Leu Glu Gln Asp Ser Gly Lys Leu Ile Val Asn
                85                  90                  95

Xaa Lys Ala Pro Leu Gln Val Ala Asn Asp Lys Leu Glu Leu Ser Tyr
            100                 105                 110

Ala Asp Pro Phe Glu Thr Ser Ala Asn Lys Leu Ser Leu Lys Val Gly
        115                 120                 125

His Gly Leu Lys Val Leu Asp Glu Lys Asn Ala Gly Gly Leu Lys Asp
    130                 135                 140

Leu Ile Gly Thr Leu Val Val Leu Thr Gly Lys Gly Ile Gly Val Glu
145                 150                 155                 160

Glu Leu Lys Asn Ala Asp Asn Thr Asn Arg Gly Val Gly Ile Asn Val
                165                 170                 175

Arg Leu Gly Lys Asp Gly Gly Leu Ser Phe Asp Lys Lys Gly Asp Xaa
            180                 185                 190

Val Ala Trp Asn Lys His Asp Asp Arg Arg Thr Leu Trp Thr Thr Pro
        195                 200                 205

Asp Pro Ser Pro Asn Cys Thr Ile Asp Glu Glu Arg Asp Ser Lys Leu
    210                 215                 220

Thr Leu Val Leu Thr Lys Cys Gly Ser Gln Ile Leu Ala Asn Val Ser
225                 230                 235                 240

Leu Leu Val Val Lys Gly Lys Phe Ser Asn Ile Asn Asn Asn Thr Asn
                245                 250                 255

Pro Thr Asp Lys Lys Ile Thr Val Lys Leu Leu Phe Asn Glu Lys Gly
            260                 265                 270

Val Leu Met Asp Ser Ser Ser Leu Lys Lys Glu Tyr Trp Asn Tyr Arg
        275                 280                 285

Asn Asp Asn Ser Thr Val Ser Gln Ala Tyr Asp Asn Ala Val Pro Phe
    290                 295                 300

Met Pro Asn Ile Lys Ala Tyr Pro Lys Pro Thr Thr Asp Thr Ser Ala

305                 310                 315                 320

Lys Pro Glu Asp Lys Lys Ser Ala Ala Lys Arg Tyr Ile Val Ser Asn
                325                 330                 335

Val Tyr Ile Gly Gly Leu Pro Asp Lys Thr Val Val Ile Thr Ile Lys
            340                 345                 350

Leu Asn Ala Glu Thr Glu Ser Ala Tyr Ser Met Thr Phe Glu Phe Thr
        355                 360                 365

Trp Ala Lys Thr Phe Glu Asn Leu Gln Phe Asp Ser Ser Ser Phe Thr
    370                 375                 380

Phe Ser Tyr Ile Ala Gln Glu
385                 390

<210> 49
<211> 338
<212> PRT
<213> adenoviridae

<220>
<221> VARIANT
<222> (1)..(338)
<223> /note="Serotype 39 fiber protein"

<400> 49
Ile Arg Ile Ser Pro Ser Ser Leu Pro Pro Leu Ser Pro Pro Met Asp
1               5                   10                  15

Ser Lys Thr Ser Pro Leu Gly Cys Tyr His Ser Asn Trp Leu Thr Gln
            20                  25                  30

Ser Pro Ser Pro Met Gly Met Ser His Ser Arg Trp Glu Gly Gly Ser
        35                  40                  45

Pro Trp Gln Glu Gly Thr Gly Asp Leu Lys Val Asn Ala Lys Ser Pro
    50                  55                  60

Leu Gln Val Ala Thr Asn Lys Gln Leu Glu Ile Ala Leu Ala Lys Pro
65                  70                  75                  80

Phe Glu Glu Lys Asp Gly Lys Leu Ala Leu Lys Ile Gly His Gly Leu
                85                  90                  95

Ala Val Val Asp Glu Asn His Thr His Leu Gln Ser Leu Ile Gly Thr
            100                 105                 110

Leu Val Ile Leu Thr Gly Lys Gly Ile Gly Thr Gly Arg Ala Glu Ser
        115                 120                 125

Gly Gly Thr Ile Asp Val Arg Leu Gly Ser Gly Gly Gly Leu Ser Phe
    130                 135                 140

Asp Lys Asp Gly Asn Leu Val Ala Trp Asn Lys Asp Asp Asp Arg Arg
145                 150                 155                 160

Thr Leu Trp Thr Thr Pro Asp Pro Ser Pro Asn Cys Lys Ile Asp Gln

165                 170                 175

Asp Lys Asp Ser Lys Leu Thr Phe Val Leu Thr Lys Cys Gly Ser Gln
            180                 185                 190

Ile Leu Ala Asn Met Ser Leu Leu Val Val Lys Gly Lys Phe Ser Met
        195                 200                 205

Ile Asn Asn Lys Val Asn Gly Thr Asp Asp Tyr Lys Lys Phe Thr Ile
    210                 215                 220

Lys Leu Leu Phe Asp Glu Lys Gly Val Leu Leu Lys Asp Ser Ser Leu
225                 230                 235                 240

Asp Lys Glu Tyr Trp Asn Tyr Arg Ser Asn Asn Asn Asn Val Gly Ser
                245                 250                 255

Ala Tyr Glu Glu Ala Val Gly Phe Met Pro Ser Thr Thr Ala Tyr Pro
            260                 265                 270

Lys Pro Pro Thr Pro Pro Thr Asn Pro Thr Thr Pro Leu Glu Lys Ser
        275                 280                 285

Gln Ala Lys Asn Lys Tyr Val Ser Asn Val Tyr Leu Gly Gly Gln Ala
    290                 295                 300

Gly Asn Pro Val Ala Thr Thr Val Ser Phe Asn Lys Glu Thr Gly Cys
305                 310                 315                 320

Thr Tyr Ser Ile Thr Phe Asp Phe Ala Trp Asn Lys Thr Tyr Glu Asn
                325                 330                 335

Val Gln Cys

<210> 50
<211> 379
<212> PRT
<213> adenoviridae

<220>
<221> VARIANT
<222> (1)..(379)
<223> /note="Serotype 42 fiber protein"

<400> 50
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asp Gly Phe Lys Asn Phe Pro Pro Gly Val Leu Ser Leu Lys
    50                  55                  60

Leu Ala Asn Pro Ile Ala Ile Thr Asn Gly Asp Val Ser Leu Lys Val

65                  70                  75                  80

Gly Gly Gly Leu Thr Leu Gln Asp Gly Thr Gly Lys Leu Thr Ile Asp
                85                  90                  95

Thr Lys Thr Pro Leu Gln Val Ala Asn Asn Lys Leu Glu Leu Ala Phe
            100                 105                 110

Asp Ala Pro Leu Tyr Glu Lys Asn Gly Lys Leu Ala Leu Lys Thr Gly
        115                 120                 125

His Gly Leu Ala Val Leu Thr Lys Asp Ile Gly Ile Pro Glu Leu Ile
    130                 135                 140

Gly Ser Leu Val Ile Leu Thr Gly Lys Gly Ile Gly Thr Gly Thr Val
145                 150                 155                 160

Ala Gly Gly Gly Thr Ile Asp Val Arg Leu Gly Asp Asp Gly Gly Leu
                165                 170                 175

Ser Phe Asp Lys Lys Gly Asp Leu Val Ala Trp Asn Lys Lys Asn Asp
            180                 185                 190

Arg Arg Thr Leu Trp Thr Thr Pro Asp Pro Ser Pro Asn Cys Arg Val
        195                 200                 205

Ser Glu Asp Lys Asp Ser Lys Leu Thr Leu Ile Leu Thr Lys Cys Gly
    210                 215                 220

Ser Gln Ile Leu Ala Ser Phe Ser Leu Leu Val Val Xaa Gly Thr Tyr
225                 230                 235                 240

Thr Thr Val Asp Lys Asn Thr Thr Asn Lys Gln Phe Ser Ile Lys Leu
                245                 250                 255

Leu Phe Asp Ala Asn Gly Lys Leu Lys Ser Glu Ser Asn Leu Ser Gly
            260                 265                 270

Tyr Trp Asn Tyr Arg Ser Asp Asn Ser Val Val Ser Thr Pro Tyr Asp
        275                 280                 285

Asn Ala Val Pro Phe Met Pro Asn Thr Thr Ala Tyr Pro Lys Ile Ile
    290                 295                 300

Asn Ser Thr Thr Asp Pro Glu Asn Lys Lys Ser Ser Ala Lys Lys Thr
305                 310                 315                 320

Ile Val Gly Asn Val Tyr Leu Glu Gly Asn Ala Gly Gln Pro Val Ala
                325                 330                 335

Val Ala Ile Ser Phe Asn Lys Glu Thr Thr Ala Asp Tyr Ser Ile Thr
            340                 345                 350

Phe Asp Phe Ala Trp Ser Lys Ala Tyr Glu Thr Pro Val Pro Phe Asp
        355                 360                 365

Thr Ser Ser Met Thr Phe Ser Tyr Ile Ala Gln Glu
    370                 375                 380

<210> 51
<211> 328
<212> PRT
<213> adenoviridae

<220>
<221> VARIANT
<222> (1)..(328)
<223> /note="Serotype 43 fiber protein"

<400> 51
Asn Ile Pro Xaa Leu Thr Pro Pro Phe Val Ser Ser Asp Gly Phe Lys
1               5                   10                  15

Asn Phe Pro Pro Gly Val Leu Ser Leu Lys Leu Ala Asp Pro Ile Thr
            20                  25                  30

Ile Thr Asn Gly Asp Val Ser Leu Lys Val Gly Gly Gly Leu Thr Val
        35                  40                  45

Glu Lys Glu Ser Gly Asn Leu Thr Val Asn Pro Lys Ala Pro Leu Gln
    50                  55                  60

Val Ala Lys Gly Gln Leu Glu Leu Ala Tyr Asp Ser Pro Phe Asp Val
65                  70                  75                  80

Lys Asn Asn Met Leu Thr Leu Lys Ala Gly His Gly Leu Ala Val Val
                85                  90                  95

Thr Lys Asp Asn Thr Asp Leu Gln Pro Leu Met Gly Thr Leu Val Val
            100                 105                 110

Leu Thr Gly Lys Gly Ile Gly Thr Gly Thr Ser Ala His Gly Gly Thr
        115                 120                 125

Ile Asp Val Arg Ile Gly Lys Asn Gly Ser Leu Ala Phe Asp Lys Asp
    130                 135                 140

Gly Asp Leu Val Ala Trp Asp Lys Glu Asn Asp Arg Arg Thr Leu Trp
145                 150                 155                 160

Thr Thr Pro Asp Thr Ser Pro Asn Cys Lys Met Ser Glu Ala Lys Asp
                165                 170                 175

Ser Lys Leu Thr Leu Ile Leu Thr Lys Cys Gly Ser Gln Ile Leu Gly
            180                 185                 190

Ser Val Ser Leu Leu Ala Val Lys Gly Glu Tyr Gln Asn Met Thr Ala
        195                 200                 205

Asn Thr Lys Lys Asn Val Lys Ile Thr Leu Leu Phe Asp Ala Asn Gly
    210                 215                 220

Val Leu Leu Ala Gly Ser Ser Xaa Xaa Lys Glu Tyr Trp Asn Phe Arg
225                 230                 235                 240

Ser Asn Asp Ser Thr Val Ser Gly Asn Tyr Glu Asn Ala Val Gln Phe
                245                 250                 255

Met Pro Asn Ile Thr Ala Tyr Lys Pro Thr Asn Ser Lys Ser Tyr Ala
            260                 265                 270

Arg Ser Val Ile Phe Gly Asn Val Tyr Ile Asp Ala Lys Pro Tyr Asn
        275                 280                 285

Pro Val Val Ile Lys Ile Ser Phe Asn Gln Glu Thr Gln Asn Asn Cys
    290                 295                 300

Val Tyr Ser Ile Ser Phe Asp Tyr Thr Leu Ser Lys Asp Tyr Pro Asn
305                 310                 315                 320

Met Gln Phe Asp Val Thr Leu Ser
                325

<210> 52
<211> 341
<212> PRT
<213> adenoviridae

<220>
<221> VARIANT
<222> (1)..(341)
<223> /note="Serotype 44 fiber protein"

<400> 52
Asn Ile Pro Phe Leu Thr Pro Pro Phe Val Ser Ser Asp Gly Phe Gln
1               5                   10                  15

Asn Phe Pro Pro Gly Val Leu Ser Leu Lys Leu Ala Asp Pro Ile Thr
            20                  25                  30

Ile Thr Asn Gly Asn Val Ser Leu Lys Val Gly Gly Gly Leu Thr Leu
        35                  40                  45

Gln Glu Gly Thr Gly Asp Leu Lys Val Asn Ala Lys Ser Pro Leu Gln
    50                  55                  60

Val Ala Thr Asn Lys Gln Leu Glu Ile Ala Leu Ala Lys Pro Phe Glu
65                  70                  75                  80

Glu Lys Asp Gly Lys Leu Ala Leu Lys Ile Gly His Gly Leu Ala Val
                85                  90                  95

Val Asp Glu Asn His Thr His Leu Gln Ser Leu Ile Gly Thr Leu Val
            100                 105                 110

Ile Leu Thr Gly Lys Gly Ile Gly Thr Gly Ser Ala Glu Ser Gly Gly
        115                 120                 125

Thr Ile Asp Val Arg Leu Gly Ser Gly Gly Gly Leu Ser Phe Asp Lys
    130                 135                 140

Asp Gly Asn Leu Val Ala Trp Asn Lys Asp Asp Asp Arg Arg Thr Leu
145                 150                 155                 160

Trp Thr Thr Pro Asp Pro Ser Pro Asn Cys Lys Ile Asp Gln Asp Lys
                165                 170                 175

Asp Ser Lys Leu Thr Phe Val Leu Thr Lys Cys Gly Ser Gln Ile Leu
            180                 185                 190

Ala Asn Met Ser Leu Leu Val Val Lys Gly Lys Phe Ser Met Ile Asn
        195                 200                 205

Asn Lys Val Asn Gly Thr Asp Asp Tyr Lys Lys Phe Thr Ile Lys Leu
    210                 215                 220

Leu Phe Asp Glu Lys Gly Val Leu Leu Lys Asp Ser Ser Leu Asp Lys
225                 230                 235                 240

Glu Tyr Trp Asn Tyr Arg Ser Asn Asn Asn Asn Val Gly Ser Ala Tyr
                245                 250                 255

Glu Glu Ala Val Gly Phe Met Pro Ser Thr Thr Ala Tyr Pro Lys Pro
            260                 265                 270

Pro Thr Pro Pro Thr Asn Pro Thr Thr Pro Leu Glu Lys Ser Gln Ala
        275                 280                 285

Lys Asn Lys Tyr Val Ser Asn Val Tyr Leu Gly Gly Gln Ala Gly Asn
    290                 295                 300

Pro Val Ala Thr Thr Val Ser Phe Asn Lys Glu Thr Gly Cys Thr Tyr
305                 310                 315                 320

Ser Ile Thr Phe Asp Phe Ala Trp Asn Lys Thr Tyr Glu Asn Val Gln
                325                 330                 335

Phe Asp Ser Ser Phe
            340

<210> 53
<211> 345
<212> PRT
<213> adenoviridae

<220>
<221> VARIANT
<222> (1)..(345)
<223> /note="Serotype 45 fiber protein"

<400> 53
Asn Ile Pro Phe Leu Thr Pro Pro Phe Val Ser Ser Asp Gly Phe Gln
1               5                   10                  15

Asn Phe Pro Pro Gly Val Leu Ser Leu Lys Leu Ala Asp Pro Ile Ala
            20                  25                  30

Ile Thr Asn Gly Asp Val Ser Leu Lys Val Gly Gly Gly Leu Thr Val
        35                  40                  45

Glu Lys Asp Ser Gly Asn Leu Lys Val Asn Pro Lys Ala Pro Leu Gln
    50                  55                  60

Val Thr Thr Asp Lys Gln Leu Glu Ile Ala Leu Ala Tyr Pro Phe Glu
65                  70                  75                  80

Val Ser Asn Gly Lys Leu Gly Ile Lys Ala Gly His Gly Leu Lys Val
                85                  90                  95

Ile Asp Lys Ile Ala Gly Leu Glu Gly Leu Ala Gly Thr Leu Val Val
            100                 105                 110

Leu Thr Gly Lys Gly Ile Gly Thr Glu Asn Leu Glu Asn Ser Asp Gly
        115                 120                 125

Ser Ser Arg Gly Val Gly Ile Asn Val Arg Leu Ala Lys Asp Gly Val
    130                 135                 140

Leu Ala Phe Asp Lys Lys Gly Asp Leu Val Ala Trp Asn Lys His Asp
145                 150                 155                 160

Asp Arg Arg Thr Leu Trp Thr Thr Pro Asp Pro Ser Pro Asn Cys Thr
                165                 170                 175

Ile Asp Gln Glu Arg Asp Ser Lys Leu Thr Leu Val Leu Thr Lys Cys
            180                 185                 190

Gly Ser Gln Ile Leu Ala Asn Val Ser Leu Leu Val Val Lys Gly Lys
        195                 200                 205

Phe Ser Asn Ile Asn Asn Asn Ala Asn Pro Thr Asp Lys Lys Ile Thr
    210                 215                 220

Val Lys Leu Leu Phe Asn Glu Lys Gly Val Leu Met Asp Ser Ser Thr
225                 230                 235                 240

Leu Lys Lys Glu Tyr Trp Asn Tyr Arg Asn Asp Asn Ser Thr Val Ser
                245                 250                 255

Gln Ala Tyr Asp Asn Ala Val Pro Phe Met Pro Asn Ile Lys Ala Tyr
            260                 265                 270

Pro Lys Pro Ser Thr Asp Thr Ser Ala Lys Pro Glu Asp Lys Lys Ser
        275                 280                 285

Ala Ala Lys Arg Tyr Ile Val Ser Asn Val Tyr Ile Gly Gly Leu Pro
    290                 295                 300

Asp Lys Thr Val Val Ile Thr Ile Lys Phe Asn Ala Glu Thr Glu Cys
305                 310                 315                 320

Ala Tyr Ser Ile Thr Phe Glu Phe Thr Trp Ala Lys Thr Phe Glu Asp
                325                 330                 335

Val Gln Cys Asp Ser Ser Ser Phe Thr
            340                 345

<210> 54
<211> 340
<212> PRT
<213> adenoviridae

<220>
<221> VARIANT
<222> (1)..(340)
<223> /note="Serotype 46 fiber protein"

<400> 54
Asn Ile Pro Phe Leu Thr Pro Pro Phe Val Ser Ser Asp Gly Phe Lys
1               5                   10                  15

Asn Phe Pro Pro Gly Val Leu Ser Leu Lys Leu Ala Asp Pro Ile Ala
            20                  25                  30

Ile Val Asn Gly Asp Val Ser Leu Lys Val Gly Gly Gly Leu Thr Leu
        35                  40                  45

Gln Glu Gly Asn Leu Thr Val Asp Ala Lys Ala Pro Leu Gln Val Ala
    50                  55                  60

Asn Asp Asn Lys Leu Glu Leu Ser Tyr Ala Asp Pro Phe Glu Val Lys
65                  70                  75                  80

Asp Thr Lys Leu Gln Leu Lys Val Gly His Gly Leu Lys Val Ile Asp
                85                  90                  95

Glu Lys Thr Ser Ser Gly Leu Gln Ser Leu Ile Gly Asn Leu Val Val
            100                 105                 110

Leu Thr Gly Lys Gly Ile Gly Thr Gln Glu Leu Lys Asp Lys Asp Asp
        115                 120                 125

Glu Thr Lys Asn Ile Gly Val Gly Ile Asn Val Arg Ile Gly Lys Asn
    130                 135                 140

Glu Ser Leu Ala Phe Asp Lys Asp Gly Asn Leu Val Ala Trp Asp Asn
145                 150                 155                 160

Glu Asn Asp Arg Arg Thr Leu Trp Thr Thr Pro Asp Thr Ser Ser Lys
                165                 170                 175

Phe Val Lys Ile Ser Thr Glu Lys Asp Ser Lys Leu Thr Leu Val Leu
            180                 185                 190

Thr Lys Cys Gly Ser Gln Ile Leu Ala Ser Val Ser Leu Leu Ala Val
        195                 200                 205

Ala Gly Ser Tyr Leu Asn Met Thr Ala Ser Thr Gln Lys Ser Ile Lys
    210                 215                 220

Val Ser Leu Met Phe Asp Ser Lys Gly Leu Leu Met Thr Thr Ser Ser
225                 230                 235                 240

Ile Asp Lys Gly Tyr Trp Asn Tyr Arg Asn Lys Asn Ser Val Val Gly
                245                 250                 255

Thr Ala Tyr Glu Asn Ala Ile Pro Phe Met Pro Asn Leu Val Ala Tyr
            260                 265                 270

Pro Arg Pro Asn Thr Pro Asp Ser Lys Ile Tyr Ala Arg Ser Lys Ile
        275                 280                 285

Val Gly Asn Val Tyr Leu Ala Gly Leu Ala Tyr Gln Pro Ile Val Ile
    290                 295                 300

Thr Val Ser Phe Asn Gln Glu Lys Asp Ala Ser Cys Ala Tyr Ser Ile
305                 310                 315                 320

Thr Phe Glu Phe Ala Trp Asn Lys Asp Tyr Val Gly Gln Phe Asp Thr
                325                 330                 335

Thr Ser Phe Thr
            340

<210> 55
<211> 389
<212> PRT
<213> adenoviridae

<220>
<221> VARIANT
<222> (1)..(389)
<223> /note="Serotype 47 fiber protein"

<400> 55
Ser Cys Pro Ser Ala Pro Thr Ile Phe Met Leu Leu Gln Met Lys Arg
1               5                   10                  15

Ala Arg Pro Ser Glu Asp Thr Phe Asn Pro Val Tyr Pro Tyr Gly Tyr
            20                  25                  30

Ala Arg Asn Gln Asn Ile Pro Phe Leu Thr Pro Pro Phe Val Ser Ser
        35                  40                  45

Asp Gly Phe Lys Asn Phe Pro Pro Gly Val Leu Ser Leu Lys Leu Ala
    50                  55                  60

Asp Pro Ile Thr Ile Thr Asn Gly Asp Val Ser Leu Lys Val Gly Gly
65                  70                  75                  80

Gly Leu Thr Leu Gln Glu Gly Thr Gly Asn Leu Thr Val Asn Ala Lys
                85                  90                  95

Ala Pro Leu Gln Val Ala Asp Asp Lys Lys Leu Glu Leu Ser Tyr Asp
            100                 105                 110

Asn Pro Phe Glu Val Ser Ala Asn Lys Leu Ser Leu Lys Val Gly His
        115                 120                 125

Gly Leu Lys Val Leu Asp Glu Lys Asn Ser Gly Gly Leu Gln Glu Leu
    130                 135                 140

Ile Gly Lys Leu Val Ile Leu Thr Gly Lys Gly Ile Gly Val Glu Glu
145                 150                 155                 160

Leu Lys Asn Ala Asp Asn Thr Asn Arg Gly Val Gly Ile Asn Val Arg
                165                 170                 175

Leu Gly Lys Asp Gly Gly Leu Ser Phe Asp Lys Lys Gly Glu Leu Val
            180                 185                 190

Ala Trp Asn Lys His Asn Asp Thr Arg Thr Leu Trp Thr Thr Pro Asp
        195                 200                 205

Pro Ser Pro Asn Cys Lys Ile Glu Gln Asp Lys Asp Ser Lys Leu Thr
    210                 215                 220

Leu Val Leu Thr Lys Cys Gly Ser Gln Ile Leu Ala Thr Met Ala Phe
225                 230                 235                 240

Gln Val Val Lys Gly Thr Tyr Glu Asn Ile Ser Lys Asn Thr Ala Lys
                245                 250                 255

Lys Ser Phe Ser Ile Lys Leu Leu Phe Asp Asp Asn Gly Lys Leu Leu
            260                 265                 270

Glu Gly Ser Ser Leu Asp Lys Asp Tyr Trp Asn Phe Arg Asn Asp Asp
        275                 280                 285

Ser Ile Met Pro Asn Gln Tyr Asp Asn Ala Val Pro Phe Met Pro Asn
    290                 295                 300

Leu Lys Ala Tyr Pro Asn Pro Lys Thr Ser Thr Val Leu Pro Ser Thr
305                 310                 315                 320

Asp Lys Lys Ser Asn Gly Lys Asn Thr Ile Val Ser Asn Leu Tyr Leu
                325                 330                 335

Glu Gly Lys Ala Tyr Gln Pro Val Ala Val Thr Ile Thr Phe Asn Lys
            340                 345                 350

Glu Thr Gly Cys Thr Tyr Ser Ile Thr Phe Glu Phe Gly Trp Ala Lys
        355                 360                 365

Thr Tyr Asp Val Pro Ile Pro Phe Asp Ser Ser Ser Phe Thr Phe Ser
    370                 375                 380

Tyr Ile Ala Gln Glu
385

<210> 56
<211> 343
<212> PRT
<213> adenoviridae

<220>
<221> VARIANT
<222> (1)..(343)
<223> /note="Serotype 48 fiber protein"

<400> 56
Ser Asp Ile Pro Phe Leu Thr Pro Pro Phe Val Ser Ser Asp Gly Phe
1               5                   10                  15

Gln Asn Phe Pro Pro Gly Val Leu Ser Leu Lys Leu Ala Asp Pro Ile
            20                  25                  30

Thr Ile Thr Asn Gly Asn Val Ser Leu Lys Val Gly Gly Gly Leu Thr
        35                  40                  45

Leu Gln Glu Gly Thr Gly Asp Leu Lys Val Asn Ala Lys Ser Pro Leu
    50                  55                  60

Gln Val Ala Thr Asn Lys Gln Leu Glu Ile Ala Leu Ala Lys Pro Phe
65                  70                  75                  80

Glu Glu Lys Asp Gly Lys Leu Ala Leu Lys Ile Gly His Glu Leu Ala
                85                  90                  95

Val Val Asp Glu Asn Leu Thr His Leu Gln Ser Leu Ile Gly Thr Leu
            100                 105                 110

Val Ile Leu Thr Gly Lys Gly Ile Gly Thr Gly Arg Ala Glu Ser Gly
        115                 120                 125

Gly Thr Ile Asp Val Arg Leu Gly Ser Gly Gly Gly Leu Ser Phe Asp
    130                 135                 140

Lys Asp Gly Asn Leu Val Ala Trp Asn Lys Asp Asp Asp Arg Arg Thr
145                 150                 155                 160

Leu Trp Thr Thr Pro Asp Pro Ser Pro Asn Cys Lys Ile Asp Gln Asp
                165                 170                 175

Lys Asp Ser Lys Leu Thr Phe Val Leu Thr Lys Cys Gly Ser Gln Ile
            180                 185                 190

Leu Ala Asn Met Ser Leu Leu Val Val Lys Gly Lys Phe Ser Met Ile
        195                 200                 205

Asn Asn Lys Val Asn Gly Thr Asp Asp Tyr Lys Lys Phe Thr Ile Lys
    210                 215                 220

Leu Leu Phe Asp Glu Lys Gly Val Leu Leu Lys Asp Ser Ser Leu Asp
225                 230                 235                 240

Lys Glu Tyr Trp Asn Tyr Arg Ser Asn Asn Asn Asn Val Gly Ser Ala
                245                 250                 255

Tyr Glu Glu Ala Val Gly Phe Met Pro Ser Thr Thr Ala Tyr Pro Lys
            260                 265                 270

Pro Pro Thr Pro Pro Thr Asn Pro Thr Thr Pro Leu Glu Lys Ser Gln
        275                 280                 285

Ala Lys Asn Lys Tyr Val Ser Asn Val Tyr Leu Gly Gly Gln Ala Gly
    290                 295                 300

Asn Pro Val Ala Thr Thr Val Ser Phe Asn Lys Glu Thr Gly Cys Thr
305                 310                 315                 320

Tyr Ser Ile Thr Phe Asp Phe Ala Trp Asn Lys Thr Tyr Lys Met Ala
                325                 330                 335

Phe Ile Pro Arg Phe Asn Phe
            340

<210> 57
<211> 394
<212> PRT
<213> adenoviridae

<220>
<221> VARIANT
<222> (1)..(394)

<223> /note="Serotype 49 fiber protein"

<400> 57
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Gly Tyr Ala Arg Asn Gln Asn Ile Pro Phe Leu Thr Pro Pro Phe Val
        35                  40                  45

Ser Ser Asp Gly Phe Gln Asn Phe Pro Pro Gly Val Leu Ser Leu Lys
    50                  55                  60

Leu Ala Asp Pro Ile Ala Ile Thr Asn Gly Asn Val Ser Leu Lys Val
65                  70                  75                  80

Gly Gly Gly Leu Thr Val Glu Gln Asp Ser Gly Asn Leu Lys Val Asn
                85                  90                  95

Pro Lys Ala Pro Leu Gln Val Ala Thr Asp Asn Gln Leu Glu Ile Ser
            100                 105                 110

Leu Ala Asp Pro Phe Glu Val Lys Asn Lys Lys Leu Ser Leu Lys Val
        115                 120                 125

Gly His Gly Leu Lys Val Ile Asp Glu Asn Ile Ser Thr Leu Gln Gly
    130                 135                 140

Leu Leu Gly Asn Leu Val Val Leu Thr Gly Met Gly Ile Gly Thr Glu
145                 150                 155                 160

Glu Leu Lys Lys Asp Asp Lys Ile Val Gly Ser Ala Val Asn Val Arg
                165                 170                 175

Leu Gly Gln Asp Gly Gly Leu Thr Phe Asp Lys Lys Gly Asp Leu Val
            180                 185                 190

Ala Trp Asn Lys Glu Asn Asp Arg Arg Thr Leu Trp Thr Thr Pro Asp
        195                 200                 205

Pro Ser Pro Asn Cys Lys Val Ser Glu Glu Lys Asp Ser Lys Leu Thr
    210                 215                 220

Leu Val Leu Thr Lys Cys Gly Ser Gln Ile Leu Ala Ser Val Ser Leu
225                 230                 235                 240

Leu Val Val Lys Gly Lys Phe Ala Asn Ile Asn Asn Lys Thr Asn Pro
                245                 250                 255

Gly Glu Asp Tyr Lys Xaa Phe Ser Val Lys Leu Leu Phe Asp Ala Asn
            260                 265                 270

Gly Lys Leu Leu Thr Gly Ser Ser Leu Asp Gly Asn Tyr Trp Asn Tyr
        275                 280                 285

Lys Asn Lys Asp Ser Val Ile Gly Ser Pro Tyr Glu Asn Ala Val Pro
    290                 295                 300

Phe Met Pro Asn Ser Thr Ala Tyr Pro Lys Ile Ile Asn Asn Gly Thr

305                 310                 315                 320

Ala Asn Pro Glu Asp Lys Lys Ser Ala Ala Lys Lys Thr Ile Val Thr
                325                 330                 335

Asn Val Tyr Leu Gly Gly Asp Ala Ala Lys Pro Val Ala Thr Thr Ile
            340                 345                 350

Ser Phe Asn Lys Glu Thr Glu Ser Asn Cys Val Tyr Ser Ile Thr Phe
        355                 360                 365

Asp Phe Ala Trp Asn Lys Thr Tyr Lys Asn Val Pro Phe Asp Ser Ser
    370                 375                 380

Ser Leu Thr Phe Ser Tyr Ile Ala Gln Glu
385                 390

<210> 58
<211> 353
<212> PRT
<213> adenoviridae

<220>
<221> VARIANT
<222> (1)..(353)
<223> /note="Serotype 51 fiber protein"

<400> 58
Ser Cys Ser Cys Pro Ser Ala Pro Thr Ile Phe Met Leu Leu Gln Met
1               5                   10                  15

Lys Arg Ala Arg Pro Ser Glu Asp Thr Phe Asn Pro Val Tyr Pro Tyr
            20                  25                  30

Glu Asp Glu Ser Thr Ser Gln His Pro Phe Ile Asn Pro Gly Phe Ile
        35                  40                  45

Ser Pro Asn Gly Phe Thr Gln Ser Pro Asp Gly Val Leu Thr Leu Asn
    50                  55                  60

Cys Leu Thr Pro Leu Thr Thr Thr Gly Gly Pro Leu Gln Leu Lys Val
65                  70                  75                  80

Gly Gly Gly Leu Ile Val Asp Asp Thr Asp Gly Thr Leu Gln Glu Asn
                85                  90                  95

Ile Arg Val Thr Ala Pro Ile Thr Lys Asn Asn His Ser Val Glu Leu
            100                 105                 110

Ser Ile Gly Asn Gly Leu Glu Thr Gln Asn Asn Lys Leu Cys Ala Lys
        115                 120                 125

Leu Gly Asn Gly Leu Lys Phe Asn Asn Gly Asp Ile Cys Ile Lys Asp
    130                 135                 140

Ser Ile Asn Thr Leu Trp Thr Gly Ile Lys Pro Pro Pro Asn Cys Gln
145                 150                 155                 160

Ile Val Glu Asn Thr Asp Thr Asn Asp Gly Lys Leu Thr Leu Val Leu
                165                 170                 175

Val Lys Asn Gly Gly Leu Val Asn Gly Tyr Val Ser Leu Val Gly Val
            180                 185                 190

Ser Asp Thr Val Asn Gln Met Phe Thr Gln Lys Ser Ala Thr Ile Gln
        195                 200                 205

Leu Arg Leu Tyr Phe Asp Ser Ser Gly Asn Leu Leu Thr Asp Glu Ser
    210                 215                 220

Asn Leu Lys Ile Pro Leu Lys Asn Lys Ser Ser Thr Ala Thr Ser Glu
225                 230                 235                 240

Ala Ala Thr Ser Ser Lys Ala Phe Met Pro Ser Thr Thr Ala Tyr Pro
                245                 250                 255

Phe Asn Thr Thr Thr Arg Asp Ser Glu Asn Tyr Ile His Gly Ile Cys
            260                 265                 270

Tyr Tyr Met Thr Ser Tyr Asp Arg Ser Leu Val Pro Leu Asn Ile Ser
        275                 280                 285

Ile Met Leu Asn Ser Arg Thr Ile Ser Ser Asn Val Ala Tyr Ala Ile
    290                 295                 300

Gln Phe Glu Trp Asn Leu Asn Ala Lys Glu Ser Pro Glu Ser Asn Ile
305                 310                 315                 320

Ala Thr Leu Thr Thr Ser Pro Phe Phe Phe Ser Tyr Ile Ile Glu Asp
                325                 330                 335

Thr Thr Lys Cys Ile Ser Leu Cys Tyr Val Ser Thr Cys Leu Phe Phe
            340                 345                 350

Asn